# EUROPEAN PATENT APPLICATION

(11) **EP 0 599 077 A2**
(43) Date of publication of application: **01.06.1994**
(21) Application number: 93117519.4
(22) Date of filing: 28.10.1993
(51) Int. Cl.: C12N 15/12, C12N 15/67, C12N 15/85, A61K 39/00, A61K 48/00

(54) **Anti-metastatic vaccine**

(30) Priority: 29.10.1992 US 968415
(71) Applicant: YEDA RESEARCH AND DEVELOPMENT CO. LTD., Rehovot 76100 (IL)
(72) Inventor: Eisenbach, Lea, Rehovot 76303 (IL); Feldmann, Michael, Rehovot 76100 (IL)
(74) Representative: VOSSIUS & PARTNER

(57) **Abstract**

In accordance with the present invention, there is provided an antitumor cellular vaccine comprising tumor cells into which *c-fos* gene alone or together with *c-jun* have been inserted.

Additionally, the present invention provides a method of treating a patient suffering from cancer to prevent and/or inhibit the development of metastasis which comprises the administration of the above mentioned cellular vaccine.

Further, the present invention provides an antitumor vaccine comprising antigens expressed by *c-fos* gene alone or together with *c-jun* gene.

SEQUENCE LISTING

(1) GENERAL INFORMATION:

(i) APPLICANT: Eisenbach, Lea
Feldman, Michael

(ii) TITLE OF INVENTION: Anti-Metastatic Vaccine

(iii) NUMBER OF SEQUENCES: 8

(iv) CORRESPONDENCE ADDRESS:
(A) ADDRESSEE: Reising, Ethington, Barnard, Perry & Milton
(B) STREET: P. O. Box 4390
(C) CITY: Troy
(D) STATE: Michigan
(E) COUNTRY: U.S.A.
(F) ZIP: 48099

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER: US 07/968,415
(B) FILING DATE:
(C) CLASSIFICATION:

(viii) ATTORNEY/AGENT INFORMATION:
(A) NAME: Kohn, Kenneth I.
(B) REGISTRATION NUMBER: 30,955
(C) REFERENCE/DOCKET NUMBER: P-301(Weiz 28-92)

(ix) TELECOMMUNICATION INFORMATION:
(A) TELEPHONE: (313) 689-3500
(B) TELEFAX: (313) 689-4071

(2) INFORMATION FOR SEQ ID NO:1:

(i) SEQUENCE CHARACTERISTICS:
(A) LENGTH: 3135 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: mRNA
(B) LOCATION: 1..3135

(ix) FEATURE:
(A) NAME/KEY: CDS
(B) LOCATION: 917..1921

(xi) SEQUENCE DESCRIPTION: SEQ ID NO:1:

CTGAGTGTGC GAGAGACAGC CTGGCAGGAG AGCGCTCAGG CAGACAGACA GACAGACGGA 60

CGGACTTGGC CAACCCGGTC GGCCGCGGAC TCCGGACTGT TCATCCGTTT GTCTTCATTT 120

TCTCACCAAC TGCTTGGATC CAGCGCCCGC GGCTCCTGCA CCGGTATTTT GGGGAGCATT 180

TGGAGAGTCC CTTCTCCCGC CTTCCACGGA GAAGAAGCTC ACAAGTCCGG GCGCTGCTGA 240

CAGCATCGAG AGCGGCTCCC GACCGCGCGA GGAAATAGGC GAGCGGCTAC CGGCCAGCAA 300

CTTTCCTGAC CCAGAGGACC GGTAACAAGT GGCCGGGAGC GAACTTTTGC AAATCTCTTC 360

TGCGCCTTAA GGCTGCCACC GAGACTGTAA AGAAAAGGGA GAAGAGGAAC CTATACTCAT 420

ACCAGTTCGC ACAGGCGGCT GAAGTTGGGC GAGCGCTAGC CGCGGCTGCC TAGCGTCCCC 480

CTCCCCCTCA CAGCGGAGGA GGGGACAGTT GTTGGAGGCC GGGCGGCAGA GCCCGATCGC 540

GGGCTTCCAC CGAGAATTCC GTGACGACTG GTCAGCACCG CCGGAGAGCC GCTGTTGCTG 600

GGACTGGTCT GCGGGCTCCA AGGAACCGCT GCTCCCCGAG AGCGCTCCGT GAGTGACCGC 660

GACTTTTCAA AGCTCGGCAT CGCGCGGGAG CCTACCAACG TGAGTGCTAG CGGAGTCTTA 720

ACCCTGCGCT CCCTGGAGCG AACTGGGGAG GAGGGCTCAG GGGGAAGCAC TGCCGTCTGG 780

AGCGCACGCT CCTAAACAAA CTTTGTTACA GAAGCAGGGA CGCGCGGGTA TCCCCCCGCT 840

TCCCGGCGCG CTGTTGCGGC CCCGAAACTT CTGCGCACAG CCCAGGCTAA CCCCGCGTGA 900

AGTGACGGAC CGTTCT ATG ACT GCA AAG ATG GAA ACG ACC TTC TAC GAC 949
Met Thr Ala Lys Met Glu Thr Thr Phe Tyr Asp
1               5                   10

GAT GCC CTC AAC GCC TCG TTC CTC CAG TCC GAG AGC GGT GCC TAC GGC 997
Asp Ala Leu Asn Ala Ser Phe Leu Gln Ser Glu Ser Gly Ala Tyr Gly
            15                  20                  25

TAC AGT AAC CCT AAG ATC CTA AAA CAG AGC ATG ACC TTG AAC CTG GCC 1045
Tyr Ser Asn Pro Lys Ile Leu Lys Gln Ser Met Thr Leu Asn Leu Ala
        30                  35                  40

GAC CCG GTG GGC AGT CTG AAG CCG CAC CTC CGC GCC AAG AAC TCG GAC 1093
Asp Pro Val Gly Ser Leu Lys Pro His Leu Arg Ala Lys Asn Ser Asp
    45                  50                  55

CTT CTC ACG TCG CCC GAC GTC GGG CTG CTC AAG CTG GCG TCG CCG GAG 1141
Leu Leu Thr Ser Pro Asp Val Gly Leu Leu Lys Leu Ala Ser Pro Glu
60                  65                  70                  75

CTG GAG CGC CTG ATC ATC CAG TCC AGC AAT GGG CAC ATC ACC ACT ACA 1189
Leu Glu Arg Leu Ile Ile Gln Ser Ser Asn Gly His Ile Thr Thr Thr
                80                  85                  90

CCG ACC CCC ACC CAG TTC TTG TGC CCC AAG AAC GTG ACC GAC GAG CAG 1237
Pro Thr Pro Thr Gln Phe Leu Cys Pro Lys Asn Val Thr Asp Glu Gln
            95                  100                 105

GAG GGC TTC GCC GAG GGC TTC GTG CGC GCC CTG GCT GAA CTG CAT AGC 1285
Glu Gly Phe Ala Glu Gly Phe Val Arg Ala Leu Ala Glu Leu His Ser
        110                 115                 120

CAG AAC ACG CTT CCC AGT GTC ACC TCC GCG GCA CAG CCG GTC AGC GGG     1333
Gln Asn Thr Leu Pro Ser Val Thr Ser Ala Ala Gln Pro Val Ser Gly
    125                 130                 135

GCG GGC ATG GTG GCT CCC GCG GTG GCC TCA GTA GCA GGC GCT GGC GGC     1381
Ala Gly Met Val Ala Pro Ala Val Ala Ser Val Ala Gly Ala Gly Gly
140                 145                 150                 155

GGT GGT GGC TAC AGC GCC AGC CTG CAC AGT GAG CCT CCG GTC TAC GCC     1429
Gly Gly Gly Tyr Ser Ala Ser Leu His Ser Glu Pro Pro Val Tyr Ala
                160                 165                 170

AAC CTC AGC AAC TTC AAC CCG GGT GCG CTG AGC AGC GGC GGT GGG GCG     1477
Asn Leu Ser Asn Phe Asn Pro Gly Ala Leu Ser Ser Gly Gly Gly Ala
            175                 180                 185

CCC TCC TAT GGC GCG GCC GGG CTG GCC TTT CCC TCG CAG CCG CAG CAG     1525
Pro Ser Tyr Gly Ala Ala Gly Leu Ala Phe Pro Ser Gln Pro Gln Gln
        190                 195                 200

CAG CAG CAG CCG CCT CAG CCG CCG CAC CAC TTG CCC CAA CAG ATC CCG     1573
Gln Gln Gln Pro Pro Gln Pro Pro His His Leu Pro Gln Gln Ile Pro
    205                 210                 215

GTG CAG CAC CCG CGG CTG CAA GCC CTG AAG GAA GAG CCG CAG ACC GTG     1621
Val Gln His Pro Arg Leu Gln Ala Leu Lys Glu Glu Pro Gln Thr Val
220                 225                 230                 235

CCG GAG ATG CCG GGA GAG ACG CCG CCC CTG TCC CCT ATC GAC ATG GAG     1669
Pro Glu Met Pro Gly Glu Thr Pro Pro Leu Ser Pro Ile Asp Met Glu
                240                 245                 250

TCT CAG GAG CGG ATC AAG GCA GAG AGG AAG CGC ATG AGG AAC CGC ATT     1717
Ser Gln Glu Arg Ile Lys Ala Glu Arg Lys Arg Met Arg Asn Arg Ile
            255                 260                 265

GCC GCC TCC AAG TGC CGG AAA AGG AAG CTG GAG CGG ATC GCT CGG CTA     1765
Ala Ala Ser Lys Cys Arg Lys Arg Lys Leu Glu Arg Ile Ala Arg Leu
        270                 275                 280

GAG GAA AAA GTG AAA ACC TTG AAA GCG CAA AAC TCC GAG CTG GCA TCC     1813
Glu Glu Lys Val Lys Thr Leu Lys Ala Gln Asn Ser Glu Leu Ala Ser
    285                 290                 295

ACG GCC AAC ATG CTC AGG GAA CAG GTG GCA CAG CTT AAG CAG AAA GTC     1861
Thr Ala Asn Met Leu Arg Glu Gln Val Ala Gln Leu Lys Gln Lys Val
300                 305                 310                 315

ATG AAC CAC GTT AAC AGT GGG TGC CAA CTC ATG CTA ACG CAG CAG TTG     1909
Met Asn His Val Asn Ser Gly Cys Gln Leu Met Leu Thr Gln Gln Leu
                320                 325                 330

CAA ACG TTT TGAGAACAGA CTGTCAGGGC TGAGGGGCAA TGGAAGAAAA             1958
Gln Thr Phe
        335

AAAATAACAG AGACAAACTT GAGAACTTGA CTGGTTGCGA CAGAGAAAAA AAAAGTGTCC     2018

GAGTACTGAA GCCAAGGGTA CACAAGATGG ACTGGGTTGC GACCTGACGG CGCCCCCAGT     2078

GTGCTGGAGT GGGAAGGACG TGGCGCGCCT GGCTTTGGCG TGGAGCCAGA GAGCAGCGGC     2138

CTATTGGCCG GCAGACTTTG CGGACGGGCT GTGCCCGCGC GCGACCAGAA CGATGGACTT     2198

TTCGTTAACA TTGACCAAGA ACTGCATGGA CCTAACATTC GATCTCATTC AGTATTAAAG     2258

GGGGGTGGGA GGGGTTACAA ACTGCAATAG AGACTGTAGA TTGCTTCTGT AGTGCTCCTT 2318

AACACAAAGC AGGGAGGGCT GGGAAGGGGG GGAGGCTTGT AAGTGCCAGG CTAGACTGCA 2378

GATGAACTCC CCTGGCCTGC CTCTCTCAAC TGTGTATGTA CATATATATT TTTTTTTAAT 2438

TTGATGAAAG CTGATTACTG TCAATAAACA GCTTCCTGCC TTTGTAAGTT ATTCCATGTT 2498

TGTTTGTTTG GGTGTCCTGC CCAGTGTTTG TAAATAAGAG ATTTGAAGCA TTCTGAGTTT 2558

ACCATTTGTA ATAAAGTATA TAATTTTTTT ATGTTTTGTT TCTGAAAATT TCCAGAAAGG 2618

ATATTTAAGA AAATACAATA AACTATTGAA AAGTAGCCCC CAACCTCTTT GCTGCATTAT 2678

CCATAGATAA TGATAGCTAG ATGAAGTGAC AGCTGAGTGC CCCCAATATA CTAGGGTGAA 2738

AGCTGTGTCC CCTGTCTGAT TTGTAGGAAT AGATACCCTG CATGCTATCA TTGGCTCATA 2798

CTCTCTCCCC CGGCAACACA CAAGTCCAGA CTGTACACCA GAAGATGGTG TGGTGTTTCT 2858

TAAGGCTGGA AGAAGGGCTG TTGCAAGGGG AGAGGGTCAG CCCGCTGGAA AGCAGACACT 2918

TTGGTTGAAA GCTGTATGAA GTGGCATGTG CTGTGATCAT TTATAATCAT AGGAAAGATT 2978

TAGTAATTAG CTGTTGATTC TCAAAGCAGG GACCCATGGA AGTTTTTAAC AAAAGGTGTC 3038

TCCTTCCAAC TTTGAATCTG ACAACTCCTA GAAAAAGATG ACCTTTGCTT GTGCATATTT 3098

ATAATAGCGT TCGTTATCAC AATAAATGTA TTCAAAT 3135

(2) INFORMATION FOR SEQ ID NO:2:

(i) SEQUENCE CHARACTERISTICS:
(A) LENGTH: 334 amino acids
(B) TYPE: amino acid
(D) TOPOLOGY: linear

(ii) MOLECULE TYPE: protein

(xi) SEQUENCE DESCRIPTION: SEQ ID NO:2:

Met Thr Ala Lys Met Glu Thr Thr Phe Tyr Asp Asp Ala Leu Asn Ala
1               5                   10                  15

Ser Phe Leu Gln Ser Glu Ser Gly Ala Tyr Gly Tyr Ser Asn Pro Lys
            20                  25                  30

Ile Leu Lys Gln Ser Met Thr Leu Asn Leu Ala Asp Pro Val Gly Ser
        35                  40                  45

Leu Lys Pro His Leu Arg Ala Lys Asn Ser Asp Leu Leu Thr Ser Pro
    50                  55                  60

Asp Val Gly Leu Leu Lys Leu Ala Ser Pro Glu Leu Glu Arg Leu Ile
65                  70                  75                  80

Ile Gln Ser Ser Asn Gly His Ile Thr Thr Thr Pro Thr Pro Thr Gln
                85                  90                  95

Phe Leu Cys Pro Lys Asn Val Thr Asp Glu Gln Glu Gly Phe Ala Glu
            100                 105                 110

Gly Phe Val Arg Ala Leu Ala Glu Leu His Ser Gln Asn Thr Leu Pro
        115                 120                 125

Ser Val Thr Ser Ala Ala Gln Pro Val Ser Gly Ala Gly Met Val Ala
    130                 135                 140

Pro Ala Val Ala Ser Val Ala Gly Ala Gly Gly Gly Gly Gly Tyr Ser
145                 150                 155                 160

Ala Ser Leu His Ser Glu Pro Pro Val Tyr Ala Asn Leu Ser Asn Phe
                165                 170                 175

Asn Pro Gly Ala Leu Ser Ser Gly Gly Gly Ala Pro Ser Tyr Gly Ala
            180                 185                 190

Ala Gly Leu Ala Phe Pro Ser Gln Pro Gln Gln Gln Gln Gln Pro Pro
        195                 200                 205

Gln Pro Pro His His Leu Pro Gln Gln Ile Pro Val Gln His Pro Arg
    210                 215                 220

Leu Gln Ala Leu Lys Glu Glu Pro Gln Thr Val Pro Glu Met Pro Gly
225                 230                 235                 240

Glu Thr Pro Pro Leu Ser Pro Ile Asp Met Glu Ser Gln Glu Arg Ile
                245                 250                 255

Lys Ala Glu Arg Lys Arg Met Arg Asn Arg Ile Ala Ala Ser Lys Cys
            260                 265                 270

Arg Lys Arg Lys Leu Glu Arg Ile Ala Arg Leu Glu Glu Lys Val Lys
        275                 280                 285

Thr Leu Lys Ala Gln Asn Ser Glu Leu Ala Ser Thr Ala Asn Met Leu
    290                 295                 300

Arg Glu Gln Val Ala Gln Leu Lys Gln Lys Val Met Asn His Val Asn
305                 310                 315                 320

Ser Gly Cys Gln Leu Met Leu Thr Gln Gln Leu Gln Thr Phe
                325                 330

(2) INFORMATION FOR SEQ ID NO:3:

(i) SEQUENCE CHARACTERISTICS:
(A) LENGTH: 3622 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: mRNA
(B) LOCATION: 287..3622

(ix) FEATURE:
(A) NAME/KEY: mRNA
(B) LOCATION: 289..3622

(ix) FEATURE:
(A) NAME/KEY: mRNA
(B) LOCATION: 293..3622

(ix) FEATURE:
(A) NAME/KEY: CDS
(B) LOCATION: 1261..2256

(xi) SEQUENCE DESCRIPTION: SEQ ID NO:3:

CCCGGGGAGG GGACCGGGGA ACAGAGGGCC GAGAGGCGTG CGGCAGGGGG GAGGGTAGGA 60

GAAAGAAGGG CCCGACTGTA GGAGGGCAGC GGAGCATTAC CTCATCCCGT GAGCCTCCGC 120

GGGCCCAGAG AAGAATCTTC TAGGGTGGAG TCTCCATGGT GACGGGCGGG CCCGCCCCCC 180

TGAGAGCGAC GCGAGCCAAT GGGAAGGCCT TGGGGTGACA TCATGGGCTA TTTTTAGGGG 240

TTGACTGGTA GCAGATAAGT GTTGAGCTCG GGCTGGATAA GGGCTCAGAG TTGCACTGAG 300

TGTGGCTGAA GCAGCGAGGC GGGAGTGGAG GTGCGCGGAG TCAGGCAGAC AGACAGACAC 360

AGCCAGCCAG CCAGGTCGGC AGTATAGTCC GAACTGCAAA TCTTATTTTC TTTTCACCTT 420

CTCTCTAACT GCCCAGAGCT AGCGCCTGTG GCTCCCGGGC TGGTGGTTCG GGAGTGTCCA 480

GAGAGCCTTG TCTCCAGCCG GCCCCGGGAG GAGAGCCCTG CTGCCCAGGC GCTGTTGACA 540

GCGGCGGAAA GCAGCGGTAC CCCACGCGCC CGCCGGGGGA CGTCGGCGAG CGGCTGCAGC 600

AGCAAAGAAC TTTCCCGGCG GGGAGGACCG GAGACAAGTG GCAGAGTCCC GGAGCGAACT 660

TTTGCAAGCC TTTCCTGCGT CTTAGGCTTC TCCACGGCGG TAAAGACCAG AAGGCGGCGG 720

AGAGCCACGC AAGAGAAGAA GGACGTGCGC TCAGCTTCGC TCGCACCGGT TGTTGAACTT 780

GGGCGAGCGC GAGCCGCGGC TGCCGGGCGC CCCCTCCCCC TAGCAGCGGA GGAGGGGACA 840

AGTCGTCGGA GTCCGGGCGG CCAAGACCCG CCGCCGGCCG GCCACTGCAG GGTCCGCACT 900

GATCCGCTCC GCGGGGAGAG CCGCTGCTCT GGGAAGTGAG TTCGCCTGCG GACTCCGAGG 960

AACCGCTGCG CCCGAAGAGC GCTCAGTGAG TGACCGCGAC TTTTCAAAGC CGGGTAGCGC 1020

GCGCGAGTCG ACAAGTAAGA GTGCGGGAGG CATCTTAATT AACCCTGCGC TCCCTGGAGC 1080

GAGCTGGTGA GGAGGGCGCA GCGGGGACGA CAGCCAGCGG GTGCGTGCGC TCTTAGAGAA 1140

ACTTTCCCTG TCAAAGGCTC CGGGGGGCGC GGGTGTCCCC CGCTTGCCAG AGCCCTGTTG 1200

CGGCCCCGAA ACTTGTGCGC GCACGCCAAA CTAACCTCAC GTGAAGTGAC GGACTGTTCT 1260

ATG ACT GCA AAG ATG GAA ACG ACC TTC TAT GAC GAT GCC CTC AAC GCC 1308
Met Thr Ala Lys Met Glu Thr Thr Phe Tyr Asp Asp Ala Leu Asn Ala
1               5                   10                  15

TCG TTC CTC CCG TCC GAG AGC GGA CCT TAT GGC TAC AGT AAC CCC AAG 1356
Ser Phe Leu Pro Ser Glu Ser Gly Pro Tyr Gly Tyr Ser Asn Pro Lys
            20                  25                  30

ATC CTG AAA CAG AGC ATG ACC CTG AAC CTG GCC GAC CCA GTG GGG AGC 1404
Ile Leu Lys Gln Ser Met Thr Leu Asn Leu Ala Asp Pro Val Gly Ser
        35                  40                  45

CTG AAG CCG CAC CTC CGC GCC AAG AAC TCG GAC CTC CTC ACC TCG CCC 1452
Leu Lys Pro His Leu Arg Ala Lys Asn Ser Asp Leu Leu Thr Ser Pro
    50                  55                  60

GAC GTG GGG CTG CTC AAG CTG GCG TCG CCC GAG CTG GAG CGC CTG ATA 1500
Asp Val Gly Leu Leu Lys Leu Ala Ser Pro Glu Leu Glu Arg Leu Ile
65                  70                  75                  80

ATC CAG TCC AGC AAC GGG CAC ATC ACC ACC ACG CCG ACC CCC ACC CAG 1548
Ile Gln Ser Ser Asn Gly His Ile Thr Thr Thr Pro Thr Pro Thr Gln
                85                  90                  95

TTC CTG TGC CCC AAG AAC GTG ACA GAT GAG CAG GAG GGG TTC GCC GAG 1596
Phe Leu Cys Pro Lys Asn Val Thr Asp Glu Gln Glu Gly Phe Ala Glu
            100                 105                 110

GGC TTC GTG CGC GCC CTG GCC GAA CTG CAC AGC CAG AAC ACG CTG CCC 1644
Gly Phe Val Arg Ala Leu Ala Glu Leu His Ser Gln Asn Thr Leu Pro
        115                 120                 125

AGC GTC ACG TCG GCG GCG CAG CCG GTC AAC GGG GCA GGC ATG GTG GCT 1692
Ser Val Thr Ser Ala Ala Gln Pro Val Asn Gly Ala Gly Met Val Ala
    130                 135                 140

CCC GCG GTA GCC TCG GTG GCA GGG GGC AGC GGC AGC GGC GGC TTC AGC 1740
Pro Ala Val Ala Ser Val Ala Gly Gly Ser Gly Ser Gly Gly Phe Ser
145                 150                 155                 160

GCC AGC CTG CAC AGC GAG CCG CCG GTC TAC GCA AAC CTC AGC AAC TTC 1788
Ala Ser Leu His Ser Glu Pro Pro Val Tyr Ala Asn Leu Ser Asn Phe
                165                 170                 175

AAC CCA GGC GCG CTG AGC AGC GGC GGC GGG GCG CCC TCC TAC GGC GCG 1836
Asn Pro Gly Ala Leu Ser Ser Gly Gly Gly Ala Pro Ser Tyr Gly Ala
            180                 185                 190

GCC GGC CTG GCC TTT CCC GCG CAA CCC CAG CAG CAG CAG CAG CCG CCG 1884
Ala Gly Leu Ala Phe Pro Ala Gln Pro Gln Gln Gln Gln Gln Pro Pro
        195                 200                 205

CAC CAC CTG CCC CAG CAG ATG CCC GTG CAG CAC CCG CGG CTG CAG GCC 1932
His His Leu Pro Gln Gln Met Pro Val Gln His Pro Arg Leu Gln Ala
    210                 215                 220

CTG AAG GAG GAG CCT CAG ACA GTG CCC GAG ATG CCC GGC GAG ACA CCG 1980
Leu Lys Glu Glu Pro Gln Thr Val Pro Glu Met Pro Gly Glu Thr Pro
225                 230                 235                 240

CCC CTG TCC CCC ATC GAC ATG GAG TCC CAG GAG CGG ATC AAG GCG GAG 2028
Pro Leu Ser Pro Ile Asp Met Glu Ser Gln Glu Arg Ile Lys Ala Glu
                245                 250                 255

AGG AAG CGC ATG AGG AAC CGC ATC GCT GCC TCC AAG TGC CGA AAA AGG 2076
Arg Lys Arg Met Arg Asn Arg Ile Ala Ala Ser Lys Cys Arg Lys Arg
            260                 265                 270

AAG CTG GAG AGA ATC GCC CGG CTG GAG GAA AAA GTG AAA ACC TTG AAA 2124
Lys Leu Glu Arg Ile Ala Arg Leu Glu Glu Lys Val Lys Thr Leu Lys
        275                 280                 285

GCT CAG AAC TCG GAG CTG GCG TCC ACG GCC AAC ATG CTC AGG GAA CAG 2172
Ala Gln Asn Ser Glu Leu Ala Ser Thr Ala Asn Met Leu Arg Glu Gln
    290                 295                 300

GTG GCA CAG CTT AAA CAG AAA GTC ATG AAC CAC GTT AAC AGT GGG TGC     2220
Val Ala Gln Leu Lys Gln Lys Val Met Asn His Val Asn Ser Gly Cys
305                 310                 315                 320

CAA CTC ATG CTA ACG CAG CAG TTG CAA ACA TTT TGAAGAGAGA CCGTCGGGGG     2273
Gln Leu Met Leu Thr Gln Gln Leu Gln Thr Phe
                325                 330

CTGAGGGGCA ACGAAGAAAA AAAATAACAC AGAGAGACAG ACTTGAGAAC TTGACAAGTT     2333

GCGACGGAGA GAAAAAAGAA GTGTCCGAGA ACTAAAGCCA AGGGTATCCA AGTTGGACTG     2393

GGTTCGGTCT GACGGCGCCC CCAGTGTGCA CGAGTGGGAA GGACTTGGTC GCGCCCTCCC     2453

TTGGCGTGGA GCCAGGGAGC GGCCGCCTGC GGGCTGCCCC GCTTTGCGGA CGGGCTGTCC     2513

CCGCGCGAAC GGAACGTTGG ACTTTCGTTA ACATTGACCA AGAACTGCAT GGACCTAACA     2573

TTCGATCTCA TTCAGTATTA AAGGGGGGAG GGGGAGGGGG TTACAAACTG CAATAGAGAC     2633

TGTAGATTGC TTCTGTAGTA CTCCTTAAGA ACACAAAGCG GGGGGAGGGT TGGGGAGGGG     2693

CGGCAGGAGG GAGGTTTGTG AGAGCGAGGC TGAGCCTACA GATGAACTCT TTCTGGCCTG     2753

CTTTCGTTAA CTGTGTATGT ACATATATAT ATTTTTTAAT TTGATTAAAG CTGATTACTG     2813

TCAATAAACA GCTTCATGCC TTTGTAAGTT ATTTCTTGTT TGTTTGTTTG GGTATCCTGC     2873

CCAGTGTTGT TTGTAAATAA GAGATTTGGA GCACTCTGAG TTTACCATTT GTAATAAAGT     2933

ATATAATTTT TTTATGTTTT GTTTCTGAAA ATTCCAGAAA GGATATTTAA GAAAATACAA     2993

TAAACTATTG GAAAGTACTC CCCTAACCTC TTTTCTGCAT CATCTGTAGA TCCTAGTCTA     3053

TCTAGGTGGA GTTGAAAGAG TTAAGAATGC TCGATAAAAT CACTCTCAGT GCTTCTTACT     3113

ATTAAGCAGT AAAAACTGTT CTCTATTAGA CTTAGAAATA AATGTACCTG ATGTACCTGA     3173

TGCTATGTCA GGCTTCATAC TCCACGCTCC CCCAGCGTAT CTATATGGAA TTGCTTACCA     3233

AAGGCTAGTG CGATGTTTCA GGAGGCTGGA GGAAGGGGGG TTGCAGTGGA GAGGGACAGC     3293

CCACTGAGAA GTCAAACATT TCAAAGTTTG GATTGCATCA AGTGGCATGT GCTGTGACCA     3353

TTTATAATGT TAGAAATTTT ACAATAGGTG CTTATTCTCA AAGCAGGAAT TGGTGGCAGA     3413

TTTTACAAAA GATGTATCCT TCCAATTTGG AATCTTCTCT TTGACAATTC CTAGATAAAA     3473

AGATGGCCTT TGTCTTATGA ATATTTATAA CAGCATTCTG TCACAATAAA TGTATTCAAA     3533

TACCAATAAC AGATCTTGAA TTGCTTCCCT TTACTACTTT TTTGTTCCCA AGTTATATAC     3593

TGAAGTTTTT ATTTTTAGTT GCTGAGGTT                                       3622

(2) INFORMATION FOR SEQ ID NO:4:

      (i) SEQUENCE CHARACTERISTICS:
            (A) LENGTH: 331 amino acids
            (B) TYPE: amino acid
            (D) TOPOLOGY: linear

     (ii) MOLECULE TYPE: protein

(xi) SEQUENCE DESCRIPTION: SEQ ID NO:4:

Met Thr Ala Lys Met Glu Thr Thr Phe Tyr Asp Asp Ala Leu Asn Ala
1               5                   10                  15

Ser Phe Leu Pro Ser Glu Ser Gly Pro Tyr Gly Tyr Ser Asn Pro Lys
            20                  25                  30

Ile Leu Lys Gln Ser Met Thr Leu Asn Leu Ala Asp Pro Val Gly Ser
        35                  40                  45

Leu Lys Pro His Leu Arg Ala Lys Asn Ser Asp Leu Leu Thr Ser Pro
    50                  55                  60

Asp Val Gly Leu Leu Lys Leu Ala Ser Pro Glu Leu Glu Arg Leu Ile
65                  70                  75                  80

Ile Gln Ser Ser Asn Gly His Ile Thr Thr Thr Pro Thr Pro Thr Gln
                85                  90                  95

Phe Leu Cys Pro Lys Asn Val Thr Asp Glu Gln Glu Gly Phe Ala Glu
            100                 105                 110

Gly Phe Val Arg Ala Leu Ala Glu Leu His Ser Gln Asn Thr Leu Pro
        115                 120                 125

Ser Val Thr Ser Ala Ala Gln Pro Val Asn Gly Ala Gly Met Val Ala
    130                 135                 140

Pro Ala Val Ala Ser Val Ala Gly Gly Ser Gly Ser Gly Gly Phe Ser
145                 150                 155                 160

Ala Ser Leu His Ser Glu Pro Pro Val Tyr Ala Asn Leu Ser Asn Phe
                165                 170                 175

Asn Pro Gly Ala Leu Ser Ser Gly Gly Gly Ala Pro Ser Tyr Gly Ala
            180                 185                 190

Ala Gly Leu Ala Phe Pro Ala Gln Pro Gln Gln Gln Gln Gln Pro Pro
        195                 200                 205

His His Leu Pro Gln Gln Met Pro Val Gln His Pro Arg Leu Gln Ala
    210                 215                 220

Leu Lys Glu Glu Pro Gln Thr Val Pro Glu Met Pro Gly Glu Thr Pro
225                 230                 235                 240

Pro Leu Ser Pro Ile Asp Met Glu Ser Gln Glu Arg Ile Lys Ala Glu
                245                 250                 255

Arg Lys Arg Met Arg Asn Arg Ile Ala Ala Ser Lys Cys Arg Lys Arg
            260                 265                 270

Lys Leu Glu Arg Ile Ala Arg Leu Glu Glu Lys Val Lys Thr Leu Lys
        275                 280                 285

Ala Gln Asn Ser Glu Leu Ala Ser Thr Ala Asn Met Leu Arg Glu Gln
    290                 295                 300

Val Ala Gln Leu Lys Gln Lys Val Met Asn His Val Asn Ser Gly Cys
305                 310                 315                 320

Gln Leu Met Leu Thr Gln Gln Leu Gln Thr Phe
                325                 330

(2) INFORMATION FOR SEQ ID NO:5:

(i) SEQUENCE CHARACTERISTICS:
(A) LENGTH: 3548 base pairs
(B) TYPE: nucleic acid
(C) STRANDEDNESS: single
(D) TOPOLOGY: linear

(ix) FEATURE:
(A) NAME/KEY: TATA_signal
(B) LOCATION: 101..106

(ix) FEATURE:
(A) NAME/KEY: polyA_signal
(B) LOCATION: 3493..3498

(ix) FEATURE:
(A) NAME/KEY: exon
(B) LOCATION: 284..424

(ix) FEATURE:
(A) NAME/KEY: exon
(B) LOCATION: 1179..1430

(ix) FEATURE:
(A) NAME/KEY: exon
(B) LOCATION: 1836..1943

(ix) FEATURE:
(A) NAME/KEY: exon
(B) LOCATION: 2061..2702

(ix) FEATURE:
(A) NAME/KEY: precursor_RNA
(B) LOCATION: 133..2702

(ix) FEATURE:
(A) NAME/KEY: intron
(B) LOCATION: 425..1178

(ix) FEATURE:
(A) NAME/KEY: intron
(B) LOCATION: 1431..1835

(ix) FEATURE:
(A) NAME/KEY: intron
(B) LOCATION: 1944..2060

(xi) SEQUENCE DESCRIPTION: SEQ ID NO:5:

GAGTTGACGA CAGAGCGCCC GCAGAGGGCC TTGGGGCGCG CTTCCCCCCC CTTCCAGTTC 60

CGCCCAGTGA CGTAGGAAGT CCATCCATTC ACAGCGCTTC TATAAAGGCG CCAGCTGAGG 120

CGCCTACTAC TCCAACCGCG ACTGCAGCGA GCAACTGAGA AGACTGGATA GAGCCGGCGG 180

TTCCGCGAAC GAGCAGTGAC CGCGCTCCCA CCCAGCTCTG CTCTGCAGCT CCCACCAGTG 240

TCTACCCCTG GACCCCTTGC CGGGCTTTCC CCAAACTTCG ACCATGATGT TCTCGGGTTT 300

CAACGCCGAC TACGAGGCGT CATCCTCCCG CTGCAGTAGC GCCTCCCCGG CCGGGGACAG 360

CCTTTCCTAC TACCATTCCC CAGCCGACTC CTTCTCCAGC ATGGGCTCTC CTGTCAACAC 420

ACAGGTGAGT TTGGCTTTGT GTAGCCGCCA GGTCCGCGCT GAGGGTCGCC GTGGAGGAGA 480
CACTGGGGTG TGACTCGCAG GGGCGGGGGG GTCTTCCTTT TTCGCTCTGG AGGGAGACTG 540
GCGCGGTCAG AGCAGCCTTA GCCTGGGAAC CCAGGACTTG TCTGAGCGCG TGCACACTTG 600
TCATAGTAAG ACTTAGTGAC CCCTTCCCGC GCGGCAGGTT TATTCTGAGT GGCCTGCCTG 660
CATTCTTCTC TCGGCCGACT TGTTTCTGAG ATCAGCCGGG GCCAACAAGT CTCGAGCAAA 720
GAGTCGCTAA CTAGAGTTTG GGAGGCGGCA AACCGCGGCA ATCCCCCCTC CCGGGGCAGC 780
CTGGAGCAGG GAGGAGGGAG GAGGGAGGAG GGTGCTGCGG GCGGGTGTGT AAGGCAGTTT 840
CATTGATAAA AAGCGAGTTC ATTCTGGAGA CTCCGGAGCA GCGCCTGCGT CAGCGCAGAC 900
GTCAGGGATA TTTATAACAA ACCCCCTTTC GAGCGAGTGA TGCCGAAGGG ATAACGGGAA 960
CGCAGCAGTA GGATGGAGGA GAAAGGCTGC GCTGCGGAAT TCAAGGGAGG ATATTGGGAG 1020
AGCTTTTATC TCCGATGAGG TGCATACAGG AAGACATAAG CAGTCTCTGA CCGGAATGCT 1080
TCTCTCTCCC TGCTTCATGC GACACTAGGG CCACTTGCTC CACCTGTGTC TGGAACCTCC 1140
TCGCTCACCT CCGCTTTCCT CTTTTTGTTT TGTTTCAGGA CTTTTGCGCA GATCTGTCCG 1200
TCTCTAGTGC CAACTTTATC CCCACGGTGA CAGCCATCTC CACCAGCCCA GACCTGCAGT 1260
GGCTGGTGCA GCCCACTCTG GTCTCCTCCG TGGCCCCATC GCAGACCAGA GCGCCCCATC 1320
CTTACGGACT CCCCACCCAG TCTGCTGGGG CTTACGCCAG AGCGGGAATG GTGAAGACCG 1380
TGTCAGGAGG CAGAGCGCAG AGCATCGGCA GAAGGGGCAA AGTAGAGCAG GTGAGCAGCG 1440
ATTCTGGACC TTTGTGGGCT GGGGGGGGGG GGGGGGGCGG AGACTGACGC ACAGACCACA 1500
CAACAGAGAA GGGACGCTAC TGACTGCACT TCCTGACCAG GAGCTGTGGC TGCTAGCCCT 1560
TTCCCTCCCT TGTCAGATTT TGACAGTTGG ACCCAAGACA AACTCTAGAC AGTTTCCCTG 1620
ACAGCTTCCT ACTTCATTCT CTAGCCGGGG AGCTTCTTTG TTCCCCTGCT AAAGATCTCA 1680
CTTTAAATGC AAATCACACT CTGCCTGCCA ACTGCAGGTT AGAAAAACTG CTTCACCGAG 1740
AGGTGCGGGT GCTGTAGGAG CCAGTTTCAC TGGGGTGACT GAATGGAGGT GACACTAGAC 1800
AACCTTAACT GAATGTTGGT CCTTTTCTTC TATAGCTATC TCCTGAAGAG GAAGAGAAAC 1860
GGAGAATCCG AAGGGAACGG AATAAGATGG CTGCAGCCAA GTGCCGGAAT CGGAGGAGGG 1920
AGCTGACAGA TACACTCCAA GCGGTAGGTT GAACCAGCTG CTGCTCCTGA AACTTTATTA 1980
AAGTTGGAGC TTGGGACTAT GGGCGCAGGG TCCTTGAGCA TGCCCGTGTC TTATGCTTTC 2040
TTATATCTCT CCCTATGCAG GAGACAGATC AACTTGAAGA TGAGAAGTCT GCGTTGCAGA 2100
CTGAGATTGC CAATCTGCTG AAAGAGAAGG AAAAACTGGA GTTTATTTTG GCAGCCCACC 2160
GACCTGCCTG CAAGATCCCC GATGACCTTG GCTTCCCAGA GGAGATGTCT GTGGCCTCCC 2220
TGGATTTGAC TGGAGGTCTG CCTGAGGCTT CCACCCCAGA GTCTGAGGAG GCCTTCACCC 2280
TGCCCCTTCT CAACGACCCT GAGCCCAAGC CATCCTTGGA GCCAGTCAAG AGCATCAGCA 2340
ACGTGGAGCT GAAGGCAGAA CCCTTTGATG ACTTCTTGTT TCCGGCATCA TCTAGGCCCA 2400

GTGGCTCAGA GACCTCCCGC TCTGTGCCAG ATGTGGACCT GTCCGGTTCC TTCTATGCAG 2460

CAGACTGGGA GCCTCTGCAC AGCAATTCCT TGGGGATGGG GCCCATGGTC ACAGAGCTGG 2520

AGCCCCTGTG TACTCCCGTG GTCACCTGTA CTCCGGGCTG CACTACTTAC ACGTCTTCCT 2580

TTGTCTTCAC CTACCCTGAA GCTGACTCCT TCCCAAGCTG TGCCGCTGCC CACCGAAAGG 2640

GCAGCAGCAG CAACGAGCCC TCCTCCGACT CCCTGAGCTC ACCCACGCTG CTGGCCCTGT 2700

GAGCAGTCAG AGAAGGCAAG GCAGCCGGCA TCCAGACGTG CCACTGCCCG AGCTGGTGCA 2760

TTACAGAGAG GAGAAACACG TCTTCCCTCG AAGGTTCCCG TCGACCTAGG GAGGACCTTA 2820

CCTGTTCGTG AAACACACCA GGCTGTGGGC CTCAAGGACT TGCAAGCATC CACATCTGGC 2880

CTCCAGTCCT CACCTCTTCC AGAGATGTAG CAAAAACAAA ACAAAACAAA ACAAAAAACC 2940

GCATGGAGTG TGTTGTTCCT AGTGACACCT GAGAGCTGGT AGTTAGTAGA GCATGTGAGT 3000

CAAGGCCTGG TCTGTGTCTC TTTTCTCTTT CTCCTTAGTT TTCTCATAGC ACTAACTAAT 3060

CTGTTGGGTT CATTATTGGA ATTAACCTGG TGCTGGATTG TATCTAGTGC AGCTGATTTT 3120

AACAATACCT ACTGTGTTCC TGGCAATAGC GTGTTCCAAT TAGAAACGAC CAATATTAAA 3180

CTAAGAAAAG ATAGGACTTT ATTTTCCAGT AGATAGAAAT CAATAGCTAT ATCCATGTAC 3240

TGTAGTCCTT CAGCGTCAAT GTTCATTGTC ATGTTACTGA TCATGCATTG TCGAGGTGGT 3300

CTGAATGTTC TGACATTAAC AGTTTTCCAT GAAAACGTTT TTATTGTGTT TTCAATTTAT 3360

TTATTAAGAT GGATTCTCAG ATATTTATAT TTTTATTTTA TTTTTTTCTA CCCTGAGGTC 3420

TTTCGACATG TGGAAAGTGA ATTTGAATGA AAAATTTTAA GCATTGTTTG CTTATTGTTC 3480

CAGGACATTG TCAATAAAAG CATTTAAGTT GAATGCGACC ACCTTCTTGC TCTCTTTATT 3540

CTCAGTTT 3548

(2) INFORMATION FOR SEQ ID NO:6:

(i) SEQUENCE CHARACTERISTICS:
(A) LENGTH: 6210 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: CDS
(B) LOCATION: join(889..1029, 1783..2034, 2466..2573, 2688
..3329)

(ix) FEATURE:
(A) NAME/KEY: misc_feature
(B) LOCATION: 402..453
(D) OTHER INFORMATION: /note= "transcriptional activator
region"

(ix) FEATURE:
(A) NAME/KEY: prim_transcript
(B) LOCATION: 734..3329

(ix) FEATURE:
(A) NAME/KEY: exon
(B) LOCATION: 889..1029
(D) OTHER INFORMATION: /note= "c-fos protein, exon1"

(ix) FEATURE:
(A) NAME/KEY: intron
(B) LOCATION: 1030..1782
(D) OTHER INFORMATION: /note= "c-fos, intron A"

(ix) FEATURE:
(A) NAME/KEY: exon
(B) LOCATION: 1783..2034

(ix) FEATURE:
(A) NAME/KEY: intron
(B) LOCATION: 2035..2465

(ix) FEATURE:
(A) NAME/KEY: exon
(B) LOCATION: 2466..2573

(ix) FEATURE:
(A) NAME/KEY: intron
(B) LOCATION: 2574..2687

(ix) FEATURE:
(A) NAME/KEY: exon
(B) LOCATION: 2688..3329

(xi) SEQUENCE DESCRIPTION: SEQ ID NO:6:

GCAGGAACAG TGCTAGTATT GCTCGAGCCC GAGGGCTGGA GGTTAGGGGA TGAAGGTCTG 60
CTTCCACGCT TTGCACTGAA TTAGGGCTAG AATTGGGGAT GGGGGTAGGG GCGCATTCCT 120
TCGGGAGCCG AGGCTTAAGT CCTCGGGGTC CTGTACTCGA TGCCGTTTCT CCTATCTCTG 180
AGCCTCAGAA CTGTCTTCAG TTTCCGTACA AGGGTAAAAA GGCGCTCTCT GCCCCATCCC 240
CCCCGACCTC GGGAACAAGG GTCCGCATTG AACCAGGTGC GAATGTTCTC TCTCATTCTG 300
CGCCGTTCCC GCCTCCCCTC CCCCAGCCGC GGCCCCCGCC TCCCCCCGCA CTGCACCCTC 360
GGTGTTGGCT GCAGCCCGCG AGCAGTTCCC GTCAATCCCT CCCCCCTTAC ACAGGATGTC 420
CATATTAGGA CATCTGCGTC AGCAGGTTTC CACGGCCTTT CCCTGTAGCC CTGGGGGGAG 480
CCATCCCCGA AACCCCTCAT CTTGGGGGGC CCACGAGACC TCTGAGACAG GAACTGCGAA 540
ATGCTCACGA GATTAGGACA CGCGCCAAGG CGGGGGCAGG GAGCTGCGAG CGCTGGGGAC 600
GCAGCCGGGC GGCCGCAGAA GCGCCCAGGC CCGCGCGCCA CCCCTCTGGC GCCACCGTGG 660
TTGAGCCCGT GACGTTTACA CTCATTCATA AAACGCTTGT TATAAAAGCA GTGGCTGCGG 720
CGCCTCGTAC TCCAACCGCA TCTGCAGCGA GCAACTGAGA AGCCAAGACT GAGCCGGCGG 780
CCGCGGCGCA GCGAACGAGC AGTGACCGTG CTCCTACCCA GCTCTGCTTC ACAGCGCCCA 840

CCTGTCTCCG CCCCTCGGCC CCTCGCCCGG CTTTGCCTAA CCGCCACG ATG ATG TTC 897
Met Met Phe
1

TCG GGC TTC AAC GCA GAC TAC GAG GCG TCA TCC TCC CGC TGC AGC AGC 945
Ser Gly Phe Asn Ala Asp Tyr Glu Ala Ser Ser Ser Arg Cys Ser Ser
    5                   10                  15

GCG TCC CCG GCC GGG GAT AGC CTC TCT TAC TAC CAC TCA CCC GCA GAC 993
Ala Ser Pro Ala Gly Asp Ser Leu Ser Tyr Tyr His Ser Pro Ala Asp
20                  25                  30                  35

TCC TTC TCC AGC ATG GGC TCG CCT GTC AAC GCG CAG GTAAGGCTGG 1039
Ser Phe Ser Ser Met Gly Ser Pro Val Asn Ala Gln
                40                  45

CTTCCCGTCG CCGCGGGGCC GGGGGCTTGG GGTCGCGGAG GAGGAGACAC CGGGCGGGAC 1099

GCTCCAGTAG ATGAGTAGGG GGCTCCCTTG TGCCTGGAGG GAGGCTGCCG TGGCCGGAGC 1159

GGTGCCGGCT CGGGGGCTCG GGACTTGCTC TGAGCGCACG CACGCTTGCC ATAGTAAGAA 1219

TTGGTTCCCC CTTCGGGAGG CAGGTTCGTT CTGAGCAACC TCTGGTCTGC ACTCCAGGAC 1279

GGATCTCTGA CATTAGCTGG AGCAGACGTG TCCCAAGCAC AAACTCGCTA ACTAGAGCCT 1339

GGCTTCTTCG GGGAGGTGGC AGAAAGCGGC AATCCCCCCT CCCCCGGCAG CCTGGAGCAC 1399

GGAGGAGGGA TGAGGGAGGA GGGTGCAGCG GGCGGGTGTG TAAGGCAGTT TCATTGATAA 1459

AAAGCGAGTT CATTCTGGAG ACTCCGGAGC GGCGCCTGCG TCAGCGCAGA CGTCAGGGAT 1519

ATTTATAACA AACCCCCTTT CAAGCAAGTG ATGCTGAAGG GATAACGGGA ACGCAGCGGC 1579

AGGATGGAAG AGACAGGCAC TGCGCTGCGG AATGCCTGGG AGGAAAAGGG GGAGACCTTT 1639

CATCCAGGAT GAGGGACATT TAAGATGAAA TGTCCGTGGC AGGATCGTTT CTCTTCACTG 1699

CTGCATGCGG CACTGGGAAC TCGCCCCACC TGTGTCCGGA ACCTGCTCGC TCACGTCGGC 1759

TTTCCCCTTC TGTTTTGTTC TAG GAC TTC TGC ACG GAC CTG GCC GTC TCC 1809
Asp Phe Cys Thr Asp Leu Ala Val Ser
        50                  55

AGT GCC AAC TTC ATT CCC ACG GTC ACT GCC ATC TCG ACC AGT CCG GAC 1857
Ser Ala Asn Phe Ile Pro Thr Val Thr Ala Ile Ser Thr Ser Pro Asp
            60                  65                  70

CTG CAG TGG CTG GTG CAG CCC GCC CTC GTC TCC TCT GTG GCC CCA TCG 1905
Leu Gln Trp Leu Val Gln Pro Ala Leu Val Ser Ser Val Ala Pro Ser
        75                  80                  85

CAG ACC AGA GCC CCT CAC CCT TTC GGA GTC CCC GCC CCC TCC GCT GGG 1953
Gln Thr Arg Ala Pro His Pro Phe Gly Val Pro Ala Pro Ser Ala Gly
    90                  95                  100

GCT TAC TCC AGG GCT GGC GTT GTG AAG ACC ATG ACA GGA GGC CGA GCG 2001
Ala Tyr Ser Arg Ala Gly Val Val Lys Thr Met Thr Gly Gly Arg Ala
105                 110                 115                 120

CAG AGC ATT GGC AGG AGG GGC AAG GTG GAA CAG GTGAGGAACT CTAGCGTACT 2054
Gln Ser Ile Gly Arg Arg Gly Lys Val Glu Gln
                125                 130

CTTCCTGGGA ATGTGGGGGC TGGGTGGGAA GCAGCCCCGG AGATGCAGGA GCCCAGTACA 2114

GAGGATGAAG CCACTGATGG GGCTGGCTGC ACATCCGTAA CTGGGAGCCC TGGCTCCAAG 2174

CCCATTCCAT CCCAACTCAG ACTCTGAGTC TCACCCTAAG AAGTACTCTC ATAGTTTCTT 2234

CCCTAAGTTT CTTACCGCAT GCTTTCAGAC TGGGCTCTTC TTTGTTCTCT TGCTGAGGAT 2294

CTTATTTTAA ATGCAAGTCA CACCTATTCT GCAACTGCAG GTCAGAAATG GTTTCACAGT 2354

GGGGTGCCAG GAAGCAGGGA AGCTGCAGGA GCCAGTTCTA CTGGGGTGGG TGAATGGAGG 2414

TGATGGCAGA CACTTTTACT GAATGTCGGT CTTTTTTTGT GATTATTCTA G TTA TCT 2471
Leu Ser

CCA GAA GAA GAA GAG AAA AGG AGA ATC CGA AGG GAA AGG AAT AAG ATG 2519
Pro Glu Glu Glu Glu Lys Arg Arg Ile Arg Arg Glu Arg Asn Lys Met
    135                 140                 145

GCT GCA GCC AAA TGC CGC AAC CGG AGG AGG GAG CTG ACT GAT ACA CTC 2567
Ala Ala Ala Lys Cys Arg Asn Arg Arg Arg Glu Leu Thr Asp Thr Leu
150                 155                 160                 165

CAA GCG GTAGGTACTC TGTGGGTTGC TCCTTTTTAA AACTTAAGGG AAAGTTGGAG 2623
Gln Ala

ATTGAGCATA AGGGCCCTTG AGTAAGACTG TGTCTTATGC TTTCCTTTAT CCCTCTGTAT 2683

ACAG GAG ACA GAC CAA CTA GAA GAT GAG AAG TCT GCT TTG CAG ACC GAG 2732
Glu Thr Asp Gln Leu Glu Asp Glu Lys Ser Ala Leu Gln Thr Glu
        170                 175                 180

ATT GCC AAC CTG CTG AAG GAG AAG GAA AAA CTA GAG TTC ATC CTG GCA 2780
Ile Ala Asn Leu Leu Lys Glu Lys Glu Lys Leu Glu Phe Ile Leu Ala
        185                 190                 195

GCT CAC CGA CCT GCC TGC AAG ATC CCT GAT GAC CTG GGC TTC CCA GAA 2828
Ala His Arg Pro Ala Cys Lys Ile Pro Asp Asp Leu Gly Phe Pro Glu
    200                 205                 210

GAG ATG TCT GTG GCT TCC CTT GAT CTG ACT GGG GGC CTG CCA GAG GTT 2876
Glu Met Ser Val Ala Ser Leu Asp Leu Thr Gly Gly Leu Pro Glu Val
215                 220                 225                 230

GCC ACC CCG GAG TCT GAG GAG GCC TTC ACC CTG CCT CTC CTC AAT GAC 2924
Ala Thr Pro Glu Ser Glu Glu Ala Phe Thr Leu Pro Leu Leu Asn Asp
                235                 240                 245

CCT GAG CCC AAG CCC TCA GTG GAA CCT GTC AAG AGC ATC AGC AGC ATG 2972
Pro Glu Pro Lys Pro Ser Val Glu Pro Val Lys Ser Ile Ser Ser Met
            250                 255                 260

GAG CTG AAG ACC GAG CCC TTT GAT GAC TTC CTG TTC CCA GCA TCA TCC 3020
Glu Leu Lys Thr Glu Pro Phe Asp Asp Phe Leu Phe Pro Ala Ser Ser
        265                 270                 275

AGG CCC AGT GGC TCT GAG ACA GCC CGC TCC GTG CCA GAC ATG GAC CTA 3068
Arg Pro Ser Gly Ser Glu Thr Ala Arg Ser Val Pro Asp Met Asp Leu
    280                 285                 290

TCT GGG TCC TTC TAT GCA GCA GAC TGG GAG CCT CTG CAC AGT GGC TCC 3116
Ser Gly Ser Phe Tyr Ala Ala Asp Trp Glu Pro Leu His Ser Gly Ser
295                 300                 305                 310

CTG GGG ATG GGG CCC ATG GCC ACA GAG CTG GAG CCC CTG TGC ACT CCG 3164
Leu Gly Met Gly Pro Met Ala Thr Glu Leu Glu Pro Leu Cys Thr Pro
                315                 320                 325

GTG GTC ACC TGT ACT CCC AGC TGC ACT GCT TAC ACG TCT TCC TTC GTC     3212
Val Val Thr Cys Thr Pro Ser Cys Thr Ala Tyr Thr Ser Ser Phe Val
            330                 335                 340

TTC ACC TAC CCC GAG GCT GAC TCC TTC CCC AGC TGT GCA GCT GCC CAC     3260
Phe Thr Tyr Pro Glu Ala Asp Ser Phe Pro Ser Cys Ala Ala Ala His
        345                 350                 355

CGC AAG GGC AGC AGC AGC AAT GAG CCT TCC TCT GAC TCG CTC AGC TCA     3308
Arg Lys Gly Ser Ser Ser Asn Glu Pro Ser Ser Asp Ser Leu Ser Ser
    360                 365                 370

CCC ACG CTG CTG GCC CTG TGAGGGGGCA GGGAAGGGGA GGCAGCCGGC            3356
Pro Thr Leu Leu Ala Leu
375                 380

ACCCACAAGT GCCACTGCCC GAGCTGGTGC ATTACAGAGA GGAGAAACAC ATCTTCCCTA    3416

GAGGGTTCCT GTAGACCTAG GGAGGACCTT ATCTGTGCGT GAAACACACC AGGCTGTGGG    3476

CCTCAAGGAC TTGAAAGCAT CCATGTGTGG ACTCAAGTCC TTACCTCTTC CGGAGATGTA    3536

GCAAAACGCA TGGAGTGTGT ATTGTTCCCA GTGACACTTC AGAGAGCTGG TAGTTAGTAG    3596

CATGTTGAGC CAGGCCTGGG TCTGTGTCTC TTTTCTCTTT CTCCTTAGTC TTCTCATAGC    3656

ATTAACTAAT CTATTGGGTT CATTATTGGA ATTAACCTGG TGCTGGATAT TTTCAAATTG    3716

TATCTAGTGC AGCTGATTTT AACAATAACT ACTGTGTTCC TGGCAATAGT GTGTTCTGAT    3776

TAGAAATGAC CAATATTATA CTAAGAAAAG ATACGACTTT ATTTTCTGGT AGATAGAAAT    3836

AAATAGCTAT ATCCATGTAC TGTAGTTTTT CTTCAACATC AATGTTCATT GTAATGTTAC    3896

TGATCATGCA TTGTTGAGGT GGTCTGAATG TTCTGACATT AACAGTTTTC CATGAAAACG    3956

TTTTATTGTG TTTTTAATTT ATTTATTAAG ATGGATTCTC AGATATTTAT ATTTTTATTT    4016

TATTTTTTTC TACCTTGAGG TCTTTTGACA TGTGGAAAGT GAATTTGAAT GAAAAATTTA    4076

AGCATTGTTT GCTTATTGTT CCAAGACATT GTCAATAAAA GCATTTAAGT TGAATGCGAC    4136

CAACCTTGTG CTCTTTTCAT TCTGGAAGTC TTGTAAGTTT CTGAAAGGTA TTATTGGAGA    4196

CCAGTTTGTC AAGAAGGGTA GCTGCTGGAG GGGGACACAC CCTCTGTCTG ATCCCTTATC    4256

AAAGAGGACA AGGAAACTAT AGAGCTGATT TTAGAATATT TTACAAATAC ATGCCTTCCA    4316

TTGGAATGCT AAGATTTTCT ACTGCTTCTG GGGACGGGAA ACCGCTGTGT AACAGCTTTT    4376

GTGGGAATAC ATTTTTTCTG TTTCAGTACT CGCAGGGGGA AATATTTAAA TTTTGTTGTG    4436

CTAATATTAA ATTCAGATGT TTTGATCTTA AAGGAACCCT TTAAGCAAAC AGAACCTAGC    4496

TTTGTACAGA CTATTTTAAC TTTTTATTCT CACAAAATCA CGTGGAGGGT TATTCTACTT    4556

CAAAGATGAG CAAATTGAAG AATGGTTAGA ATAAACAACT TTCTTGATAT TCCGTTATCG    4616

GCATTAGAAT CTTCCTGCTC GTTATCGTAT CCAGCAGGCT GAACTGCCTC TTGATACTTG    4676

GTTAAAAAAA ATTTTCAGGC CGGGCGCGGT GGCCCATGCC TGTAATCCTA GCACTTTGGG    4736

AGGCCGAGGC AGGCGGATCA CCTGAGGTCG GGAGTTCGAG ACCAGCCTGA CCAACATGGA    4796

GAAACCCCGT CTTTACTAAA AATACAAAAT TAGCCTGGTG TGGTGGTGCA TGCCTGTAAT    4856

CCTAGCTACT TGAGAGGCTG AGACAGGAAA ATCACTTGAA CTCGGGAGGC GGATGTTGCA 4916

GCGAACTGAG ATTGCGCCAT TGCACTCCAG CCTGGGCAAC AAGATTGAAA CTCTGTTTAA 4976

AAAAAAAAGT TTTCACTAAT GTGTACATTT TTTTGTACTC TTTTATTCTC GAAAGGGAAG 5036

GAGGGCTATT GCCCTATCCC TTATTAATAA ATGCATTGTG GTTTCTGGTT TCTCTAATAC 5096

CATATGCCCT TCATTCAGTT TATAGTGGGC GGAAGTGGGG GAGAAAAAGT TGCTCAGAAA 5156

TCAAAAGATA TCTCAAACAG CACAAATAAT GGCTGATCGT TCTGCAAACA AAAAGTTACA 5216

TAATAGCTCA AGAAGGAGAA GTCAACATGA CTCTGAACAA GCTTTAACTT AGAAACTTTA 5276

TCATCTTAAG GAAGAACGTG ACCTTTGTCC AGGACGTCTC TGGTAATGGG GCACTTACAC 5336

ACACATGCAC ACGTACAAAC CACAGGGAAA GGAGACCGCC CTTCTGCCTC TGCTCGCGAG 5396

TATCACGCAG GCACCATGCA CTATGTTTTC ACACACACTG GGTGGAAGAA GAGCTTCAGC 5456

GCCAGTCTTC TAATGCTTTG GTGATAATGA AAATCACTGG GTGCTTATGG GGTGTCATAT 5516

TCAATCGAGT TAAAAGTTTT AATTCAAAAT GACAGTTTTA CTGAGGTTGA TGTTCTCGTC 5576

TATGATATCT CTGCCCCTCC CATAAAAATG GACATTTAAA AGCAACTTAC CGCTCTTTAG 5636

ATCACTCCTA TATCACACAC CACTTGGGGT GCTGTTTCTG CTAGACTTGT GATGACAGTG 5696

GCCTTAGGAT CCCTGTTTGC TGTTCAAAGG GCAAATATTT TATAGCCTTT AAATATACCT 5756

AAACTAAATA CAGAATTAAT ATAACTAACA AACACCTGGT CTGAAATAAC AAGGTGATCT 5816

ACCCTGGAAG GAACCCAGCT GGTGGGCCAG GAGCGGTGGC TCACACCTGT AATTCCAGCA 5876

CTTTGGGAGG CTGAGACAGG AGGATCACTG GAGTCCAGGA GTTTGAGACC AGCCTGGGCA 5936

ACATGGCAAA ACCCAGTGTG CTTCTGTTGT CCCAGCTACA CTACTCAGGA GGCTGAGGCA 5996

GGAGTATGAC TTGAGCCTGG GAGGGGGAGG TTGCAGAGAA CTGATATTGC ACCACCACTG 6056

CACTCCAGCC TGGGTGACAC AGCAAAACCC TATCTCAAAA AAAAAAAAAA AAAAAAGGAA 6116

CCCAGCTGGT TCCTGTAGGT GTGCAATAAT AACAACCAGA GGAAGAAAAG GAAGACGATT 6176

TCCCAGATGA AGAAGGGCAG CTGGACCTTC GGAC 6210

(2) INFORMATION FOR SEQ ID NO:7:

(i) SEQUENCE CHARACTERISTICS:
(A) LENGTH: 380 amino acids
(B) TYPE: amino acid
(D) TOPOLOGY: linear

(ii) MOLECULE TYPE: protein

(xi) SEQUENCE DESCRIPTION: SEQ ID NO:7:

Met Met Phe Ser Gly Phe Asn Ala Asp Tyr Glu Ala Ser Ser Ser Arg
1               5                   10                  15

Cys Ser Ser Ala Ser Pro Ala Gly Asp Ser Leu Ser Tyr Tyr His Ser
            20                  25                  30

Pro Ala Asp Ser Phe Ser Ser Met Gly Ser Pro Val Asn Ala Gln Asp
        35                  40                  45

Phe Cys Thr Asp Leu Ala Val Ser Ser Ala Asn Phe Ile Pro Thr Val
    50                  55                  60

Thr Ala Ile Ser Thr Ser Pro Asp Leu Gln Trp Leu Val Gln Pro Ala
65                  70                  75                  80

Leu Val Ser Ser Val Ala Pro Ser Gln Thr Arg Ala Pro His Pro Phe
                85                  90                  95

Gly Val Pro Ala Pro Ser Ala Gly Ala Tyr Ser Arg Ala Gly Val Val
            100                 105                 110

Lys Thr Met Thr Gly Gly Arg Ala Gln Ser Ile Gly Arg Arg Gly Lys
        115                 120                 125

Val Glu Gln Leu Ser Pro Glu Glu Glu Glu Lys Arg Arg Ile Arg Arg
    130                 135                 140

Glu Arg Asn Lys Met Ala Ala Ala Lys Cys Arg Asn Arg Arg Arg Glu
145                 150                 155                 160

Leu Thr Asp Thr Leu Gln Ala Glu Thr Asp Gln Leu Glu Asp Glu Lys
                165                 170                 175

Ser Ala Leu Gln Thr Glu Ile Ala Asn Leu Leu Lys Glu Lys Glu Lys
            180                 185                 190

Leu Glu Phe Ile Leu Ala Ala His Arg Pro Ala Cys Lys Ile Pro Asp
        195                 200                 205

Asp Leu Gly Phe Pro Glu Glu Met Ser Val Ala Ser Leu Asp Leu Thr
    210                 215                 220

Gly Gly Leu Pro Glu Val Ala Thr Pro Glu Ser Glu Glu Ala Phe Thr
225                 230                 235                 240

Leu Pro Leu Leu Asn Asp Pro Glu Pro Lys Pro Ser Val Glu Pro Val
                245                 250                 255

Lys Ser Ile Ser Ser Met Glu Leu Lys Thr Glu Pro Phe Asp Asp Phe
            260                 265                 270

Leu Phe Pro Ala Ser Ser Arg Pro Ser Gly Ser Glu Thr Ala Arg Ser
        275                 280                 285

Val Pro Asp Met Asp Leu Ser Gly Ser Phe Tyr Ala Ala Asp Trp Glu
    290                 295                 300

Pro Leu His Ser Gly Ser Leu Gly Met Gly Pro Met Ala Thr Glu Leu
305                 310                 315                 320

Glu Pro Leu Cys Thr Pro Val Val Thr Cys Thr Pro Ser Cys Thr Ala
                325                 330                 335

Tyr Thr Ser Ser Phe Val Phe Thr Tyr Pro Glu Ala Asp Ser Phe Pro
            340                 345                 350

Ser Cys Ala Ala Ala His Arg Lys Gly Ser Ser Ser Asn Glu Pro Ser
        355                 360                 365

Ser Asp Ser Leu Ser Ser Pro Thr Leu Leu Ala Leu
    370                 375                 380

(2) INFORMATION FOR SEQ ID NO:8:

(i) SEQUENCE CHARACTERISTICS:
(A) LENGTH: 49 base pairs
(B) TYPE: nucleic acid
(C) STRANDEDNESS: double
(D) TOPOLOGY: linear

(xi) SEQUENCE DESCRIPTION: SEQ ID NO:8:

GGCAGTGAGG TCAGGGGTGG GGAAGCCCAG GGCTGGGGAT TCCCCATCT 49

SEQUENCE LISTING

(1) GENERAL INFORMATION:

(i) APPLICANT:
(A) NAME: Yeda Research and Development Co., Ltd.
(B) STREET: P.O. Box 95
(C) CITY: Rehovot
(E) COUNTRY: Israel
(F) POSTAL CODE (ZIP): 76100

(ii) TITLE OF INVENTION: Anti-Metastatic Vaccine

(iii) NUMBER OF SEQUENCES: 8

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(v) CURRENT APPLICATION DATA:
APPLICATION NUMBER: EP 93 11 7519.4
(vi) PRIOR APPLICATION DATA:
(A) APPLICATION NUMBER: US 968,415
(B) FILING DATE: 29-OCT-1992

(2) INFORMATION FOR SEQ ID NO: 1:

(i) SEQUENCE CHARACTERISTICS:
(A) LENGTH: 3135 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: mRNA
(B) LOCATION: 1..3135

(ix) FEATURE:
(A) NAME/KEY: CDS
(B) LOCATION: 917..1918

(xi) SEQUENCE DESCRIPTION: SEQ ID NO: 1:

CTGAGTGTGC GAGAGACAGC CTGGCAGGAG AGCGCTCAGG CAGACAGACA GACAGACGGA 60

CGGACTTGGC CAACCCGGTC GGCCGCGGAC TCCGGACTGT TCATCCGTTT GTCTTCATTT 120

TCTCACCAAC TGCTTGGATC CAGCGCCCGC GGCTCCTGCA CCGGTATTTT GGGGAGCATT 180

TGGAGAGTCC CTTCTCCCGC CTTCCACGGA GAAGAAGCTC ACAAGTCCGG GCGCTGCTGA 240

CAGCATCGAG AGCGGCTCCC GACCGCGCGA GGAAATAGGC GAGCGGCTAC CGGCCAGCAA 300

CTTTCCTGAC CCAGAGGACC GGTAACAAGT GGCCGGGAGC GAACTTTTGC AAATCTCTTC 360

TGCGCCTTAA GGCTGCCACC GAGACTGTAA AGAAAAGGGA GAAGAGGAAC CTATACTCAT 420

ACCAGTTCGC ACAGGCGGCT GAAGTTGGGC GAGCGCTAGC CGCGGCTGCC TAGCGTCCCC 480

CTCCCCCTCA CAGCGGAGGA GGGGACAGTT GTTGGAGGCC GGGCGGCAGA GCCCGATCGC 540

GGGCTTCCAC CGAGAATTCC GTGACGACTG GTCAGCACCG CCGGAGAGCC GCTGTTGCTG 600

GGACTGGTCT GCGGGCTCCA AGGAACCGCT GCTCCCCGAG AGCGCTCCGT GAGTGACCGC 660

GACTTTTCAA AGCTCGGCAT CGCGCGGGAG CCTACCAACG TGAGTGCTAG CGGAGTCTTA 720

ACCCTGCGCT CCCTGGAGCG AACTGGGGAG GAGGGCTCAG GGGGAAGCAC TGCCGTCTGG 780

AGCGCACGCT CCTAAACAAA CTTTGTTACA GAAGCAGGGA CGCGCGGGTA TCCCCCCGCT 840

TCCCGGCGCG CTGTTGCGGC CCCGAAACTT CTGCGCACAG CCCAGGCTAA CCCCGCGTGA 900

AGTGACGGAC CGTTCT ATG ACT GCA AAG ATG GAA ACG ACC TTC TAC GAC 949
Met Thr Ala Lys Met Glu Thr Thr Phe Tyr Asp
1               5                   10

GAT GCC CTC AAC GCC TCG TTC CTC CAG TCC GAG AGC GGT GCC TAC GGC 997
Asp Ala Leu Asn Ala Ser Phe Leu Gln Ser Glu Ser Gly Ala Tyr Gly
            15                  20                  25

TAC AGT AAC CCT AAG ATC CTA AAA CAG AGC ATG ACC TTG AAC CTG GCC 1045
Tyr Ser Asn Pro Lys Ile Leu Lys Gln Ser Met Thr Leu Asn Leu Ala
        30                  35                  40

GAC CCG GTG GGC AGT CTG AAG CCG CAC CTC CGC GCC AAG AAC TCG GAC 1093
Asp Pro Val Gly Ser Leu Lys Pro His Leu Arg Ala Lys Asn Ser Asp
    45                  50                  55

CTT CTC ACG TCG CCC GAC GTC GGG CTG CTC AAG CTG GCG TCG CCG GAG 1141
Leu Leu Thr Ser Pro Asp Val Gly Leu Leu Lys Leu Ala Ser Pro Glu
60                  65                  70                  75

CTG GAG CGC CTG ATC ATC CAG TCC AGC AAT GGG CAC ATC ACC ACT ACA 1189
Leu Glu Arg Leu Ile Ile Gln Ser Ser Asn Gly His Ile Thr Thr Thr
                80                  85                  90

CCG ACC CCC ACC CAG TTC TTG TGC CCC AAG AAC GTG ACC GAC GAG CAG 1237
Pro Thr Pro Thr Gln Phe Leu Cys Pro Lys Asn Val Thr Asp Glu Gln
            95                  100                 105

GAG GGC TTC GCC GAG GGC TTC GTG CGC GCC CTG GCT GAA CTG CAT AGC 1285
Glu Gly Phe Ala Glu Gly Phe Val Arg Ala Leu Ala Glu Leu His Ser
        110                 115                 120

CAG AAC ACG CTT CCC AGT GTC ACC TCC GCG GCA CAG CCG GTC AGC GGG 1333
Gln Asn Thr Leu Pro Ser Val Thr Ser Ala Ala Gln Pro Val Ser Gly
    125                 130                 135

GCG GGC ATG GTG GCT CCC GCG GTG GCC TCA GTA GCA GGC GCT GGC GGC 1381
Ala Gly Met Val Ala Pro Ala Val Ala Ser Val Ala Gly Ala Gly Gly
140                 145                 150                 155

GGT GGT GGC TAC AGC GCC AGC CTG CAC AGT GAG CCT CCG GTC TAC GCC 1429
Gly Gly Gly Tyr Ser Ala Ser Leu His Ser Glu Pro Pro Val Tyr Ala
                160                 165                 170

AAC CTC AGC AAC TTC AAC CCG GGT GCG CTG AGC AGC GGC GGT GGG GCG 1477
Asn Leu Ser Asn Phe Asn Pro Gly Ala Leu Ser Ser Gly Gly Gly Ala
            175                 180                 185

CCC TCC TAT GGC GCG GCC GGG CTG GCC TTT CCC TCG CAG CCG CAG CAG 1525
Pro Ser Tyr Gly Ala Ala Gly Leu Ala Phe Pro Ser Gln Pro Gln Gln
        190                 195                 200

CAG CAG CAG CCG CCT CAG CCG CCG CAC CAC TTG CCC CAA CAG ATC CCG 1573
Gln Gln Gln Pro Pro Gln Pro Pro His His Leu Pro Gln Gln Ile Pro
    205                 210                 215

GTG CAG CAC CCG CGG CTG CAA GCC CTG AAG GAA GAG CCG CAG ACC GTG 1621
Val Gln His Pro Arg Leu Gln Ala Leu Lys Glu Glu Pro Gln Thr Val
220                 225                 230                 235

CCG GAG ATG CCG GGA GAG ACG CCG CCC CTG TCC CCT ATC GAC ATG GAG 1669
Pro Glu Met Pro Gly Glu Thr Pro Pro Leu Ser Pro Ile Asp Met Glu
                240                 245                 250

TCT CAG GAG CGG ATC AAG GCA GAG AGG AAG CGC ATG AGG AAC CGC ATT 1717
Ser Gln Glu Arg Ile Lys Ala Glu Arg Lys Arg Met Arg Asn Arg Ile
            255                 260                 265

GCC GCC TCC AAG TGC CGG AAA AGG AAG CTG GAG CGG ATC GCT CGG CTA 1765
Ala Ala Ser Lys Cys Arg Lys Arg Lys Leu Glu Arg Ile Ala Arg Leu
        270                 275                 280

GAG GAA AAA GTG AAA ACC TTG AAA GCG CAA AAC TCC GAG CTG GCA TCC 1813
Glu Glu Lys Val Lys Thr Leu Lys Ala Gln Asn Ser Glu Leu Ala Ser
    285                 290                 295

ACG GCC AAC ATG CTC AGG GAA CAG GTG GCA CAG CTT AAG CAG AAA GTC 1861
Thr Ala Asn Met Leu Arg Glu Gln Val Ala Gln Leu Lys Gln Lys Val
300                 305                 310                 315

ATG AAC CAC GTT AAC AGT GGG TGC CAA CTC ATG CTA ACG CAG CAG TTG 1909
Met Asn His Val Asn Ser Gly Cys Gln Leu Met Leu Thr Gln Gln Leu
                320                 325                 330

CAA ACG TTT TGAGAACAGA CTGTCAGGGC TGAGGGGCAA TGGAAGAAAA 1958
Gln Thr Phe

AAAATAACAG AGACAAACTT GAGAACTTGA CTGGTTGCGA CAGAGAAAAA AAAAGTGTCC 2018

GAGTACTGAA GCCAAGGGTA CACAAGATGG ACTGGGTTGC GACCTGACGG CGCCCCCAGT 2078

GTGCTGGAGT GGGAAGGACG TGGCGCGCCT GGCTTTGGCG TGGAGCCAGA GAGCAGCGGC 2138

CTATTGGCCG GCAGACTTTG CGGACGGGCT GTGCCCGCGC GCGACCAGAA CGATGGACTT 2198

TTCGTTAACA TTGACCAAGA ACTGCATGGA CCTAACATTC GATCTCATTC AGTATTAAAG 2258

GGGGGTGGGA GGGGTTACAA ACTGCAATAG AGACTGTAGA TTGCTTCTGT AGTGCTCCTT 2318

AACACAAAGC AGGGAGGGCT GGGAAGGGGG GGAGGCTTGT AAGTGCCAGG CTAGACTGCA 2378

GATGAACTCC CCTGGCCTGC CTCTCTCAAC TGTGTATGTA CATATATATT TTTTTTTAAT 2438

TTGATGAAAG CTGATTACTG TCAATAAACA GCTTCCTGCC TTTGTAAGTT ATTCCATGTT 2498

TGTTTGTTTG GGTGTCCTGC CCAGTGTTTG TAAATAAGAG ATTTGAAGCA TTCTGAGTTT 2558

ACCATTTGTA ATAAAGTATA TAATTTTTTT ATGTTTTGTT TCTGAAAATT TCCAGAAAGG 2618

ATATTTAAGA AAATACAATA AACTATTGAA AAGTAGCCCC CAACCTCTTT GCTGCATTAT 2678

CCATAGATAA TGATAGCTAG ATGAAGTGAC AGCTGAGTGC CCCCAATATA CTAGGGTGAA 2738

AGCTGTGTCC CCTGTCTGAT TTGTAGGAAT AGATACCCTG CATGCTATCA TTGGCTCATA 2798

CTCTCTCCCC CGGCAACACA CAAGTCCAGA CTGTACACCA GAAGATGGTG TGGTGTTTCT 2858

TAAGGCTGGA AGAAGGGCTG TTGCAAGGGG AGAGGGTCAG CCCGCTGGAA AGCAGACACT 2918

TTGGTTGAAA GCTGTATGAA GTGGCATGTG CTGTGATCAT TTATAATCAT AGGAAAGATT 2978

TAGTAATTAG CTGTTGATTC TCAAAGCAGG GACCCATGGA AGTTTTTAAC AAAAGGTGTC 3038

TCCTTCCAAC TTTGAATCTG ACAACTCCTA GAAAAAGATG ACCTTTGCTT GTGCATATTT 3098

ATAATAGCGT TCGTTATCAC AATAAATGTA TTCAAAT 3135

(2) INFORMATION FOR SEQ ID NO: 2:

(i) SEQUENCE CHARACTERISTICS:
(A) LENGTH: 334 amino acids
(B) TYPE: amino acid
(D) TOPOLOGY: linear

(ii) MOLECULE TYPE: protein

(xi) SEQUENCE DESCRIPTION: SEQ ID NO: 2:

Met Thr Ala Lys Met Glu Thr Thr Phe Tyr Asp Asp Ala Leu Asn Ala
1               5                   10                  15

Ser Phe Leu Gln Ser Glu Ser Gly Ala Tyr Gly Tyr Ser Asn Pro Lys
            20                  25                  30

Ile Leu Lys Gln Ser Met Thr Leu Asn Leu Ala Asp Pro Val Gly Ser
        35                  40                  45

Leu Lys Pro His Leu Arg Ala Lys Asn Ser Asp Leu Leu Thr Ser Pro
    50                  55                  60

Asp Val Gly Leu Leu Lys Leu Ala Ser Pro Glu Leu Glu Arg Leu Ile
65                  70                  75                  80

Ile Gln Ser Ser Asn Gly His Ile Thr Thr Thr Pro Thr Pro Thr Gln
                85                  90                  95

Phe Leu Cys Pro Lys Asn Val Thr Asp Glu Gln Glu Gly Phe Ala Glu
            100                 105                 110

Gly Phe Val Arg Ala Leu Ala Glu Leu His Ser Gln Asn Thr Leu Pro
        115                 120                 125

Ser Val Thr Ser Ala Ala Gln Pro Val Ser Gly Ala Gly Met Val Ala
    130                 135                 140

Pro Ala Val Ala Ser Val Ala Gly Ala Gly Gly Gly Gly Gly Tyr Ser
145                 150                 155                 160

Ala Ser Leu His Ser Glu Pro Pro Val Tyr Ala Asn Leu Ser Asn Phe
                165                 170                 175

Asn Pro Gly Ala Leu Ser Ser Gly Gly Gly Ala Pro Ser Tyr Gly Ala
            180                 185                 190

Ala Gly Leu Ala Phe Pro Ser Gln Pro Gln Gln Gln Gln Gln Pro Pro
        195                 200                 205

Gln Pro Pro His His Leu Pro Gln Gln Ile Pro Val Gln His Pro Arg
    210                 215                 220

Leu Gln Ala Leu Lys Glu Glu Pro Gln Thr Val Pro Glu Met Pro Gly
225                 230                 235                 240

Glu Thr Pro Pro Leu Ser Pro Ile Asp Met Glu Ser Gln Glu Arg Ile
                245                 250                 255

Lys Ala Glu Arg Lys Arg Met Arg Asn Arg Ile Ala Ala Ser Lys Cys
            260                 265                 270

Arg Lys Arg Lys Leu Glu Arg Ile Ala Arg Leu Glu Glu Lys Val Lys
        275                 280                 285

Thr Leu Lys Ala Gln Asn Ser Glu Leu Ala Ser Thr Ala Asn Met Leu
    290                 295                 300

Arg Glu Gln Val Ala Gln Leu Lys Gln Lys Val Met Asn His Val Asn
305                 310                 315                 320

Ser Gly Cys Gln Leu Met Leu Thr Gln Gln Leu Gln Thr Phe
                325                 330

(2) INFORMATION FOR SEQ ID NO: 3:

     (i) SEQUENCE CHARACTERISTICS:
          (A) LENGTH: 3622 base pairs
          (B) TYPE: nucleic acid
          (C) STRANDEDNESS: single
          (D) TOPOLOGY: linear

    (ii) MOLECULE TYPE: cDNA

(ix) FEATURE:
(A) NAME/KEY: mRNA
(B) LOCATION: 287..3622

(ix) FEATURE:
(A) NAME/KEY: mRNA
(B) LOCATION: 289..3622

(ix) FEATURE:
(A) NAME/KEY: mRNA
(B) LOCATION: 293..3622

(ix) FEATURE:
(A) NAME/KEY: CDS
(B) LOCATION: 1261..2253

(xi) SEQUENCE DESCRIPTION: SEQ ID NO: 3:

CCCGGGGAGG GGACCGGGGA ACAGAGGGCC GAGAGGCGTG CGGCAGGGGG GAGGGTAGGA 60

GAAAGAAGGG CCCGACTGTA GGAGGGCAGC GGAGCATTAC CTCATCCCGT GAGCCTCCGC 120

GGGCCCAGAG AAGAATCTTC TAGGGTGGAG TCTCCATGGT GACGGGCGGG CCCGCCCCCC 180

TGAGAGCGAC GCGAGCCAAT GGGAAGGCCT TGGGGTGACA TCATGGGCTA TTTTTAGGGG 240

TTGACTGGTA GCAGATAAGT GTTGAGCTCG GGCTGGATAA GGGCTCAGAG TTGCACTGAG 300

TGTGGCTGAA GCAGCGAGGC GGGAGTGGAG GTGCGCGGAG TCAGGCAGAC AGACAGACAC 360

AGCCAGCCAG CCAGGTCGGC AGTATAGTCC GAACTGCAAA TCTTATTTTC TTTTCACCTT 420

CTCTCTAACT GCCCAGAGCT AGCGCCTGTG GCTCCCGGGC TGGTGGTTCG GGAGTGTCCA 480

GAGAGCCTTG TCTCCAGCCG GCCCCGGGAG GAGAGCCCTG CTGCCCAGGC GCTGTTGACA 540

GCGGCGGAAA GCAGCGGTAC CCCACGCGCC CGCCGGGGGA CGTCGGCGAG CGGCTGCAGC 600

AGCAAAGAAC TTTCCCGGCG GGGAGGACCG GAGACAAGTG GCAGAGTCCC GGAGCGAACT 660

TTTGCAAGCC TTTCCTGCGT CTTAGGCTTC TCCACGGCGG TAAAGACCAG AAGGCGGCGG 720

AGAGCCACGC AAGAGAAGAA GGACGTGCGC TCAGCTTCGC TCGCACCGGT TGTTGAACTT 780

GGGCGAGCGC GAGCCGCGGC TGCCGGGCGC CCCCTCCCCC TAGCAGCGGA GGAGGGGACA 840

AGTCGTCGGA GTCCGGGCGG CCAAGACCCG CCGCCGGCCG GCCACTGCAG GGTCCGCACT 900

GATCCGCTCC GCGGGGAGAG CCGCTGCTCT GGGAAGTGAG TTCGCCTGCG GACTCCGAGG 960

AACCGCTGCG CCCGAAGAGC GCTCAGTGAG TGACCGCGAC TTTTCAAAGC CGGGTAGCGC 1020

GCGCGAGTCG ACAAGTAAGA GTGCGGGAGG CATCTTAATT AACCCTGCGC TCCCTGGAGC 1080

GAGCTGGTGA GGAGGGCGCA GCGGGGACGA CAGCCAGCGG GTGCGTGCGC TCTTAGAGAA 1140

ACTTTCCCTG TCAAAGGCTC CGGGGGGCGC GGGTGTCCCC CGCTTGCCAG AGCCCTGTTG 1200

CGGCCCCGAA ACTTGTGCGC GCACGCCAAA CTAACCTCAC GTGAAGTGAC GGACTGTTCT 1260

ATG ACT GCA AAG ATG GAA ACG ACC TTC TAT GAC GAT GCC CTC AAC GCC 1308
Met Thr Ala Lys Met Glu Thr Thr Phe Tyr Asp Asp Ala Leu Asn Ala
1               5                   10                  15

TCG TTC CTC CCG TCC GAG AGC GGA CCT TAT GGC TAC AGT AAC CCC AAG 1356
Ser Phe Leu Pro Ser Glu Ser Gly Pro Tyr Gly Tyr Ser Asn Pro Lys
            20                  25                  30

ATC CTG AAA CAG AGC ATG ACC CTG AAC CTG GCC GAC CCA GTG GGG AGC 1404
Ile Leu Lys Gln Ser Met Thr Leu Asn Leu Ala Asp Pro Val Gly Ser
        35                  40                  45

CTG AAG CCG CAC CTC CGC GCC AAG AAC TCG GAC CTC CTC ACC TCG CCC 1452
Leu Lys Pro His Leu Arg Ala Lys Asn Ser Asp Leu Leu Thr Ser Pro
    50                  55                  60

GAC GTG GGG CTG CTC AAG CTG GCG TCG CCC GAG CTG GAG CGC CTG ATA 1500
Asp Val Gly Leu Leu Lys Leu Ala Ser Pro Glu Leu Glu Arg Leu Ile
65                  70                  75                  80

ATC CAG TCC AGC AAC GGG CAC ATC ACC ACC ACG CCG ACC CCC ACC CAG 1548
Ile Gln Ser Ser Asn Gly His Ile Thr Thr Thr Pro Thr Pro Thr Gln
                85                  90                  95

TTC CTG TGC CCC AAG AAC GTG ACA GAT GAG CAG GAG GGG TTC GCC GAG 1596
Phe Leu Cys Pro Lys Asn Val Thr Asp Glu Gln Glu Gly Phe Ala Glu
            100                 105                 110

GGC TTC GTG CGC GCC CTG GCC GAA CTG CAC AGC CAG AAC ACG CTG CCC 1644
Gly Phe Val Arg Ala Leu Ala Glu Leu His Ser Gln Asn Thr Leu Pro
        115                 120                 125

AGC GTC ACG TCG GCG GCG CAG CCG GTC AAC GGG GCA GGC ATG GTG GCT 1692
Ser Val Thr Ser Ala Ala Gln Pro Val Asn Gly Ala Gly Met Val Ala
    130                 135                 140

CCC GCG GTA GCC TCG GTG GCA GGG GGC AGC GGC AGC GGC GGC TTC AGC 1740
Pro Ala Val Ala Ser Val Ala Gly Gly Ser Gly Ser Gly Gly Phe Ser
145                 150                 155                 160

GCC AGC CTG CAC AGC GAG CCG CCG GTC TAC GCA AAC CTC AGC AAC TTC 1788
Ala Ser Leu His Ser Glu Pro Pro Val Tyr Ala Asn Leu Ser Asn Phe
                165                 170                 175

AAC CCA GGC GCG CTG AGC AGC GGC GGC GGG GCG CCC TCC TAC GGC GCG 1836
Asn Pro Gly Ala Leu Ser Ser Gly Gly Gly Ala Pro Ser Tyr Gly Ala
            180                 185                 190

GCC GGC CTG GCC TTT CCC GCG CAA CCC CAG CAG CAG CAG CAG CCG CCG 1884
Ala Gly Leu Ala Phe Pro Ala Gln Pro Gln Gln Gln Gln Gln Pro Pro
        195                 200                 205

CAC CAC CTG CCC CAG CAG ATG CCC GTG CAG CAC CCG CGG CTG CAG GCC 1932
His His Leu Pro Gln Gln Met Pro Val Gln His Pro Arg Leu Gln Ala
    210                 215                 220

CTG AAG GAG GAG CCT CAG ACA GTG CCC GAG ATG CCC GGC GAG ACA CCG 1980
Leu Lys Glu Glu Pro Gln Thr Val Pro Glu Met Pro Gly Glu Thr Pro
225                 230                 235                 240

CCC CTG TCC CCC ATC GAC ATG GAG TCC CAG GAG CGG ATC AAG GCG GAG 2028
Pro Leu Ser Pro Ile Asp Met Glu Ser Gln Glu Arg Ile Lys Ala Glu
                245                 250                 255

AGG AAG CGC ATG AGG AAC CGC ATC GCT GCC TCC AAG TGC CGA AAA AGG 2076
Arg Lys Arg Met Arg Asn Arg Ile Ala Ala Ser Lys Cys Arg Lys Arg
            260                 265                 270

AAG CTG GAG AGA ATC GCC CGG CTG GAG GAA AAA GTG AAA ACC TTG AAA 2124
Lys Leu Glu Arg Ile Ala Arg Leu Glu Glu Lys Val Lys Thr Leu Lys
        275                 280                 285

GCT CAG AAC TCG GAG CTG GCG TCC ACG GCC AAC ATG CTC AGG GAA CAG 2172
Ala Gln Asn Ser Glu Leu Ala Ser Thr Ala Asn Met Leu Arg Glu Gln
    290                 295                 300

GTG GCA CAG CTT AAA CAG AAA GTC ATG AAC CAC GTT AAC AGT GGG TGC 2220
Val Ala Gln Leu Lys Gln Lys Val Met Asn His Val Asn Ser Gly Cys
305                 310                 315                 320

CAA CTC ATG CTA ACG CAG CAG TTG CAA ACA TTT TGAAGAGAGA CCGTCGGGGG 2273
Gln Leu Met Leu Thr Gln Gln Leu Gln Thr Phe
                325                 330

CTGAGGGGCA ACGAAGAAAA AAAATAACAC AGAGAGACAG ACTTGAGAAC TTGACAAGTT 2333

GCGACGGAGA GAAAAAAGAA GTGTCCGAGA ACTAAAGCCA AGGGTATCCA AGTTGGACTG 2393

GGTTCGGTCT GACGGCGCCC CCAGTGTGCA CGAGTGGGAA GGACTTGGTC GCGCCCTCCC 2453

TTGGCGTGGA GCCAGGGAGC GGCCGCCTGC GGGCTGCCCC GCTTTGCGGA CGGGCTGTCC 2513

CCGCGCGAAC GGAACGTTGG ACTTTCGTTA ACATTGACCA AGAACTGCAT GGACCTAACA 2573

TTCGATCTCA TTCAGTATTA AAGGGGGGAG GGGGAGGGGG TTACAAACTG CAATAGAGAC 2633

TGTAGATTGC TTCTGTAGTA CTCCTTAAGA ACACAAAGCG GGGGGAGGGT TGGGGAGGGG 2693

CGGCAGGAGG GAGGTTTGTG AGAGCGAGGC TGAGCCTACA GATGAACTCT TTCTGGCCTG 2753

CTTTCGTTAA CTGTGTATGT ACATATATAT ATTTTTTAAT TTGATTAAAG CTGATTACTG 2813

TCAATAAACA GCTTCATGCC TTTGTAAGTT ATTTCTTGTT TGTTTGTTTG GGTATCCTGC 2873

CCAGTGTTGT TTGTAAATAA GAGATTTGGA GCACTCTGAG TTTACCATTT GTAATAAAGT 2933

ATATAATTTT TTTATGTTTT GTTTCTGAAA ATTCCAGAAA GGATATTTAA GAAAATACAA 2993

TAAACTATTG GAAAGTACTC CCCTAACCTC TTTTCTGCAT CATCTGTAGA TCCTAGTCTA 3053

TCTAGGTGGA GTTGAAAGAG TTAAGAATGC TCGATAAAAT CACTCTCAGT GCTTCTTACT 3113

ATTAAGCAGT AAAAACTGTT CTCTATTAGA CTTAGAAATA AATGTACCTG ATGTACCTGA 3173

TGCTATGTCA GGCTTCATAC TCCACGCTCC CCCAGCGTAT CTATATGGAA TTGCTTACCA 3233

AAGGCTAGTG CGATGTTTCA GGAGGCTGGA GGAAGGGGGG TTGCAGTGGA GAGGGACAGC 3293

CCACTGAGAA GTCAAACATT TCAAAGTTTG GATTGCATCA AGTGGCATGT GCTGTGACCA 3353

TTTATAATGT TAGAAATTTT ACAATAGGTG CTTATTCTCA AAGCAGGAAT TGGTGGCAGA 3413

TTTTACAAAA GATGTATCCT TCCAATTTGG AATCTTCTCT TTGACAATTC CTAGATAAAA 3473

AGATGGCCTT TGTCTTATGA ATATTTATAA CAGCATTCTG TCACAATAAA TGTATTCAAA 3533

TACCAATAAC AGATCTTGAA TTGCTTCCCT TTACTACTTT TTTGTTCCCA AGTTATATAC 3593

TGAAGTTTTT ATTTTTAGTT GCTGAGGTT 3622

(2) INFORMATION FOR SEQ ID NO: 4:

(i) SEQUENCE CHARACTERISTICS:
(A) LENGTH: 331 amino acids
(B) TYPE: amino acid
(D) TOPOLOGY: linear

(ii) MOLECULE TYPE: protein

(xi) SEQUENCE DESCRIPTION: SEQ ID NO: 4:

Met Thr Ala Lys Met Glu Thr Thr Phe Tyr Asp Asp Ala Leu Asn Ala
1               5                   10                  15

Ser Phe Leu Pro Ser Glu Ser Gly Pro Tyr Gly Tyr Ser Asn Pro Lys
            20                  25                  30

Ile Leu Lys Gln Ser Met Thr Leu Asn Leu Ala Asp Pro Val Gly Ser
        35                  40                  45

Leu Lys Pro His Leu Arg Ala Lys Asn Ser Asp Leu Leu Thr Ser Pro
    50                  55                  60

Asp Val Gly Leu Leu Lys Leu Ala Ser Pro Glu Leu Glu Arg Leu Ile
65                  70                  75                  80

Ile Gln Ser Ser Asn Gly His Ile Thr Thr Thr Pro Thr Pro Thr Gln
                85                  90                  95

Phe Leu Cys Pro Lys Asn Val Thr Asp Glu Gln Glu Gly Phe Ala Glu
            100                 105                 110

Gly Phe Val Arg Ala Leu Ala Glu Leu His Ser Gln Asn Thr Leu Pro
        115                 120                 125

Ser Val Thr Ser Ala Ala Gln Pro Val Asn Gly Ala Gly Met Val Ala
    130                 135                 140

Pro Ala Val Ala Ser Val Ala Gly Gly Ser Gly Ser Gly Gly Phe Ser
145                 150                 155                 160

Ala Ser Leu His Ser Glu Pro Pro Val Tyr Ala Asn Leu Ser Asn Phe
                165                 170                 175

Asn Pro Gly Ala Leu Ser Ser Gly Gly Gly Ala Pro Ser Tyr Gly Ala
            180                 185                 190

Ala Gly Leu Ala Phe Pro Ala Gln Pro Gln Gln Gln Gln Gln Pro Pro
        195                 200                 205

His His Leu Pro Gln Gln Met Pro Val Gln His Pro Arg Leu Gln Ala
    210                 215                 220

Leu Lys Glu Glu Pro Gln Thr Val Pro Glu Met Pro Gly Glu Thr Pro
225                 230                 235                 240

Pro Leu Ser Pro Ile Asp Met Glu Ser Gln Glu Arg Ile Lys Ala Glu
                245                 250                 255

Arg Lys Arg Met Arg Asn Arg Ile Ala Ala Ser Lys Cys Arg Lys Arg
            260                 265                 270

Lys Leu Glu Arg Ile Ala Arg Leu Glu Glu Lys Val Lys Thr Leu Lys
        275                 280                 285

Ala Gln Asn Ser Glu Leu Ala Ser Thr Ala Asn Met Leu Arg Glu Gln
    290                 295                 300

Val Ala Gln Leu Lys Gln Lys Val Met Asn His Val Asn Ser Gly Cys
305                 310                 315                 320

Gln Leu Met Leu Thr Gln Gln Leu Gln Thr Phe
                325                 330

(2) INFORMATION FOR SEQ ID NO: 5:

     (i) SEQUENCE CHARACTERISTICS:
          (A) LENGTH: 3548 base pairs
          (B) TYPE: nucleic acid
          (C) STRANDEDNESS: single
          (D) TOPOLOGY: linear

    (ix) FEATURE:
          (A) NAME/KEY: TATA_signal
          (B) LOCATION: 101..106

    (ix) FEATURE:
          (A) NAME/KEY: polyA_signal
          (B) LOCATION: 3493..3498

    (ix) FEATURE:
          (A) NAME/KEY: exon
          (B) LOCATION: 284..424

    (ix) FEATURE:
          (A) NAME/KEY: exon
          (B) LOCATION: 1179..1430

    (ix) FEATURE:
          (A) NAME/KEY: exon
          (B) LOCATION: 1836..1943

(ix) FEATURE:
(A) NAME/KEY: exon
(B) LOCATION: 2061..2702

(ix) FEATURE:
(A) NAME/KEY: precursor_RNA
(B) LOCATION: 133..2702

(ix) FEATURE:
(A) NAME/KEY: intron
(B) LOCATION: 425..1178

(ix) FEATURE:
(A) NAME/KEY: intron
(B) LOCATION: 1431..1835

(ix) FEATURE:
(A) NAME/KEY: intron
(B) LOCATION: 1944..2060

(xi) SEQUENCE DESCRIPTION: SEQ ID NO: 5:

GAGTTGACGA CAGAGCGCCC GCAGAGGGCC TTGGGGCGCG CTTCCCCCCC CTTCCAGTTC 60

CGCCCAGTGA CGTAGGAAGT CCATCCATTC ACAGCGCTTC TATAAAGGCG CCAGCTGAGG 120

CGCCTACTAC TCCAACCGCG ACTGCAGCGA GCAACTGAGA AGACTGGATA GAGCCGGCGG 180

TTCCGCGAAC GAGCAGTGAC CGCGCTCCCA CCCAGCTCTG CTCTGCAGCT CCCACCAGTG 240

TCTACCCCTG GACCCCTTGC CGGGCTTTCC CCAAACTTCG ACCATGATGT TCTCGGGTTT 300

CAACGCCGAC TACGAGGCGT CATCCTCCCG CTGCAGTAGC GCCTCCCCGG CCGGGGACAG 360

CCTTTCCTAC TACCATTCCC CAGCCGACTC CTTCTCCAGC ATGGGCTCTC CTGTCAACAC 420

ACAGGTGAGT TTGGCTTTGT GTAGCCGCCA GGTCCGCGCT GAGGGTCGCC GTGGAGGAGA 480

CACTGGGGTG TGACTCGCAG GGGCGGGGGG GTCTTCCTTT TTCGCTCTGG AGGGAGACTG 540

GCGCGGTCAG AGCAGCCTTA GCCTGGGAAC CCAGGACTTG TCTGAGCGCG TGCACACTTG 600

TCATAGTAAG ACTTAGTGAC CCCTTCCCGC GCGGCAGGTT TATTCTGAGT GGCCTGCCTG 660

CATTCTTCTC TCGGCCGACT TGTTTCTGAG ATCAGCCGGG GCCAACAAGT CTCGAGCAAA 720

GAGTCGCTAA CTAGAGTTTG GGAGGCGGCA AACCGCGGCA ATCCCCCCTC CCGGGGCAGC 780

CTGGAGCAGG GAGGAGGGAG GAGGGAGGAG GGTGCTGCGG GCGGGTGTGT AAGGCAGTTT 840

CATTGATAAA AAGCGAGTTC ATTCTGGAGA CTCCGGAGCA GCGCCTGCGT CAGCGCAGAC 900

GTCAGGGATA TTTATAACAA ACCCCCTTTC GAGCGAGTGA TGCCGAAGGG ATAACGGGAA 960

CGCAGCAGTA GGATGGAGGA GAAAGGCTGC GCTGCGGAAT TCAAGGGAGG ATATTGGGAG 1020

AGCTTTTATC TCCGATGAGG TGCATACAGG AAGACATAAG CAGTCTCTGA CCGGAATGCT 1080

TCTCTCTCCC TGCTTCATGC GACACTAGGG CCACTTGCTC CACCTGTGTC TGGAACCTCC 1140
TCGCTCACCT CCGCTTTCCT CTTTTTGTTT TGTTTCAGGA CTTTTGCGCA GATCTGTCCG 1200
TCTCTAGTGC CAACTTTATC CCCACGGTGA CAGCCATCTC CACCAGCCCA GACCTGCAGT 1260
GGCTGGTGCA GCCCACTCTG GTCTCCTCCG TGGCCCCATC GCAGACCAGA GCGCCCCATC 1320
CTTACGGACT CCCCACCCAG TCTGCTGGGG CTTACGCCAG AGCGGGAATG GTGAAGACCG 1380
TGTCAGGAGG CAGAGCGCAG AGCATCGGCA GAAGGGGCAA AGTAGAGCAG GTGAGCAGCG 1440
ATTCTGGACC TTTGTGGGCT GGGGGGGGGG GGGGGGGCGG AGACTGACGC ACAGACCACA 1500
CAACAGAGAA GGGACGCTAC TGACTGCACT TCCTGACCAG GAGCTGTGGC TGCTAGCCCT 1560
TTCCCTCCCT TGTCAGATTT TGACAGTTGG ACCCAAGACA AACTCTAGAC AGTTTCCCTG 1620
ACAGCTTCCT ACTTCATTCT CTAGCCGGGG AGCTTCTTTG TTCCCCTGCT AAAGATCTCA 1680
CTTTAAATGC AAATCACACT CTGCCTGCCA ACTGCAGGTT AGAAAAACTG CTTCACCGAG 1740
AGGTGCGGGT GCTGTAGGAG CCAGTTTCAC TGGGGTGACT GAATGGAGGT GACACTAGAC 1800
AACCTTAACT GAATGTTGGT CCTTTTCTTC TATAGCTATC TCCTGAAGAG GAAGAGAAAC 1860
GGAGAATCCG AAGGGAACGG AATAAGATGG CTGCAGCCAA GTGCCGGAAT CGGAGGAGGG 1920
AGCTGACAGA TACACTCCAA GCGGTAGGTT GAACCAGCTG CTGCTCCTGA AACTTTATTA 1980
AAGTTGGAGC TTGGGACTAT GGGCGCAGGG TCCTTGAGCA TGCCCGTGTC TTATGCTTTC 2040
TTATATCTCT CCCTATGCAG GAGACAGATC AACTTGAAGA TGAGAAGTCT GCGTTGCAGA 2100
CTGAGATTGC CAATCTGCTG AAAGAGAAGG AAAAACTGGA GTTTATTTTG GCAGCCCACC 2160
GACCTGCCTG CAAGATCCCC GATGACCTTG GCTTCCCAGA GGAGATGTCT GTGGCCTCCC 2220
TGGATTTGAC TGGAGGTCTG CCTGAGGCTT CCACCCCAGA GTCTGAGGAG GCCTTCACCC 2280
TGCCCCTTCT CAACGACCCT GAGCCCAAGC CATCCTTGGA GCCAGTCAAG AGCATCAGCA 2340
ACGTGGAGCT GAAGGCAGAA CCCTTTGATG ACTTCTTGTT TCCGGCATCA TCTAGGCCCA 2400
GTGGCTCAGA GACCTCCCGC TCTGTGCCAG ATGTGGACCT GTCCGGTTCC TTCTATGCAG 2460
CAGACTGGGA GCCTCTGCAC AGCAATTCCT TGGGGATGGG GCCCATGGTC ACAGAGCTGG 2520
AGCCCCTGTG TACTCCCGTG GTCACCTGTA CTCCGGGCTG CACTACTTAC ACGTCTTCCT 2580
TTGTCTTCAC CTACCCTGAA GCTGACTCCT TCCCAAGCTG TGCCGCTGCC CACCGAAAGG 2640
GCAGCAGCAG CAACGAGCCC TCCTCCGACT CCCTGAGCTC ACCCACGCTG CTGGCCCTGT 2700
GAGCAGTCAG AGAAGGCAAG GCAGCCGGCA TCCAGACGTG CCACTGCCCG AGCTGGTGCA 2760
TTACAGAGAG GAGAAACACG TCTTCCCTCG AAGGTTCCCG TCGACCTAGG GAGGACCTTA 2820

CCTGTTCGTG AAACACACCA GGCTGTGGGC CTCAAGGACT TGCAAGCATC CACATCTGGC 2880

CTCCAGTCCT CACCTCTTCC AGAGATGTAG CAAAAACAAA ACAAAACAAA ACAAAAAACC 2940

GCATGGAGTG TGTTGTTCCT AGTGACACCT GAGAGCTGGT AGTTAGTAGA GCATGTGAGT 3000

CAAGGCCTGG TCTGTGTCTC TTTTCTCTTT CTCCTTAGTT TTCTCATAGC ACTAACTAAT 3060

CTGTTGGGTT CATTATTGGA ATTAACCTGG TGCTGGATTG TATCTAGTGC AGCTGATTTT 3120

AACAATACCT ACTGTGTTCC TGGCAATAGC GTGTTCCAAT TAGAAACGAC CAATATTAAA 3180

CTAAGAAAAG ATAGGACTTT ATTTTCCAGT AGATAGAAAT CAATAGCTAT ATCCATGTAC 3240

TGTAGTCCTT CAGCGTCAAT GTTCATTGTC ATGTTACTGA TCATGCATTG TCGAGGTGGT 3300

CTGAATGTTC TGACATTAAC AGTTTTCCAT GAAAACGTTT TTATTGTGTT TTCAATTTAT 3360

TTATTAAGAT GGATTCTCAG ATATTTATAT TTTTATTTTA TTTTTTTCTA CCCTGAGGTC 3420

TTTCGACATG TGGAAAGTGA ATTTGAATGA AAAATTTTAA GCATTGTTTG CTTATTGTTC 3480

CAGGACATTG TCAATAAAAG CATTTAAGTT GAATGCGACC ACCTTCTTGC TCTCTTTATT 3540

CTCAGTTT 3548

(2) INFORMATION FOR SEQ ID NO: 6:

(i) SEQUENCE CHARACTERISTICS:
(A) LENGTH: 6210 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: CDS
(B) LOCATION: join(889..1029, 1783..2034, 2466..2573, 2688
..3326)

(ix) FEATURE:
(A) NAME/KEY: misc_feature
(B) LOCATION: 402..453
(D) OTHER INFORMATION: /note= ""transcriptional activator
region""

(ix) FEATURE:
(A) NAME/KEY: prim_transcript
(B) LOCATION: 734..3329

(ix) FEATURE:
(A) NAME/KEY: exon
(B) LOCATION: 889..1029
(D) OTHER INFORMATION: /note= ""c-fos protein, exon1""

(ix) FEATURE:
(A) NAME/KEY: intron

(B) LOCATION: 1030..1782
(D) OTHER INFORMATION: /note= ""c-fos, intron A""

(ix) FEATURE:
(A) NAME/KEY: exon
(B) LOCATION: 1783..2034

(ix) FEATURE:
(A) NAME/KEY: intron
(B) LOCATION: 2035..2465

(ix) FEATURE:
(A) NAME/KEY: exon
(B) LOCATION: 2466..2573

(ix) FEATURE:
(A) NAME/KEY: intron
(B) LOCATION: 2574..2687

(ix) FEATURE:
(A) NAME/KEY: exon
(B) LOCATION: 2688..3329

(xi) SEQUENCE DESCRIPTION: SEQ ID NO: 6:

GCAGGAACAG TGCTAGTATT GCTCGAGCCC GAGGGCTGGA GGTTAGGGGA TGAAGGTCTG 60

CTTCCACGCT TTGCACTGAA TTAGGGCTAG AATTGGGGAT GGGGGTAGGG GCGCATTCCT 120

TCGGGAGCCG AGGCTTAAGT CCTCGGGGTC CTGTACTCGA TGCCGTTTCT CCTATCTCTG 180

AGCCTCAGAA CTGTCTTCAG TTTCCGTACA AGGGTAAAAA GGCGCTCTCT GCCCCATCCC 240

CCCCGACCTC GGGAACAAGG GTCCGCATTG AACCAGGTGC GAATGTTCTC TCTCATTCTG 300

CGCCGTTCCC GCCTCCCCTC CCCCAGCCGC GGCCCCCGCC TCCCCCCGCA CTGCACCCTC 360

GGTGTTGGCT GCAGCCCGCG AGCAGTTCCC GTCAATCCCT CCCCCCTTAC ACAGGATGTC 420

CATATTAGGA CATCTGCGTC AGCAGGTTTC CACGGCCTTT CCCTGTAGCC CTGGGGGGAG 480

CCATCCCCGA AACCCCTCAT CTTGGGGGGC CCACGAGACC TCTGAGACAG GAACTGCGAA 540

ATGCTCACGA GATTAGGACA CGCGCCAAGG CGGGGGCAGG GAGCTGCGAG CGCTGGGGAC 600

GCAGCCGGGC GGCCGCAGAA GCGCCCAGGC CCGCGCGCCA CCCCTCTGGC GCCACCGTGG 660

TTGAGCCCGT GACGTTTACA CTCATTCATA AAACGCTTGT TATAAAAGCA GTGGCTGCGG 720

CGCCTCGTAC TCCAACCGCA TCTGCAGCGA GCAACTGAGA AGCCAAGACT GAGCCGGCGG 780

CCGCGGCGCA GCGAACGAGC AGTGACCGTG CTCCTACCCA GCTCTGCTTC ACAGCGCCCA 840

CCTGTCTCCG CCCCTCGGCC CCTCGCCCGG CTTTGCCTAA CCGCCACG ATG ATG TTC 897
Met Met Phe
1

TCG GGC TTC AAC GCA GAC TAC GAG GCG TCA TCC TCC CGC TGC AGC AGC 945
Ser Gly Phe Asn Ala Asp Tyr Glu Ala Ser Ser Ser Arg Cys Ser Ser
    5                   10                  15

GCG TCC CCG GCC GGG GAT AGC CTC TCT TAC TAC CAC TCA CCC GCA GAC 993
Ala Ser Pro Ala Gly Asp Ser Leu Ser Tyr Tyr His Ser Pro Ala Asp
20                  25                  30                  35

TCC TTC TCC AGC ATG GGC TCG CCT GTC AAC GCG CAG GTAAGGCTGG 1039
Ser Phe Ser Ser Met Gly Ser Pro Val Asn Ala Gln
                40                  45

CTTCCCGTCG CCGCGGGGCC GGGGGCTTGG GGTCGCGGAG GAGGAGACAC CGGGCGGGAC 1099

GCTCCAGTAG ATGAGTAGGG GGCTCCCTTG TGCCTGGAGG GAGGCTGCCG TGGCCGGAGC 1159

GGTGCCGGCT CGGGGGCTCG GGACTTGCTC TGAGCGCACG CACGCTTGCC ATAGTAAGAA 1219

TTGGTTCCCC CTTCGGGAGG CAGGTTCGTT CTGAGCAACC TCTGGTCTGC ACTCCAGGAC 1279

GGATCTCTGA CATTAGCTGG AGCAGACGTG TCCCAAGCAC AAACTCGCTA ACTAGAGCCT 1339

GGCTTCTTCG GGGAGGTGGC AGAAAGCGGC AATCCCCCCT CCCCCGGCAG CCTGGAGCAC 1399

GGAGGAGGGA TGAGGGAGGA GGGTGCAGCG GGCGGGTGTG TAAGGCAGTT TCATTGATAA 1459

AAAGCGAGTT CATTCTGGAG ACTCCGGAGC GGCGCCTGCG TCAGCGCAGA CGTCAGGGAT 1519

ATTTATAACA AACCCCCTTT CAAGCAAGTG ATGCTGAAGG GATAACGGGA ACGCAGCGGC 1579

AGGATGGAAG AGACAGGCAC TGCGCTGCGG AATGCCTGGG AGGAAAAGGG GGAGACCTTT 1639

CATCCAGGAT GAGGGACATT TAAGATGAAA TGTCCGTGGC AGGATCGTTT CTCTTCACTG 1699

CTGCATGCGG CACTGGGAAC TCGCCCCACC TGTGTCCGGA ACCTGCTCGC TCACGTCGGC 1759

TTTCCCCTTC TGTTTTGTTC TAG GAC TTC TGC ACG GAC CTG GCC GTC TCC 1809
Asp Phe Cys Thr Asp Leu Ala Val Ser
        50                  55

AGT GCC AAC TTC ATT CCC ACG GTC ACT GCC ATC TCG ACC AGT CCG GAC 1857
Ser Ala Asn Phe Ile Pro Thr Val Thr Ala Ile Ser Thr Ser Pro Asp
            60                  65                  70

CTG CAG TGG CTG GTG CAG CCC GCC CTC GTC TCC TCT GTG GCC CCA TCG 1905
Leu Gln Trp Leu Val Gln Pro Ala Leu Val Ser Ser Val Ala Pro Ser
        75                  80                  85

CAG ACC AGA GCC CCT CAC CCT TTC GGA GTC CCC GCC CCC TCC GCT GGG 1953
Gln Thr Arg Ala Pro His Pro Phe Gly Val Pro Ala Pro Ser Ala Gly
    90                  95                  100

GCT TAC TCC AGG GCT GGC GTT GTG AAG ACC ATG ACA GGA GGC CGA GCG 2001
Ala Tyr Ser Arg Ala Gly Val Val Lys Thr Met Thr Gly Gly Arg Ala
105                 110                 115                 120

CAG AGC ATT GGC AGG AGG GGC AAG GTG GAA CAG GTGAGGAACT CTAGCGTACT 2054
Gln Ser Ile Gly Arg Arg Gly Lys Val Glu Gln
                125                 130

CTTCCTGGGA ATGTGGGGGC TGGGTGGGAA GCAGCCCCGG AGATGCAGGA GCCCAGTACA 2114

GAGGATGAAG CCACTGATGG GGCTGGCTGC ACATCCGTAA CTGGGAGCCC TGGCTCCAAG 2174

CCCATTCCAT CCCAACTCAG ACTCTGAGTC TCACCCTAAG AAGTACTCTC ATAGTTTCTT 2234

CCCTAAGTTT CTTACCGCAT GCTTTCAGAC TGGGCTCTTC TTTGTTCTCT TGCTGAGGAT 2294

CTTATTTTAA ATGCAAGTCA CACCTATTCT GCAACTGCAG GTCAGAAATG GTTTCACAGT 2354

GGGGTGCCAG GAAGCAGGGA AGCTGCAGGA GCCAGTTCTA CTGGGGTGGG TGAATGGAGG 2414

TGATGGCAGA CACTTTTACT GAATGTCGGT CTTTTTTTGT GATTATTCTA G TTA TCT 2471
Leu Ser

CCA GAA GAA GAA GAG AAA AGG AGA ATC CGA AGG GAA AGG AAT AAG ATG 2519
Pro Glu Glu Glu Glu Lys Arg Arg Ile Arg Arg Glu Arg Asn Lys Met
    135                 140                 145

GCT GCA GCC AAA TGC CGC AAC CGG AGG AGG GAG CTG ACT GAT ACA CTC 2567
Ala Ala Ala Lys Cys Arg Asn Arg Arg Arg Glu Leu Thr Asp Thr Leu
150                 155                 160                 165

CAA GCG GTAGGTACTC TGTGGGTTGC TCCTTTTTAA AACTTAAGGG AAAGTTGGAG 2623
Gln Ala

ATTGAGCATA AGGGCCCTTG AGTAAGACTG TGTCTTATGC TTTCCTTTAT CCCTCTGTAT 2683

ACAG GAG ACA GAC CAA CTA GAA GAT GAG AAG TCT GCT TTG CAG ACC GAG 2732
Glu Thr Asp Gln Leu Glu Asp Glu Lys Ser Ala Leu Gln Thr Glu
        170                 175                 180

ATT GCC AAC CTG CTG AAG GAG AAG GAA AAA CTA GAG TTC ATC CTG GCA 2780
Ile Ala Asn Leu Leu Lys Glu Lys Glu Lys Leu Glu Phe Ile Leu Ala
        185                 190                 195

GCT CAC CGA CCT GCC TGC AAG ATC CCT GAT GAC CTG GGC TTC CCA GAA 2828
Ala His Arg Pro Ala Cys Lys Ile Pro Asp Asp Leu Gly Phe Pro Glu
    200                 205                 210

GAG ATG TCT GTG GCT TCC CTT GAT CTG ACT GGG GGC CTG CCA GAG GTT 2876
Glu Met Ser Val Ala Ser Leu Asp Leu Thr Gly Gly Leu Pro Glu Val
215                 220                 225                 230

GCC ACC CCG GAG TCT GAG GAG GCC TTC ACC CTG CCT CTC CTC AAT GAC 2924
Ala Thr Pro Glu Ser Glu Glu Ala Phe Thr Leu Pro Leu Leu Asn Asp
                235                 240                 245

CCT GAG CCC AAG CCC TCA GTG GAA CCT GTC AAG AGC ATC AGC AGC ATG 2972
Pro Glu Pro Lys Pro Ser Val Glu Pro Val Lys Ser Ile Ser Ser Met
            250                 255                 260

GAG CTG AAG ACC GAG CCC TTT GAT GAC TTC CTG TTC CCA GCA TCA TCC 3020
Glu Leu Lys Thr Glu Pro Phe Asp Asp Phe Leu Phe Pro Ala Ser Ser
        265                 270                 275

AGG CCC AGT GGC TCT GAG ACA GCC CGC TCC GTG CCA GAC ATG GAC CTA 3068
Arg Pro Ser Gly Ser Glu Thr Ala Arg Ser Val Pro Asp Met Asp Leu
    280                 285                 290

TCT GGG TCC TTC TAT GCA GCA GAC TGG GAG CCT CTG CAC AGT GGC TCC 3116
Ser Gly Ser Phe Tyr Ala Ala Asp Trp Glu Pro Leu His Ser Gly Ser
295                 300                 305                 310

CTG GGG ATG GGG CCC ATG GCC ACA GAG CTG GAG CCC CTG TGC ACT CCG 3164
Leu Gly Met Gly Pro Met Ala Thr Glu Leu Glu Pro Leu Cys Thr Pro
                315                 320                 325

GTG GTC ACC TGT ACT CCC AGC TGC ACT GCT TAC ACG TCT TCC TTC GTC 3212
Val Val Thr Cys Thr Pro Ser Cys Thr Ala Tyr Thr Ser Ser Phe Val
            330                 335                 340

TTC ACC TAC CCC GAG GCT GAC TCC TTC CCC AGC TGT GCA GCT GCC CAC 3260
Phe Thr Tyr Pro Glu Ala Asp Ser Phe Pro Ser Cys Ala Ala Ala His
        345                 350                 355

CGC AAG GGC AGC AGC AGC AAT GAG CCT TCC TCT GAC TCG CTC AGC TCA 3308
Arg Lys Gly Ser Ser Ser Asn Glu Pro Ser Ser Asp Ser Leu Ser Ser
    360                 365                 370

CCC ACG CTG CTG GCC CTG TGAGGGGGCA GGGAAGGGGA GGCAGCCGGC 3356
Pro Thr Leu Leu Ala Leu
375                 380

ACCCACAAGT GCCACTGCCC GAGCTGGTGC ATTACAGAGA GGAGAAACAC ATCTTCCCTA 3416

GAGGGTTCCT GTAGACCTAG GGAGGACCTT ATCTGTGCGT GAAACACACC AGGCTGTGGG 3476

CCTCAAGGAC TTGAAAGCAT CCATGTGTGG ACTCAAGTCC TTACCTCTTC CGGAGATGTA 3536

GCAAAACGCA TGGAGTGTGT ATTGTTCCCA GTGACACTTC AGAGAGCTGG TAGTTAGTAG 3596

CATGTTGAGC CAGGCCTGGG TCTGTGTCTC TTTTCTCTTT CTCCTTAGTC TTCTCATAGC 3656

ATTAACTAAT CTATTGGGTT CATTATTGGA ATTAACCTGG TGCTGGATAT TTTCAAATTG 3716

TATCTAGTGC AGCTGATTTT AACAATAACT ACTGTGTTCC TGGCAATAGT GTGTTCTGAT 3776

TAGAAATGAC CAATATTATA CTAAGAAAAG ATACGACTTT ATTTTCTGGT AGATAGAAAT 3836

AAATAGCTAT ATCCATGTAC TGTAGTTTTT CTTCAACATC AATGTTCATT GTAATGTTAC 3896

TGATCATGCA TTGTTGAGGT GGTCTGAATG TTCTGACATT AACAGTTTTC CATGAAAACG 3956

TTTTATTGTG TTTTTAATTT ATTTATTAAG ATGGATTCTC AGATATTTAT ATTTTTATTT 4016

TATTTTTTTC TACCTTGAGG TCTTTTGACA TGTGGAAAGT GAATTTGAAT GAAAAATTTA 4076

AGCATTGTTT GCTTATTGTT CCAAGACATT GTCAATAAAA GCATTTAAGT TGAATGCGAC 4136

CAACCTTGTG CTCTTTTCAT TCTGGAAGTC TTGTAAGTTT CTGAAAGGTA TTATTGGAGA 4196

CCAGTTTGTC AAGAAGGGTA GCTGCTGGAG GGGGACACAC CCTCTGTCTG ATCCCTTATC 4256

AAAGAGGACA AGGAAACTAT AGAGCTGATT TTAGAATATT TTACAAATAC ATGCCTTCCA 4316

TTGGAATGCT AAGATTTTCT ACTGCTTCTG GGGACGGGAA ACCGCTGTGT AACAGCTTTT 4376
GTGGGAATAC ATTTTTTCTG TTTCAGTACT CGCAGGGGGA AATATTTAAA TTTTGTTGTG 4436
CTAATATTAA ATTCAGATGT TTTGATCTTA AAGGAACCCT TTAAGCAAAC AGAACCTAGC 4496
TTTGTACAGA CTATTTTAAC TTTTTATTCT CACAAAATCA CGTGGAGGGT TATTCTACTT 4556
CAAAGATGAG CAAATTGAAG AATGGTTAGA ATAAACAACT TTCTTGATAT TCCGTTATCG 4616
GCATTAGAAT CTTCCTGCTC GTTATCGTAT CCAGCAGGCT GAACTGCCTC TTGATACTTG 4676
GTTAAAAAAA ATTTTCAGGC CGGGCGCGGT GGCCCATGCC TGTAATCCTA GCACTTTGGG 4736
AGGCCGAGGC AGGCGGATCA CCTGAGGTCG GGAGTTCGAG ACCAGCCTGA CCAACATGGA 4796
GAAACCCCGT CTTTACTAAA AATACAAAAT TAGCCTGGTG TGGTGGTGCA TGCCTGTAAT 4856
CCTAGCTACT TGAGAGGCTG AGACAGGAAA ATCACTTGAA CTCGGGAGGC GGATGTTGCA 4916
GCGAACTGAG ATTGCGCCAT TGCACTCCAG CCTGGGCAAC AAGATTGAAA CTCTGTTTAA 4976
AAAAAAAAGT TTTCACTAAT GTGTACATTT TTTTGTACTC TTTTATTCTC GAAAGGGAAG 5036
GAGGGCTATT GCCCTATCCC TTATTAATAA ATGCATTGTG GTTTCTGGTT TCTCTAATAC 5096
CATATGCCCT TCATTCAGTT TATAGTGGGC GGAAGTGGGG GAGAAAAAGT TGCTCAGAAA 5156
TCAAAAGATA TCTCAAACAG CACAAATAAT GGCTGATCGT TCTGCAAACA AAAAGTTACA 5216
TAATAGCTCA AGAAGGAGAA GTCAACATGA CTCTGAACAA GCTTTAACTT AGAAACTTTA 5276
TCATCTTAAG GAAGAACGTG ACCTTTGTCC AGGACGTCTC TGGTAATGGG GCACTTACAC 5336
ACACATGCAC ACGTACAAAC CACAGGGAAA GGAGACCGCC CTTCTGCCTC TGCTCGCGAG 5396
TATCACGCAG GCACCATGCA CTATGTTTTC ACACACACTG GGTGGAAGAA GAGCTTCAGC 5456
GCCAGTCTTC TAATGCTTTG GTGATAATGA AAATCACTGG GTGCTTATGG GGTGTCATAT 5516
TCAATCGAGT TAAAAGTTTT AATTCAAAAT GACAGTTTTA CTGAGGTTGA TGTTCTCGTC 5576
TATGATATCT CTGCCCCTCC CATAAAAATG GACATTTAAA AGCAACTTAC CGCTCTTTAG 5636
ATCACTCCTA TATCACACAC CACTTGGGGT GCTGTTTCTG CTAGACTTGT GATGACAGTG 5696
GCCTTAGGAT CCCTGTTTGC TGTTCAAAGG GCAAATATTT TATAGCCTTT AAATATACCT 5756
AAACTAAATA CAGAATTAAT ATAACTAACA AACACCTGGT CTGAAATAAC AAGGTGATCT 5816
ACCCTGGAAG GAACCCAGCT GGTGGGCCAG GAGCGGTGGC TCACACCTGT AATTCCAGCA 5876
CTTTGGGAGG CTGAGACAGG AGGATCACTG GAGTCCAGGA GTTTGAGACC AGCCTGGGCA 5936
ACATGGCAAA ACCCAGTGTG CTTCTGTTGT CCCAGCTACA CTACTCAGGA GGCTGAGGCA 5996
GGAGTATGAC TTGAGCCTGG GAGGGGGAGG TTGCAGAGAA CTGATATTGC ACCACCACTG 6056

CACTCCAGCC TGGGTGACAC AGCAAAACCC TATCTCAAAA AAAAAAAAAA AAAAAAGGAA 6116

CCCAGCTGGT TCCTGTAGGT GTGCAATAAT AACAACCAGA GGAAGAAAAG GAAGACGATT 6176

TCCCAGATGA AGAAGGGCAG CTGGACCTTC GGAC 6210

(2) INFORMATION FOR SEQ ID NO: 7:

(i) SEQUENCE CHARACTERISTICS:
(A) LENGTH: 380 amino acids
(B) TYPE: amino acid
(D) TOPOLOGY: linear

(ii) MOLECULE TYPE: protein

(xi) SEQUENCE DESCRIPTION: SEQ ID NO: 7:

Met Met Phe Ser Gly Phe Asn Ala Asp Tyr Glu Ala Ser Ser Ser Arg
1               5                   10                  15

Cys Ser Ser Ala Ser Pro Ala Gly Asp Ser Leu Ser Tyr Tyr His Ser
            20                  25                  30

Pro Ala Asp Ser Phe Ser Ser Met Gly Ser Pro Val Asn Ala Gln Asp
        35                  40                  45

Phe Cys Thr Asp Leu Ala Val Ser Ser Ala Asn Phe Ile Pro Thr Val
    50                  55                  60

Thr Ala Ile Ser Thr Ser Pro Asp Leu Gln Trp Leu Val Gln Pro Ala
65                  70                  75                  80

Leu Val Ser Ser Val Ala Pro Ser Gln Thr Arg Ala Pro His Pro Phe
                85                  90                  95

Gly Val Pro Ala Pro Ser Ala Gly Ala Tyr Ser Arg Ala Gly Val Val
            100                 105                 110

Lys Thr Met Thr Gly Gly Arg Ala Gln Ser Ile Gly Arg Arg Gly Lys
        115                 120                 125

Val Glu Gln Leu Ser Pro Glu Glu Glu Glu Lys Arg Arg Ile Arg Arg
    130                 135                 140

Glu Arg Asn Lys Met Ala Ala Ala Lys Cys Arg Asn Arg Arg Arg Glu
145                 150                 155                 160

Leu Thr Asp Thr Leu Gln Ala Glu Thr Asp Gln Leu Glu Asp Glu Lys
                165                 170                 175

Ser Ala Leu Gln Thr Glu Ile Ala Asn Leu Leu Lys Glu Lys Glu Lys
            180                 185                 190

Leu Glu Phe Ile Leu Ala Ala His Arg Pro Ala Cys Lys Ile Pro Asp
        195                 200                 205

Asp Leu Gly Phe Pro Glu Glu Met Ser Val Ala Ser Leu Asp Leu Thr
    210                 215                 220

Gly Gly Leu Pro Glu Val Ala Thr Pro Glu Ser Glu Glu Ala Phe Thr
225                 230                 235                 240

Leu Pro Leu Leu Asn Asp Pro Glu Pro Lys Pro Ser Val Glu Pro Val
                245                 250                 255

Lys Ser Ile Ser Ser Met Glu Leu Lys Thr Glu Pro Phe Asp Asp Phe
            260                 265                 270

Leu Phe Pro Ala Ser Ser Arg Pro Ser Gly Ser Glu Thr Ala Arg Ser
        275                 280                 285

Val Pro Asp Met Asp Leu Ser Gly Ser Phe Tyr Ala Ala Asp Trp Glu
    290                 295                 300

Pro Leu His Ser Gly Ser Leu Gly Met Gly Pro Met Ala Thr Glu Leu
305                 310                 315                 320

Glu Pro Leu Cys Thr Pro Val Val Thr Cys Thr Pro Ser Cys Thr Ala
                325                 330                 335

Tyr Thr Ser Ser Phe Val Phe Thr Tyr Pro Glu Ala Asp Ser Phe Pro
            340                 345                 350

Ser Cys Ala Ala Ala His Arg Lys Gly Ser Ser Ser Asn Glu Pro Ser
        355                 360                 365

Ser Asp Ser Leu Ser Ser Pro Thr Leu Leu Ala Leu
    370                 375                 380

(2) INFORMATION FOR SEQ ID NO: 8:

    (i) SEQUENCE CHARACTERISTICS:
        (A) LENGTH: 49 base pairs
        (B) TYPE: nucleic acid
        (C) STRANDEDNESS: double
        (D) TOPOLOGY: linear

    (xi) SEQUENCE DESCRIPTION: SEQ ID NO: 8:

GGCAGTGAGG TCAGGGGTGG GGAAGCCCAG GGCTGGGGAT TCCCCATCT                49

## Description

The present invention relates to anti-tumor cellular vaccines and more specifically to vaccines for providing immunotherapy by gene therapy to specifically control the generation of metastases.

Generally, there is a correlation between the expression of particular genes and HLA antigens as well as a correlation between the presence of certain tumor associated antigents (TAA) antigens and metastasis. However, there has been a problem in linking genetic control to control of metastasis and its inhibitions.

By way of background, malignant tumors arise from a protracted sequence of events. Each step in the sequence of events creates an additional phenotypic aberration (1). Research indicates that tumor cells acquire the ability to metastasize through genetic variation (2). Expression or loss of expression of specific genes has been associated with the metastatic phenotype of various cell lines (3,4).

Under normal conditions, the metastatic cells are subjected to attack by the host defense system, as long as the host defense system is competent to do so. An effective immune response capable of eliminating dissemination of tumor cells is predominantly mediated by cytotoxic T lymphocytes (CTL), which recognize proteolitically derived, foreign peptide epitopes bound to class I antigens of the major histocompatibility complex (MHC class I).

The inventors of the present invention reported that down regulation of MHC class I antigen was correlated with high malignancy in human and murine tumors (5). Transfection of MHC class I genes, particularly of H-2K genes was shown to confer on these tumors high immunogenic and low metastatic phenotypes.

Applicants previously found by testing high and low metastatic tumor cell populations that there was a correlation between the relative expression of class I antigens on different clones of a malignant carcinoma and the expression of the *c-fos* protooncogene (6,7). Although the involvement of oncogenes in the cellular transformation and tumorigenesis is apparently well established, the connection between oncogene or protooncogene expression and the metastatic competence of tumor cells remains unclear. Hence, use of this system clinically has not yet been perfected.

Studies by applicants suggest that the *c-fos* protooncogene participates in the control of MHC gene expression as evidenced by the following three studies. First, the *c-fos* gene was expressed in cells of the low metastatic clones of the 3LL tumor at much higher levels, for both *c-fos* transcripts and proteins, relative to cells of the high metastatic clones of the 3LL tumor (7). When the high metastatic clones were treated by interferon, a transient elevation of *c-fos* expression was observed followed by induction of H-2 transcription and there was a quantitative correlation between *c-fos* and H-2 induction (7,8). Second, a temporal correlation was found between the expression of the MHC class I antigens and the expression of *c-fos* antigens in a number of differentiating leukemic cells (9). Human U937 histiocytic lymphoma cells and HL60 promyelocytic leukemia cells, induced to differentiate to macrophages by 12-*O*-tetradecanoylphorbol 13-acetate (TPA), show induction of *c-fos* and HLA expression (9). In murine erythroleukemic cells, dimethylsulfoxide induced a decline in constitutive *c-fos* levels that were accompanied by suppression of MHC expression (9). Thirdly, transfection with *c-fos* genes of the clone D122 (3LL carcinoma), was shown to induce the transcription of H-2K mRNA and to elevate the levels of H-2 proteins, but not some of the other gene products (7,8).

Of critical importance is the finding that the rapid and transient induction of *c-fos* following cell stimulation by different external signals has established *c-fos* as a key member of the immediate early gene family and has implicated Fos in signal transduction and the control of cell proliferation (11,12) as well as in cell differentiation (13). Moreover, the Fos gene acts as a transcriptional regulator whose function is dependent upon formation of heterodimeric complexes with members of the *jun* family of protooncogenes (*c-jun*, *junB*, and *junD*) and further it binds to AP-1 consensus sequences, to CREB sequences and to Ap1/CREB like variations in the regulatory regions of target genes (12,14,15,16).

Applicant herein provides evidence of control of the *c-fos* and the unexpected results obtained thereby, and in combination with *c-jun*, control and/or inhibit metastasis in human tumors and murine models which are strongly predictive of human response. Based on experimental evidence herein relating to human tumor cells and HLA genes, applicant has determined the ability of the transfection and expression of particular genes to cause a dramatic decrease of tumorigenic and metastatic properties of various human tumors. Accordingly, applicant has derived an antitumor cellular vaccine and also a subcellular vaccine more specifically based on specifically expressed antigens for controlling tumorigenicity and metastatic properties of human tumor cells.

In accordance with the present invention, there is provided an antitumor cellular vaccine comprising tumor cells into which a *c-fos* gene (SEQ ID No:6) alone or together with *c-jun* gene (SEQ ID No:3) has been inserted.

Additionally, the antitumor cellular vaccine of the present invention is suitable for treating a patient suffering from cancer to prevent and/or inhibit the development of metastasis.

Further, the present invention provides an antitumor vaccine comprising antigens expressed by the *c-fos* gene alone or together with the *c-jun* gene.

Other advantages of the present invention will be readily appreciated as the same becomes better understood by reference to the following detailed description when considered in connection with the accompanying drawings wherein:

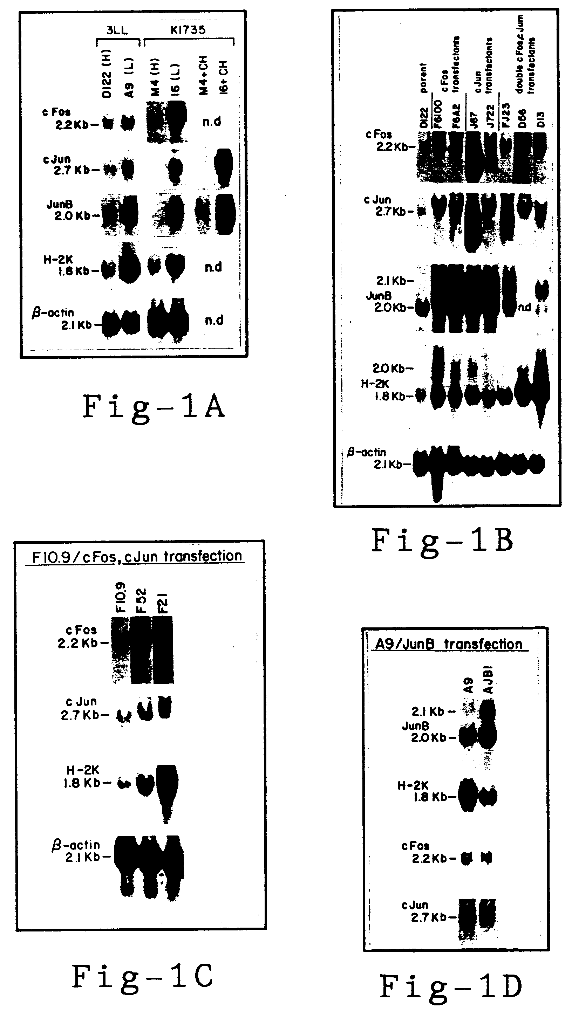

Figures 1A-1D show expression of *c-fos*, *c-jun*, *junB* and H-2K^{b}: (A) in low (L) and high (H) metastatic clones of 3LL and K1735 tumors, (B) in D122 clones (3LL) transfected by *c-fos*, *c-jun*, or both, (C) in clone F10.9 (B16) transfected by *c-fos* and *c-jun*, and (D) in clone A9 (3LL) transfected by *junB*;

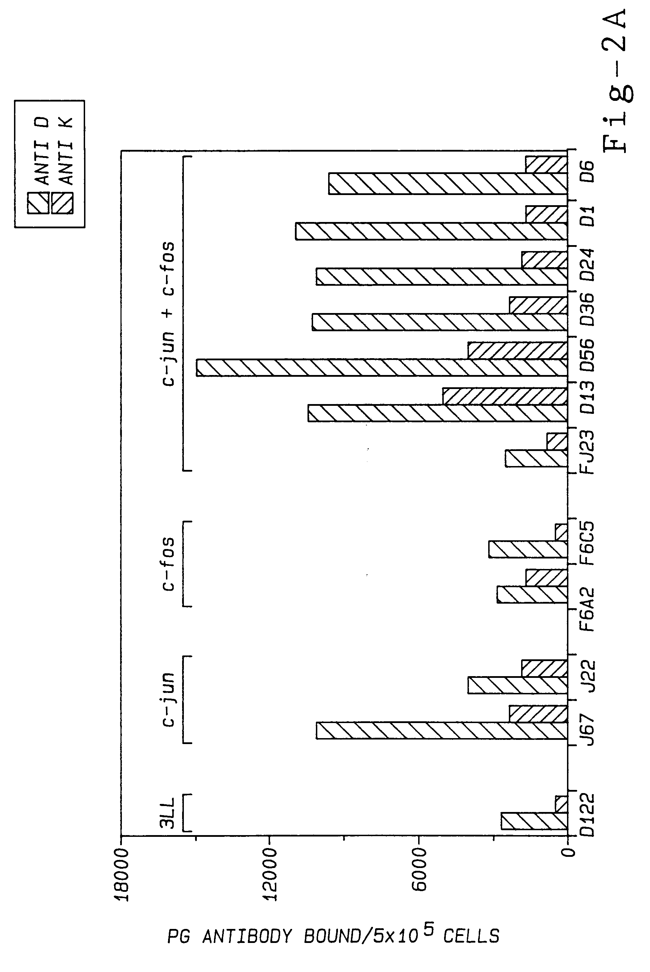

Figures 2A-2C show cell surface expression of MHC class I: (A) on clone D122 (3LL) and transfectants, (B) on clone F10.9 (B16) and transfectants, and (C) on clone A9 (3LL) and *junB* transfectants, where direct RIA were performed as described in experimental procedures and results are the means of five experiments, Standard errors were under 10% of the means;

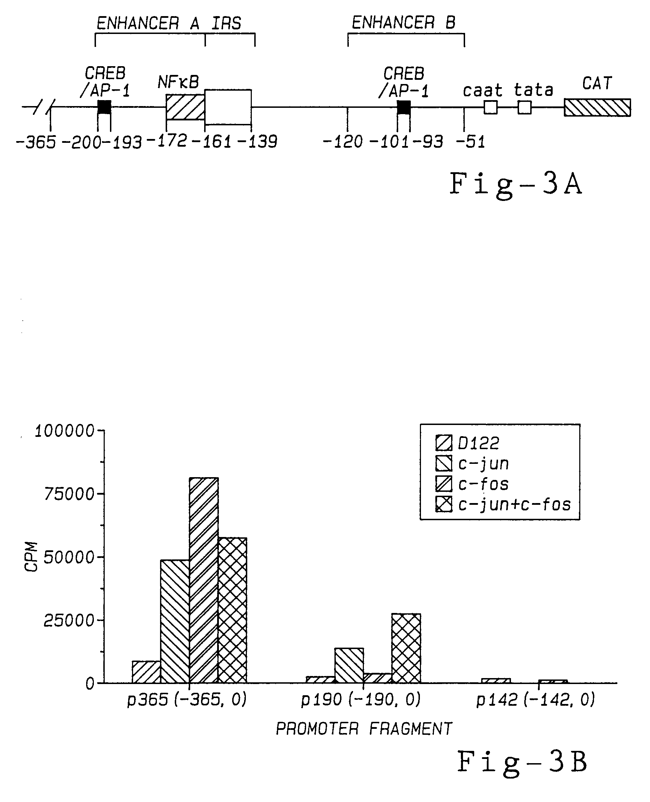

Figures 3A-3D show regulation of MHC class I promoter activity by *c-fos*, *c-jun* and *junB*: (A) schematic drawing of the K^{b} enhancer - promoter domain -365 to 0 and some of the known binding elements, (B) D122 (-), J67 (*c-jun*), F6A2 (*c-fos*) and D13 (*c-fos*+*c-jun*) stable transfectants were transiently transfected by H2 promoter - CAT constructs, or (C) by collagenase - CAT constructs, (D) the A9 (-) and AJB1 (*junB*) stable transfectants, were transiently transfected by the H2 promoter - CAT constructs;

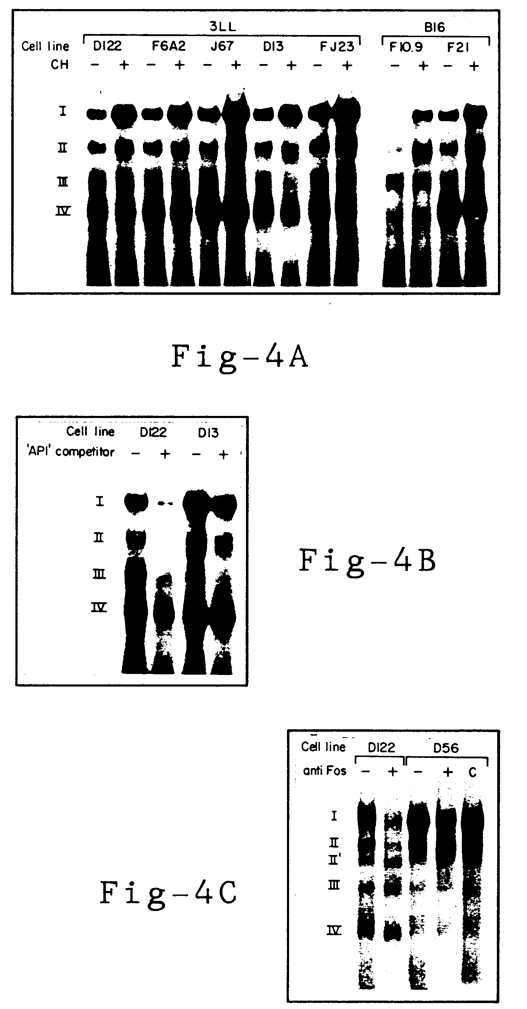

Figures 4A-4C show specific DNA binding activity of nuclear proteins from *c-fos*/*c-jun* transfectants, to the K^{b} enhancer A: (A) nuclear extracts from D122 and F10.9 transfectants, untreated (-) or treated (+) with cycloheximide were reacted at room temperature with enhancer A oligonucleotide and separated on acrylamide gel as described in experimental procedures, (B) reactions were performed as in A, in presence of 25 molar excess of competitor 'AP1', (C) nuclear extracts were incubated with the rabbit anti-*fos*-β-galactosidase serum (+) or anti H-2D^{b} nonrelevant antibodies (c) for 60 minutes at 4°C, probes were added and incubation was continued as before;

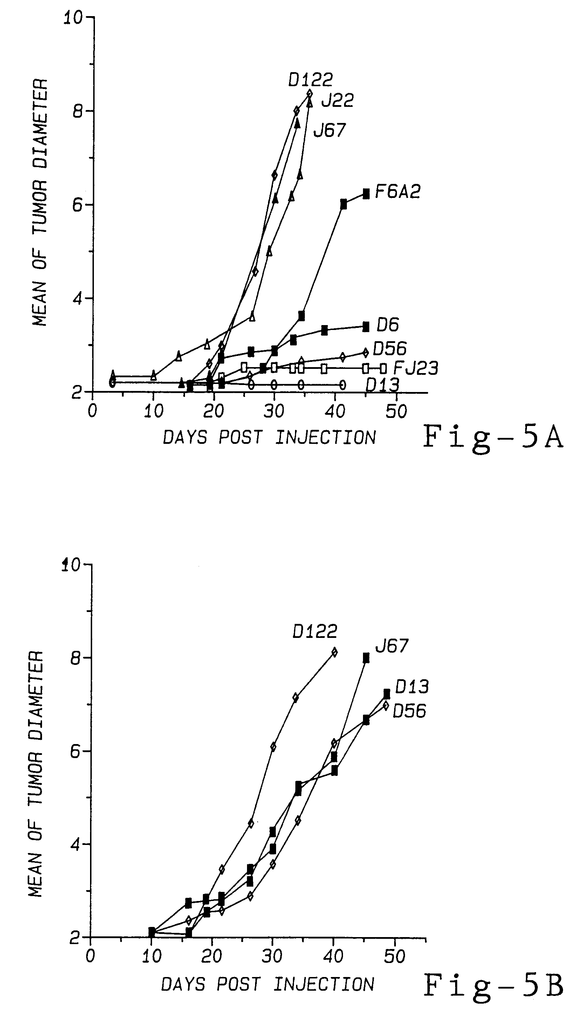

Figures 5A-5C show the effect of *c-fos* / *c-jun* transfection on tumorigenicity and metastasis, (A) D122 transfectants were injected i.f.p. into C57BL (A) or CD1 nude (B) mice, wherein foot diameters were measured as described in experimental procedures and the mean of tumor diameter of 16 mice in two experiments are shown, (C) D122, F10.9, A9 and transfectants were injected i.v. into C57BL mice;

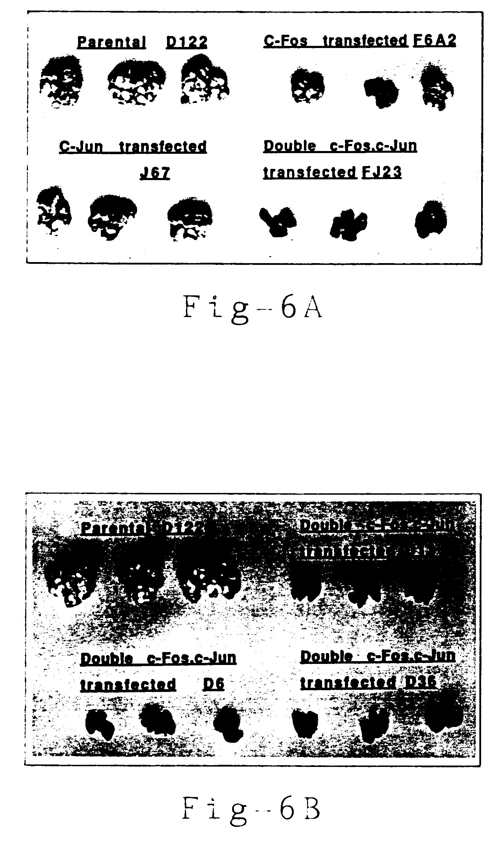

Figures 6A-6D show experimental metastatic potential of D122, F10.9, A9 and transfectants: (A,B) D122, F6A2, J67, FJ23, D13, D6 and D36 clones, (C,D) F10.9, F21, F33, F32 and F52 clones wherein lungs were removed, weighed, fixed in Bouin solution and clarified in 70% ethanol; and

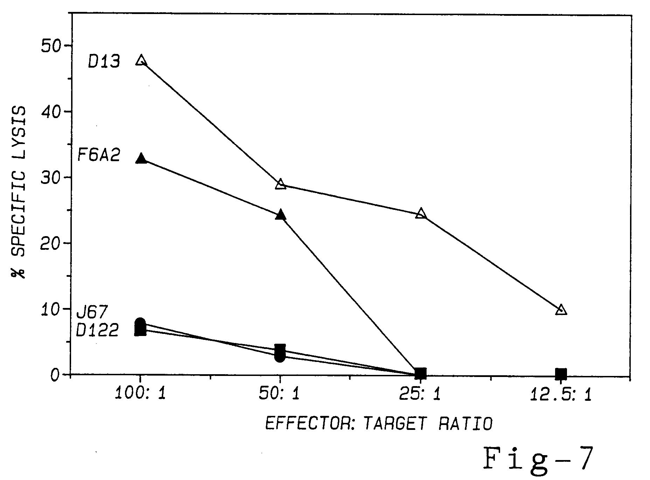

Figure 7 shows lytic activity of CTLs induced by D122 and transfectants on homologous target cells, C57BL mice were immunized with D122(-), J67 (*c-jun*), F6A2 (*c-fos*) and D13 (*c-fos*+*c-jun*) as described and EL4 (C57BL thymoma) and Yac-1 (an NK sensitive target) were used as control target cells, and were not lysed by any of the effector cells.

Generally, the present invention provides an antitumor vaccine which when injected into an animal produces stimulation of cytotoxic T lymphocytes (CTL) to produce an antitumorigenic and antimetastatic immune response. That is, the injection of the antitumor vaccine, whether in cellular or subcellular form, results in cytotoxic T cell lymphocyte activation which reduces or inhibits the generation of metastasis.

Specifically, the antitumor vaccine preferably is a cellular vaccine comprising tumor cells into which *c-fos* gene (SEQ ID No:6) alone, or the *c-fos* gene in combination with the *c-jun* gene (SEQ ID No:3), have been inserted. The tumor cells can be selected from a wide variety of tumor cells, preferably cells which can be efficiently and easily transfected with the above mentioned genes. For example, the cells can be carcinoma or melanoma cells, as well as other types of tumor cells. These cells can be obtained from the patient per se or can be obtained from a cell line not originally from the patient.

Tumor cells from the patients will be obtained either from biopsies, surgical material such as primary tumors, metastases in lymph nodes or other metastases, or in some cases from pleural effluents. Cell lines will be selected on the basis of the type of cancer: melanoma lines for melanoma patients, breast carcinoma lines for breast carcinoma patients, etc. The cell lines will be HLA matched. This means that HLA typing will be performed on each patient and cell lines which match in at least one HLA class I allele to the patient will be selected. If necessary a combination of cell lines will be used, each matching in at least one allele to the patient HLA. For example, a patient that is A1; A2; B7; B27; CW3; CW4 may get a vaccine consisting of six cell lines, each carrying one of these alleles. Additionally, if TAA typing is available, a vaccine that is also matched by TAA screening and HLA screening will be constructed. Preferably, the tumor cells are derived from tumor cells having metastatic competence, those cells being at least similar if not the same as the cells already existing in the patient. Such cells are rendered ametastatic by methods described below.

The means for inserting the *c-fos* gene or the *c-fos* gene together with the *c-jun* gene can be accomplished by methods known in the art. For example, the cellular vaccine can comprise human tumor cells transfected with either the *c-fos* gene alone or both *c-fos* gene and *c-jun* gene. For example, the two genes, the *c-fos* gene and the *c-jun* gene, can be introduced into the tumor cells on a single expression vector enabling constitutive production of the *c-fos* and *c-jun* gene products *in vivo*. Such methods have been previously detailed (7,23,44). Alternatively, the genes can be introduced into the tumor cells on different expression vectors enabling constitutive Production of the gene products *in vivo* by methods similar to those for the joint introduction, however, wherein each gene is transfected on an independent expression vector. Of course, consistent with the present invention, the *c-fos* gene alone can be introduced into the tumor cells on an expression vector.

A mammalian expression vector is a DNA construct that contains elements necessary for expression in mammalian cells, such as promoters, enhancers, termination and poly A signals, splice signals and of course a cDNA or genomic structure of the gene of interest. Such vectors may also contain sequences that enable them to replicate in bacteria, in which case the plasmid will contain bacterial replication signals and a gene for selection in a particular antibiotic such as an ampicillin-resistance gene.

Examples of expression vectors known in the art are genomic clones that contain autologous promoters and other processing signals (7); plasmid based vectors that do not carry a eukaryotic replicon, such as PTK2 (Wigeler, et al., 1977, Cell 11:223); PSV₂neo (Souther and Berg, 1982, J. Mol. Appl. Genet. 1:327); PRO-neo (Van Doren et al., 1984, J. Virol. 50:606); PSV₂gpt (Mulligan and Berg, 1980, Science, 209:1442), and others known in the art; plasmid DNA expression vectors containing regulatory sequences from eukaryotic viruses such as simian virus 40 vectors (Solnick, D., 1981, Cell 24:135), bovine papilloma virus based vectors (Sarver et al., 1981, Mol. Cell Biol. 1:486), Epstein-Barr virus based vectors (Yates et al., 1985 Nature 313:812). In addition Retroviral vectors (Gilboa et al., 1986, Biotechniques 4:504), Adenovirus (Berkner K.L., 1988, Biotechniques 6:616), and Vaccinia virus (Fuerst et al., 1987, Mol. Cell Biol. 7:2538) which are used as infectious particles but do not replicate in the recipient cell because of structural manipulations can be used.

With some expression vectors, such as retroviral vectors or adenovirus based vectors, the structure of the vector also suggests the method of gene transfer (infection). With other expression vectors, different gene transfer methods, which are the methods of introduction of DNA containing the expression vector into the target cells, are used. Examples of gene transfer methods are: calcium phosphate (40), Lyposomes (46), DEAE Dextran (47), Polybrene (48), Protoplast fusion (49), and others known in the art.

In each of the above mentioned methods, gene transfer is performed before cell inactivation, if the cells are to be inactivated.

Preferably, the tumor cells used for the antitumor cellular vaccine are inactivated, although inactivation is not necessary in all instances. Inactivation of the tumor cells is preferably performed by irradiation with gamma source at 3,000 to 10,000 Rad or by treatment with mitomycin C at concentrations of 30 to 100 µg/ml for one hour (50). An alternative is to utilize both treatments to insure inactivation of the tumor cells (51). Of course, other methods known in the art, such as fixation in glutaraldehyde (0.1 to 1%) for 5 to 30 minutes may also be used (52).

The *c-fos*, and *c-jun* genes are preferably cloned from cDNA libraries as set forth below under the Methods section. The *c-fos* + *c-jun* fused plasmid is described below also. The preferred method of transfection is described below under "preparation of stable transfectants". However, alternative methods can be used such as using the *c-fos* (SEQ ID No:6) and *c-jun* (SEQ ID No:3), both of human origin under the control of various promoters, such as the viral long term repeats (LTR) of various origins, under the control of a β actin promoter, or under strong promoters of enzymes, such as DNA polymerase type III as known in the art (53).

Preferably, the vaccine is formulated as an injection. The vaccine can contain inactivated tumor cells, inactivated as described above, in a saline or phosphate buffered saline. Preferably, no adjuvants would probably be required although alternative methods of preparation can include adjuvants such as BCG (54) or Alum (55), may be considered for use. Intactness of cells may be important for antigen presentation and cytokine release. Adjuvants usually cause cell lysis, for example Freund's complete adjuvant (56).

Preferably, the vaccine would include 1 x 10⁶ to about 1 x 10⁹ transfected tumor cells. Such dosages in human patients would be monitored either by skin tests or by reactivity of lymphocytes in one of the following methods: 1) nontransfected and transfected cells after inactivation are intradermally injected into the thigh or upper arm of the patient and DTH activity is measured by diameter of both the erythema and induration at 24 and 48 hours. Such instances would normally be negative. The patient is vaccinated and retested every two to four weeks. Maximal DTH is an indication for sufficient vaccination. 2) Blood can be drawn from the patient before vaccination. White blood cells are separated and viably frozen. Similar samples are taken at various time points after vaccination and booster injections. The ability of the white blood cells from different stages of vaccination to lyse nontransfected and transfected cells is measured in a CTL (cytotoxic T-lymphocyte) assay (57). Stabilization of the lytic activity is a measure for maximum vaccination.

The antitumor vaccine made in accordance with the present invention need not necessarily be a cellular vaccine, what is critical is that the vaccine include immunogenically competent HLA antigens derived from the expression of the *c-fos* gene alone or together with the *c-jun* gene and immunogenic tumor associated antigen (TAA) derived peptides. Such preparations can be made from cell membrane preparations of the transfected tumor cells. The difficulty of this approach is that such membrane preparations can hide the antigens depending upon the folding of the membranes. However, such vaccines have been made in the art for other preparations (58).

The vaccines of the present invention are suitable for the treatment of a patient suffering from cancer, the treatment preventing and/or inhibiting the development of metastasis.

Generally, this treatment includes a step of administering to the patient the vaccine described above. More specifically, the patient would be treated as follows. First, cells from the primary tumor of the patient would be removed by biopsy or surgery. As discussed above, an alternative approach would be to use cells derived from other patients. In either case, the cells would be dispersed in a medium, such as Dulbecco's modified eagle medium (DMEM), RPMI or other mediums used for the preparation of cellular vaccines. A vector, such as the vectors discussed above, is inserted into the cells. The vector would comprise the human *c-fos* gene or the human *c-fos* and *c-jun* genes. During this step, the positive transfectants that are high expressers of *c-fos* and MHC class I genes can be selected either by an appropriate antibiotic (G418 if a neo resistant gene is present on the vector, (59) or by binding of anti-HLA antibodies and enrichment by a Fluorescence Activated Cell Sorter (FACS). The transfectants are inactivated as described above by gamma or x-ray irradiation and/or treatment with mitomycin C as described above. Finally, an effective amount of inactivated tumor *c-fos* or *c-fos* and *c-jun* transfected cells is administered into the patient, preferably by injection. A highly immunogenic vaccine is thus obtained for preventing and/or inhibiting tumor metastasis in the patient.

The following experimental evidence provides a basis for utilization of the present invention and its effectiveness in decreasing and/or preventing metastasis. Further, experimental evidence demonstrates the mechanism of action by which cytotoxic T lymphocytes (CTL) are activated to produce the immunogenic response.

### EXPERIMENTAL PROCEDURES

### TUMOR CELLS

Tumor cells were maintained in DMEM, 10% fetal calf serum, and supplements (6). The Lewis lung carcinoma (3LL) and the B16 melanoma (B16) are malignant tumors which originated spontaneously in C57BL/6 (H-2^{b}) mice (17,18). A9 and D122 are low and high metastatic clones, respectively, cloned from the 3LL carcinoma cells by limiting dilution (6). The high metastatic line B16-F10 was selected from B16 by I.J. Fidler (17). F10.9 is a high metastatic single cell clone derived from the B16-F10 line (19). The primary K1735 melanoma arose in an inbred C3H/HeN mouse following a short exposure to U.V. irradiation, it was transplanted once into an immunosuppressed recipient and then established in culture (20). The low-metastatic line 16 and the high metastatic line M4 were selected from K1735 by I.J. Fidler (20).

### ASSAYS FOR TUMORIGENICITY AND METASTASIS

C57BL/6J (Jackson Laboratories, Bar Harbor, Maine) or CD1 nude mice (Weizmann Institute Breeding Center) were injected with 2X10⁵ 3LL or B16 tumor cells and C3H/HeN (Jackson Laboratories, Bar Harbor, Maine) mice were injected with 2X10⁵ K1735 tumor cells, intrafootpad (i.f.p.) in the right hindleg. To monitor tumor growth, the diameter of tumor bearing feet were measured every 1-3 days with calipers. When tumor diameter reached 8 mm, the tumor bearing foot was amputated. Survival and formation of spontaneous lung metastases was monitored at the time that mice became moribund. Experimental lung metastases were tested by injecting C57BL/6J or CD1 nude mice with 5X10⁵ 3LL or 5X10⁴ B16 tumor cells, and C3H/HeN mice with 5X10⁵ K1735 tumor cells, into the mouse tail vein. Mice were sacrificed 30-35 days later for D122, F10.9 and M4 (high metastatic) clones, and 65-70 days later for A9 (low metastatic) clones, and lungs were excised and weighted. Lung weights are the averages of three experiments. Maintenance and experimental procedures of mice were performed according to NIH guidelines.

### GENE EXPRESSION

RNAs were prepared from 1-3 X 10⁸ cells propagated in tissue culture or treated with 10µg/ml of cycloheximide for one hour, by the method of Chirgwin (21). Northern blots were prepared from formaldehyde-containing agarose gels loaded with 30µg total RNA per lane as described (7) and assayed by hybridization to [³²P] labeled probes. The following probes were used: *c-fos* - 1.2 kbp HincII-EcoRI fragment from the pBK28 fos plasmid (22), *c-jun* -1.1 kbp EcoRI-PstI fragment from chJ-2 plasmid (23), *junB* - 1.2kbp HincII-Nde I fragment from RSVjunB plasmid (24), for H-2K - the H-2K specific 30-mer oligonucleotide which codes for amino acids 148-157 of the H-2K^{b} protein. This oligonucleotide crossreacts with H-2K^{k} (26/30 nucleotides). For the β actin probe, a 4.3 kbp insert of pAC18.1 was used (25). Hybridizations were performed in 50% formamide at 42°. Blots were washed in 0.1SSC-0.2% SDS at 60°. Blots hybridized to oligonucleotide probes were washed in 0.5 SSC-0.2% SDS at 50°.

### PLASMIDS AND CONSTRUCTS

The plasmids Psv-cJun (23), pBK28 (22) and RSVjunB (24) have been previously described and characterized by others. *c-fos*+*c-jun* fused plasmid (CON. 9) was constructed by ligation of a HindIII-NdeI fragment from Psv-cjun (containing SV40 early promoter, *c-jun* cDNA and SV40 polyadenylation signal) into HindIII linearized pBK28, *c-fos* expression vector, blunt ending and religation. The *c-jun* and *c-fos* are transcribed from separate promoters (SV40 and LTR, respectively) and the construct contains two poly A addition signals. PUC-365-CAT was prepared by cloning of a BamHI-XbaI restriction fragment from pH-2CAT (39) into a Puc19 vector. PUC-142-CAT and PUC-190-CAT were subcloned from p138H-2K CAT and p190H-2K CAT, respectively, into Puc19 vector (26), p38-H-2K-CAT, the collagenase promoter-CAT constructs, -73COL·CAT and -60COL·CAT (32) and the β galactosidase containing expression vector PCH110 (Pharmacia, Inc.) were described before by others.

### PREPARATION OF STABLE TRANSFECTANTS

Transfections of *c-jun*, *junB*, *c-fos* or *c-fos*+*c-jun* (CON.9) expression vectors into the high metastatic clone D122 (3LL), transfection of *c-fos*+*c-jun* (CON.9) into the high metastatic clone F10.9 (B16) and the transfection of *junB* into the low metastatic clone A9 (3LL) were performed by the calcium phosphate technique, in cotransfection with a 1:9 or 1:19 ratio of PSV₂neomycin resistance gene (PSV₂neo) (40). Control transfection was done with PSV₂neo alone. To increase the efficiency of transfection, a 10 minute treatment with DMEM-15% DMSO (dimethylsulfoxide) was performed after incubation with DNA precipitates. Colonies growing in 400µg/ml G418 (GIBCO) four weeks after transfection were expanded and analyzed for expression of the inserted genes by Northern blot analysis as described above. The selected D122 *c-fos* high expressor F6A3 clone was further supertransfected by the *c-jun* expression vector, in cotransfection with 1:9 ratio of pSV2 hygromycin B resistance plasmid (pSV₂hygro) and the selection was done in 200µg/ml hygromycin B containing media. Control transfectants carrying pSV₂neo or pSV₂hygro show hybridization patterns, MHC class I expression and malignant properties equal to parental cells. (19)

### CELL-SURFACE H-2 ANTIGENS

Protein A-purified monoclonal antibodies 20-8-4 (αK^{b}) and 28-14-8 (αD^{b}) (27) were iodinated by chloramine T by standard protocols. Five hundred ng of labeled antibody were mixed with 5X10⁵ freshly trypsinized cells in 0.1 ml phosphate-buffered saline (PBS) in BSA-coated tubes. Triplicate samples were incubated at 0° for 90 minutes. After four washings in PBS-0.5% bovine serum albumin (BSA) 0.02% sodium azide, samples were monitored in a gamma scintillation counter.

### TRANSIENT EXPRESSION ASSAYS

The Chloramphenicol acetyl transferase (CAT) gene codes for an enzyme that transfers a labeled ¹⁴C acetyl or butyryl group from [¹⁴C] acetyl CoA or [¹⁴C] butyryl CoA to chloramphenicol. It serves as a "reporter gene": when the structural CAT gene is ligated to promoter sequences of various genes and these constructs are transfected (transiently or stably) into cells, one can measure the CAT activity in cell lysates and learn from the data about the activity of the promoter fused to CAT in the particular cell lines. There is almost no background in mammalian cells since CAT is a bacterial enzyme (41).

For transient assays, 1X10⁶ cells were plated in 10cm dishes, 24 hours before DNA transfection. Cells were incubated for 12 hours with calcium phosphate-precipitated plasmid DNAs (20µg of the CAT derivative plus 4µg of pCH110) then, following a 10 minute DMSO (15%, 37°C) shock, the cells were rinsed once, reefed with fresh medium and 24-48 hours later the cells were processed for enzymatic assays. CAT activity, using 30µg of total cell extract protein, was determined as described (41), and β-galactosidase activity was determined as described (42). Experiments were repeated at least three times. Activity of CAT was normalized to activity of β-galactosidase to correct for difference in transfection efficiency.

### GEL RETARDATION ASSAY

Nuclear extracts were prepared from 10X10⁶ cells and used in gel retardation assays as described (28). Protein concentration were determined using the Bradford method (60) and ranged from 4-7mg/ml. A synthetic double stranded 49 bp oligodeoxynucleotide containing the entire enhancer A was used as a probe. The sequence (SEQ ID No:8) of the enhancer A fragment is: 
GGCAGTGAGGTCAGGGGTGGGGAAGCCCAGGGCTGGGGATTCCCCATCT
GTCACTCCAGTCCCCACCCCTTCGGGTCCCGACCCCTAAGGGGTAGAGG
-205 -154
The AP1/CREB like nonlabeled competitor oligonucleotide was used in the binding assay (indicated by the broken line above domain -205 to -186 containing the AP1 like binding site in enhancer A (from -203 to -197). A nonrelevant 19-mer nonlabeled competitor oligonucleotide was used as a control.

DNA-protein binding was conducted in 20µl volumes. The nuclear extract (3-5µg) was incubated with 3µg of poly(dI·dC) (Pharmacia Inc.) for 15 minutes at room temperature. Approximately 0.1pmol of ³²P-labeled DNA (∼10,000 cpm) was added to the preincubated nuclear extract. Unlabeled competitor DNA was added to the binding reaction two minutes before the labeled oligomer. Rabbit anti-*fos*-β-galactosidase fusion protein antiserum (43), when used, was added to the nuclear extracts for one hour at 4°C, prior to ³²P-labeled DNA addition. No disruption of the nucleoprotein complex binding was observed when a control antisera was added to the extract. DNA-protein complexes were resolved on 4% polyacrylamide gel (39:1 acrylamide to bisacrylamde) in 0.4X TBE (1X TBE is 50 mM Tris-borate [pH 8.3] 1mM EDTA). The gels were dried and autoradiographed with intensifying screens at -70°C.

### IN VITRO LYTIC ACTIVITY (CTL) ASSAY

C57BL/6J mice were immunized intraperitoneal (i.p), three times at 7-day intervals, with 2 X 10⁶ tumor cells, irradiated (5000 Rad) and mitomycin C treated (80µg/ml/5X10⁶ cells). Spleen cells were removed 10 days after the last immunization and restimulated *in vitro* for 5 days with irradiated cells (as before) at ratio of 20:1 (responders/stimulators). Spleen cells were seeded at 4 X 10⁶ cells/ml, in RPMI medium containing 10% FCS, 0.4% combined antibiotics, 2 mM glutamine, 1mM sodium pyruvate, 1mM nonessential amino acids, 2 X 10⁵ M β-mercaptoethanol and 10 mM Hepes pH 7.4. On day 5, stimulated spleen cells were separated on lymphocyte preparation medium (Cedarlane, Ontario), washed three times with PBS and suspended at a concentration of 5 X 10⁶ cells/ml. Five thousand target cells labeled with ³⁵S-methionine (NEN) for 6 hours were suspended in 96 u-shaped wells and graded numbers of effector cells were added at E/T ratios of 100:1, 50:1, 25:1 and 12.5:1. Plates were incubated for 16 hours for D122 and *fos*/*jun* transfectant targets, and 5 hours for EL4 and Yac1 targets at 37°C, 5% CO₂. Microplates were spun at 1000 rpm for 10 minutes and 100-µl aliquots of supernatant were transferred into tubes, mixed with 3 ml scintillation fluid and monitored in a beta counter. Spontaneous release was determined by incubation of labeled cells with medium alone; maximal counts were determined by incubating the same target cells with 100µl 0.1 NaOH. Spontaneous release was below 20% of maximal release. Specific release is reported as the mean of triplicates values.

### RESULTS OF EXPERIMENTATION

### EXPRESSION OF c-fos, c-jun, junB AND H-2K GENES IN MURINE TUMORS.

Low metastatic 3LL clones were characterized as high expressors of *c-fos* and MHC class I genes in contrast to high metastatic 3LL clones that express low levels of both genes (7,29). To determine whether this correlation could be extended to other murine tumor systems the steady state mRNA expression of *c-jun*, *junB*, *c-fos* and H-2K (MHC) genes was assayed in K1735 melanoma (30) and B16 melanoma (17) metastatic tumors as well as in the Lewis lung carcinoma (3LL).

Northern blot analysis of the high metastatic clones D122 (3LL), M4 (K1735) and F10.9 (B16), and the low metastatic clones A9 (3LL) and 16 (K1735) is shown in Figure 1 (A and C). Hybridization was performed at 42°C in 50% formamide and 10% dextran sulfate for 36 hours. Blots were washed in 0.1 SSC, 0.1% SDS at 60°C. Hybridization to β actin probe was included as a measure for equal levels of RNA in the samples. The typical 2.2 kb *c-fos*, 2.7 kb *c-jun* and 2.0 kb *junB* transcripts appear in high metastatic D122 cells and at much higher levels in A9 (3LL) low metastatic cells (Figure 1A lanes 1-2). Thus the correlation observed before (6,7) between low metastatic potential and elevated expression of *c-fos* and H-2K is also observed for *c-jun* and to a lesser extent for *junB* expression. Cells of the low metastatic clone 16 (K1735) expressed high levels of the *c-jun*, *junB*, *c-fos* and H-2K transcripts (Figure 1A lane 4), while only minor levels of these transcripts were observed in cells of the high metastatic clones, M4 (K1735) Figure 1A lane 3) and F10.9 (B16) (Figure 1C lane 1). Several variants of the presumably low metastatic B16 line, F1 that were tested, were all high metastatic *in vivo* and show expression profiles similar to F10.9. Thus no direct correlation can be shown in the B16 tumor.

The differential expression of *junB* and *c-jun* in K1735 clones did not change after a cycloheximide (CHX) treatment, which is known to stabilize and superinduce *c-fos* and *c-jun* mRNA's (31). CHX treatment caused a significant induction of *c-jun* and *junB* mRNA's in clone 16 cells, but only a marginal elevation in the M4 cells (Fig. 1A panel 2 and 3, lanes 5-6). In accordance with H-2K mRNA levels, clone 16 cells manifested high levels of H-2K^{k} cell surface glycoproteins, as opposed to almost complete lack of H-2K^{k} antigens on the cell surface of M4 cells (data not shown). The expression of H-2D^{k} was similar between the two clones (data not shown).

### EFFECT OF c-jun, junB AND c-fos TRANSFECTION ON EXOGENOUS AND ENDOGENOUS GENE EXPRESSION.

The correlation between *c-fos* and *c-jun* gene expression and the low metastatic phenotype observed in 3LL carcinoma and in K1735 melanoma clones, raised questions concerning the cause-effect relation between these phenomena. To investigate the possibility that *c-fos* +*c-jun* family of genes play a role in regulating MHC class I expression, low K^{b} expressing D122 (3LL) cells were transfected with *c-jun*, *junB*, *c-fos* or *c-jun*+*c-fos* plasmids. Plasmids Psv-c-jun which contain the mouse *c-jun* cDNA (SEQ ID No:1) under SV40 early promoter control, RSV-JunB which contains the mouse *JunB* cDNA under an LTR control and pBK28 which contains the human *c-fos* (SEQ ID No:6) cDNA under *v-fos* promoter control were used. Cotransfection was carried out with PSV₂neo cDNA at 9:1 ratio and selection in neomycin (G418) followed.

The *c-jun* + *c-fos* transfections were done either by supertransfection of a *c-fos* positive D122 clone (F6A3), with *c-jun* and hygromycin-B resistance (psV2hygro) plasmids or by transfection of a *c-jun* + *c-fos* expression construct *Con.9*, which assures that *c-fos* and *c-jun* transfected genes would be integrated in a similar copy number and in the same place in the cell genome. The low K^{b}, D^{b} expressor clone F10.9 (B16), was also transfected with the *c-fos* + *c-jun* plasmid *Con.9*. Figure 1B demonstrates the expression patterns of the various D122 stable transfectants and the effect on expression of endogenous genes of the *fos* and *jun* family as well as on the expression of the endogenous H-2K genes. Expression of endogenous *junB* is highly induced by *c-fos* and *c-jun* overexpression, as demonstrated on the Northern blots in Fig. 1B (panel 3). Interestingly the parental D122 cells express a 2.0 kb transcript while the *c-fos* and *c-jun* transfectants express a closely spaced junB mRNA doublet of 2.0 and 2.1 kb. This doublet is likely to represent the use of different polyadenylation sites (44). Notably, transfectants of *c-fos*+*c-jun* (clones FJ23 and D13) express less *junB* transcripts than transfectants of either *c-fos* or *c-jun* alone.

The positive transcriptional regulation of the *junB* gene, by *c-jun* and *c-fos* has not been described before. However, the promoter of *c-jun* was shown to contain an AP1 binding site and *c-jun* (probably by Jun-Fos complex) is an efficient activator of its own expression (32). Since the gene for *junB* is structurally related to *c-jun* (33) it is conceivable that it might be similarly regulated.

*c-fos* transcript is overexpressed in *c-fos* and in *c-fos*+*c-jun* transfected clones (Fig. 1B panel 1). Since endogenous and exogenous transcripts are of a similar size, it is not clear whether the exogenous *c-fos* expression or *c-fos*+*c-jun* coexpression, actually reduced the endogenous *c-fos* expression. Unexpectedly *c-jun* transfected also show high levels of the 2.2 kb endogenous *c-fos* transcript (Fig. 1B panel 1).

Although repression of *c-fos* promoter, by the Fos protein has been demonstrated in NIH3T3 and HeLa cells (34), and this repression was enhanced by coexpression with *c-jun* (35), in several other cell systems, introductions of exogenous *c-fos* genes, did not down regulate the expression of the endogenous *c-fos* gene (36).

Moreover, introduction of *c-fos* into ES cells and production of chimeric c-fos mice showed that in these ES cells, in the chimeric mouse tissues and in tumor derived cell lines from these mice, both endogenous and exogenous *c-fos* RNA as well as *c-jun* RNA were highly elevated (37).

This may possibly be the result of the distinct cellular environment in the different cell lines. It has been previously suggested that the repression of *c-fos* is the result of *c-fos*+*c-jun* complex interaction with another cellular protein or alternatively Fos and Jun may act independently on the same or different target proteins (35). Thus, it may depend on the precise composition of the cellular proteins whether a repressing or an activating effect is achieved.

Increased levels of 2.7 kb endogenous *c-jun* transcripts are expressed in all *c-fos* transfected cells (Fig. 1B panel 2). Induction of *c-jun* in D122 cells transfected with human or mouse genomic *c-fos* were also observed (unpublished results). Fig. 1B panel 2 also shows *c-jun* overexpression in *c-jun* and double transfected cells. *c-jun* was shown to transactivate its own promoter (32), and in addition, the transfected *c-jun* cDNA (Psv-c-jun) is driven by the SV40 promoter, which contains two AP1 binding sites, and may also be positively regulated by *c-jun*. This can account for the very high mRNA *c-jun* observed in J67 and FJ23 cells.

To summarize, *c-fos* and *c-jun* transfections into D122 cells induced endogenous *junB* mRNA. In addition, *c-fos* or *c-jun* transfection resulted in a reciprocal transcriptional activation, namely, the *c-fos* transfected D122 cells showed increased levels of the endogenous *c-jun* transcripts and the *c-jun* transfected D122 cells showed an increase in the endogenous *c-fos* gene expression. A similar analysis of the RNA from *junB* transfected clones did not reveal significant changes in endogenous *c-fos* or *c-jun* expression compared to the parental D122 cells (data not shown).

### ELEVATED MHC CLASS I IN c-fos and c-jun TRANSFECTANTS, REDUCED H-2 IN junB TRANSFECTANTS.

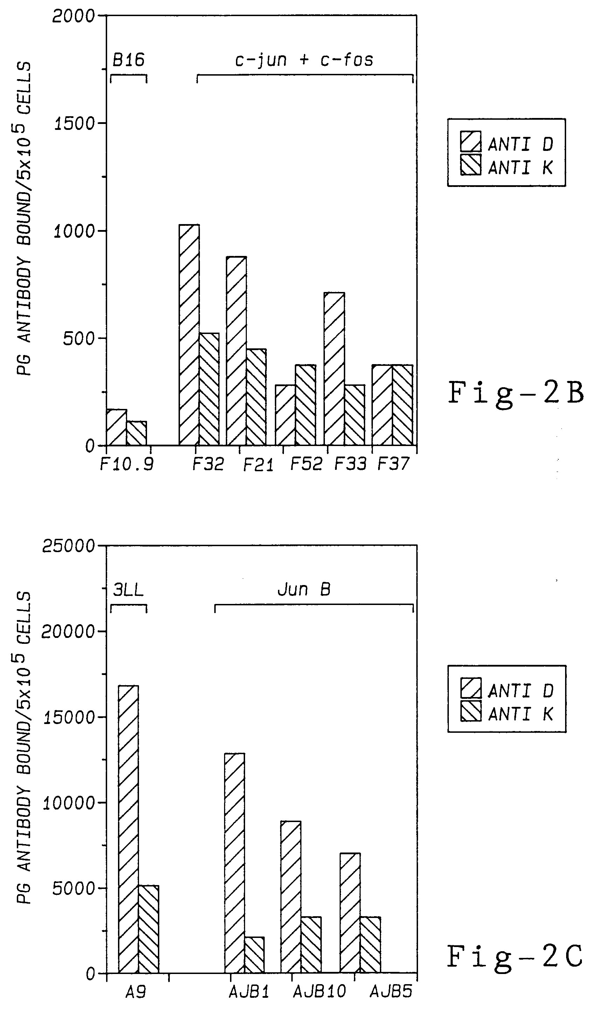

Hybridization of RNA extracted from the various *c-jun*, *c-fos* and double transfected cell lines and the parental D122 clone to an H-2K^{b} specific probe is shown at Fig. 1B panel 4. All the transfectants that expressed high levels of *c-fos* and *c-jun* mRNA show transcriptional activation of the 1.8 kb endogenous H-2K^{b} mRNA. The highest H-2K mRNA expressor among these transfectants is clone D13 which contains the fused *c-fos*+*c-jun* genes. Notably the D13 cells show the lowest levels of *junB* mRNA transfectants, 3 out of 12 *c-fos* transfectants and 11 out of 35 *c-fos*+*c-jun* double transfectants which did not express elevated *c-jun* and *c-fos* mRNA's, did not show activation of H-2K transcription (data not shown), while all *c-fos* and *c-jun* single and double positive clones showed elevated steady state levels of H-2 mRNA. The data shown in Figure 1C demonstrate similar results in F10.9 (B16 melanoma) cells, namely the positive stable F10.9 *c-jun*+*c-fos* transfected cell lines (F52 and F21) manifest a marked transcriptional activation of the H-2K^{b} gene relative to the parental, low expressor F10.9 clone (Fig. 1C panel 3). The H-2K^{b} mRNA expression pattern in D122, *junB* transfected clones remained very low, similarly to the parental D122 phenotype (data not shown). To evaluate the increase in H-2 related proteins in the *c-fos* and *c-jun* transfected D122 (3LL) and F10.9 (B16) clones, direct radioimmunoassays were performed using [¹²⁵I] labeled monoclonal antibodies to H-2K^{b} and H-2D^{b} antigens (27). Two to nine fold elevated levels of H-2K^{b} and 1.2-7 fold elevated H-2D^{b} expression on the cell surface of *c-fos*, *c-jun* and double transfected cells was observed (Fig. 2A and B).

These results indicate that MHC (H-2K and H-2D) expression from endogenous genes can be induced by *c-fos* and *c-jun* transfection, either by single gene transfer or by cotransfection with both genes. In contrast, the D122 *junB* transfected clones show a decrease in the H-2^{b} cell surface expression (data not shown).

To further examine the possibility that *junB* might down regulate MHC class I expression, the *junB* gene was transfected into the high H-2K^{b}, H-2D^{b} expressor A9 (3LL) clone. Figure 1D shows the hybridization of RNA extracted from the highest *junB* expressor clone AJB1 and parental A9 clone cells to the specific *junB*, *c-jun*, *c-fos* and H-2K probes. Most of the exogenously expressed JunB seems to appear as a 2.1 kb transcript in a closely spaced junB mRNA doublet of 2.0 kb and 2.1 kb while the endogenous *junB* transcript is primarily of the 2.0 kb (Fig. 1D, panel 1). The H-2K hybridization of pattern (Fig. 1D panel 2) indicates a decrease in the H-2K^{b} steady state mRNA levels in the transfected cells, relative to the parental high H-2K^{b} expressor A9 cells.

No significant changes were observed in *c-fos* and *c-jun* expression following the *junB* transfection (Fig. 1D, panels 3 and 4). Hybridization to β actin probe showed that equal amounts of RNA were used (not shown). Figure 2C demonstrates the cell surface expression of H-2K and H-2D proteins in the *junB* transfected clones. A decrease of 40%-70% below control (A9) is observed for the H-2K^{b} molecules and of 10%-40% for the H-2D^{b} molecules in A9 *junB* transfectants (Fig. 2C).

### ACTIVATION OF THE H-2 PROMOTER BY c-fos AND c-jun

To address the possible mechanism by which cJun, JunB and cFos proteins regulate H-2 expression, portions of the H-2K promoter were fused to the CAT gene and activities were monitored following transient transfections into the stable *jun(s)* and *c-fos* overexpressing cells.

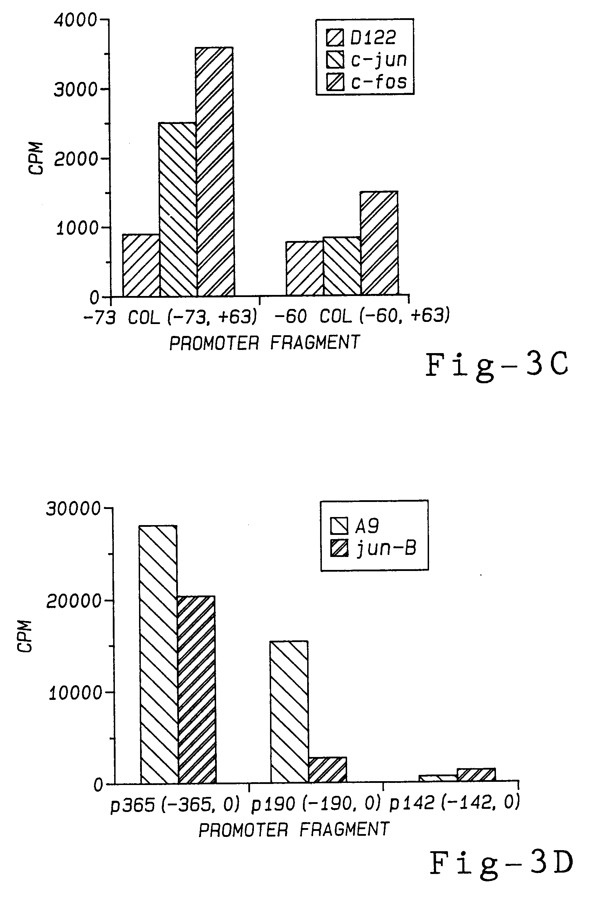

The plasmids PUC-365-CAT, PUC-190-CAT, PUC-142-CAT and p38-H-2K-CAT (p365, p190, p142, and p38 in Fig. 3) were used which are different deletion constructs of the 5' flanking region from the protein cap site, fused to the CAT gene (see fig. 3A). At least three separate assays were performed with each transfect as described in experimental procedures. Kinetics between 1-30 hours were monitored. Enzyme activities are linear up to 20 hours. Activities at 15 hours, of one representative assay are described. The construct PCH110 (containing the β-galactosidase gene fused to the SV40 promoter) was cotransfected with each of the CAT plasmids as an internal control to transfection efficiency.

As shown in Figure 3B high cJun, cFos expression of the transfected cells resulted in a marked activation of the p365 construct. The activation magnitude correlated well with the induction of H-2K mRNA, (Fig. 1B), and with the increase in H-2 related proteins on the cell surface (Fig. 2A) of the transfected clones. In contrast, transfection with p142 that contains enhancer B only (Fig. 3B) or with p38 that contains only the TATA box (data not shown), into parental D122 or *c-fos*, *c-jun* transfectants, yielded very low CAT activity. The lower activation of p190 compared to the activation of p365 in *c-fos* and/or *c-jun* expressor cells (Fig. 3B), indicates that the AP1 like binding sequence (-200 -193) plays a major role in the activation of H-2 by the cFos and cJun proteins. Yet the activation of the p190 construct in cJun and cFos/cJun expressors, relative to the nonexpressor D122, indicates that the domain downstream to -190 is also regulated by *c-jun* or *c-jun* + *c-fos*. This cannot be attributed to the AP1-like domain in enhancer B since the p142 construct is hardly active in all lines (Fig. 3B).

These data suggest that the region between -190 and -142 includes an additional target of activation by the Fos-Jun complex which is not directly mediated by an AP1-sites in the promoter, since the sequence between -190 -142 does not contain an obvious AP1 binding site. It is possible that another gene product that is regulated via TRE/AP1 is a regulator of the IRS, or of the NFkB binding domain (see Fig. 3A) or that the cJun or cFos proteins interact directly with one of the nuclear proteins that bind in the -190 -142 promoter domain (see also reference 38).

To verify that a classical AP1 enhancer is also activated in these cells, the constructs -73Col·CAT and -60Col·CAT were used which contain the collagenase promoter region from position -73 to +63 or -60 to +63 respectively, fused to CAT gene at position +63 (32). The two constructs differ from each other by the deletion of the AP1 sequence between -73 and -60 in the collagenase promoter. The basal level of CAT expression from these constructs in the absence of appropriate stimulating factors is usually very low. The CAT expression following transient transfections of -73Col·CAT (Fig. 3C) resembled the expression patterns observed with the p365 construct of the H-2 promoter (Fig. 3C), namely, exogenous cFos and cJun expressors stimulated expression from either promoters, relative to the basal D122 activity. The AP1 deletion mutant -60Col·CAT (Fig. 3C) showed a low activity in Fos and Jun expressor cells, similar to the activity in parental D122 cells.

### PROTEIN BINDING TO ENHANCER A IN c-fos/c-jun TRANSFECTANTS.

Gel mobility shift assays were performed to analyze the binding properties of nuclear proteins from *c-fos* and *c-jun* overexpressing cells, to the oligodeoxynucleotide sequence of the entire enhancer A region (see Fig. 3A and experimental procedures). As shown by Figure 4A, extracts from the D122 cells show formation of four complexes (bands I-IV) with enhancer A. CHX treatment induces mostly band I. D122 transfection with *c-fos* alone, *c-jun* alone or both, shows elevated formation of complexes I and IV, and to a lesser extent of band II, as well as a decrease of the intensity of band III (Fig. 4A,B). The non-labeled 'AP1' oligonucleotide (that contains AP1/CREB and flanking AP2 sequences, see experimental procedures) competes with bands I-III, and to a lesser extent with band IV (Fig. 4B).

Formation of complexes III and IV seems to be temperature dependent, since preincubation of nuclear extract with anti-Fos antibody or a control antibody at 4°C for one hour prior to addition of the labeled probe decreased significantly bands III and IV. In addition, under these conditions band II seems to split into two distinct complexes, II and II' (Fig. 4C). Complex I, which is elevated by *c-fos*/*c-jun* transfection, is specifically inhibited by pretreatment of the nuclear extracts with anti-Fos antibodies, but not with an unrelated, cell surface antibody (anti H-2D^{b}). Complex II that is marginally affected by the *c-fos*/*c-jun* transfection, seems also to be inhibited by anti-Fos, while complex II' is not.

Nuclear complex profiles of F10.9(B16) melanoma cells, show quantitatively a different profile; complex I, II, and IV are hardly visible, and band III constitutes the major complex. Upon CHX treatment bands I and II are intensified. Again, transfection of *c-fos*+*c-jun* elevated complexes I, II and IV, and reduced complex III (Fig 4A). These experiments indicate that transfection with c-*fos*/*c-jun* or both, increase the binding of specific complexes to enhancer A, and that the Fos protein is a part of at least two of these complexes (I and II).

### NEGATIVE REGULATION OF H-2 BY JunB DOES NOT REQUIRE THE AP1 BINDING SITE

The repression of H-2 cell surface expression of *JunB* transfectants (Fig. 2C) was well correlated with the reduced levels of H-2K mRNA (Fig. 1D). To delineate the target of the JunB negative regulation on H-2 promoter transient transfection of H-2 CAT constructs was performed into the constitutively JunB overexpressing cells (Fig. 2C). Fig. 3D shows that construct p365 which has the maximal basal activity in A9 (3LL) cells is repressed in the JunB expressor AJB1 cells to approximately 70% of the basal (A9) activity (Fig. 3D, compare lanes 1 and 2). The activity of p190, which is 60% of the activity of p365 in A9 cells (compare lanes 1 and 3), was almost completely abolished in the JunB expressor AJB1 cells (Fig. 3D, compare lanes 3 and 4). The construct p142 (Fig. 3B,3D), as well as p38 (not shown) were inactive in both cell lines.

The moderate repression of p365 activity in JunB A9 compared to the striking decrease of p190 activity suggests that the region -190 to -142 includes the major target for JunB transrepression. This would probably be an indirect target, due to lack of known AP1 binding sites in this region. In addition the region -365 to -190 seems to include a site that interferes actively with the JunB suppression of the H-2 promoter. Possibly, the binding of the cJun-cFos complex to the AP1 site at -200 -193, is capable of preventing (partially) the repression by JunB. A possible mechanism may involve competitive interactions between JunB, cJun and cFos.

### MODULATION OF TUMORIGENIC AND METASTATIC POTENCY

Transfected clones were tested for their tumorigenic and metastatic properties in syngeneic C57BL/6 mice which are the original host strain of 3LL and B16 tumors.

Fig. 5A demonstrates the mean growth rates of local tumors from the parental D122 clone and from transfected cell lines. Mice were sacrificed at the time of death of control groups. Lung weights were evaluated as described in experimental procedures. Two independent experiments clearly indicated that the *c-jun* transfectants grew at similar rates to the parental, highly tumorigenic D122 cells. On the other hand, in mice inoculated with the *c-fos* transfectant, F6A2, the growth of the tumor was significantly slower (p<0.005) and in the groups that were inoculated with high expressor *c-fos*+*c-jun* transfectants (FJ23, D6, D13, D56) the tumors did not grow at all in 92% (45 out of 49) of the mice.

To test the ability of these D122 transfectants to metastasize spontaneously in C57BL/6 recipients, primary tumors were amputated when the tumor reached 8mm in diameter and formation of lung metastasis was monitored in moribund mice.

Mice in the control, D122 injected, group, as well as the *c-jun* transfected groups showed very high levels of metastases 65 to 70 days after injection (data not shown). Mice injected with *c-fos* transfectants showed similar levels of metastases 85 to 90 days after injection. In contrast, among mice in six groups injected with double transfectants, 95% were metastasis free 120 days after injection indicating that a complete eradication of tumor cells had occurred at the primary injection site.

Taken together, these spontaneous metastasis assays, which closely parallel the course of disease in human, suggest an absolute suppression of the parental tumorigenic and metastatic phenotype following *c-fos*+*c-jun* cotransfection. This was not due to intrinsic growth inhibitory effects on the cells, since in vitro all transfectants proliferated at a rate similar to that of the parental cells (data not shown).

To test whether the nonmetastatic phenotype manifested by the *c-fos*+*c-jun* transfectants is due to their failure to grow in the foot pad or whether the reduction in metastatic potential will also be manifested by an experimental metastasis assay, these clones were injected intravenously to C57BL/6 mice. The mice were sacrificed when the mice in the control D122 group showed signs of respiratory distress, generally 35 to 39 days after injection. The lungs were excised and metastatic loads were evaluated.

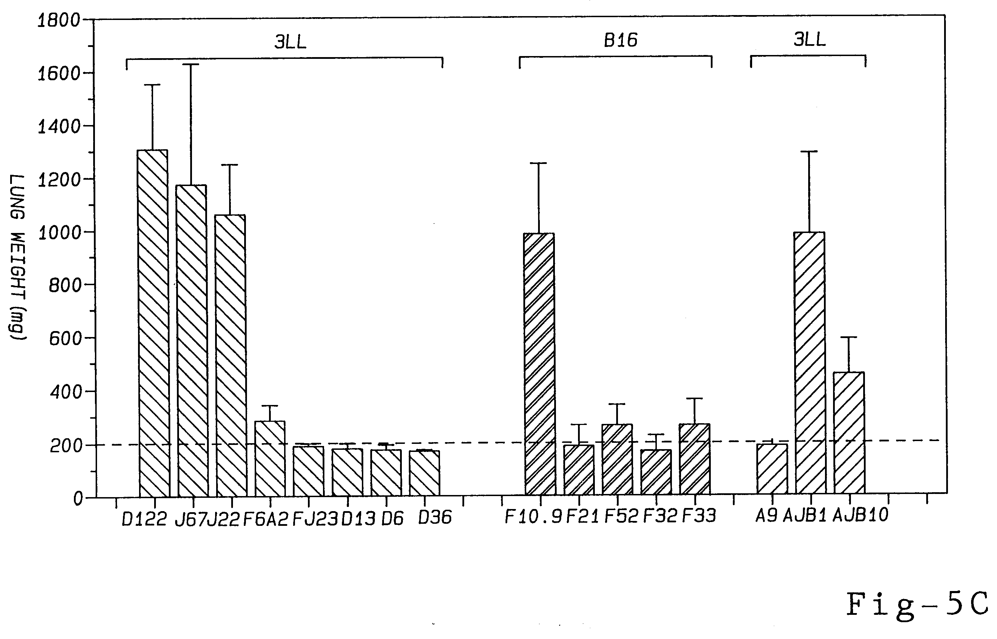

Figures 5C and 6 A-B show the results of two i.v. experiments. The results are consistent with results of the spontaneous (intra footpad) experiments. Both the parental D122 and the *c-jun* transfectants were highly metastatic, the *c-fos* transfected group were of moderate metastatic potential and the double *c-fos*+*c-jun* transfectants were of low or nonmetastatic phenotype. Thus, low tumorigenicity and low metastatic potential were fully correlated for the D122 *c-fos*+*c-jun* transfectants. In randomly selected 3LL clones, no such correlation was previously observed between the high and low metastatic clones (6).

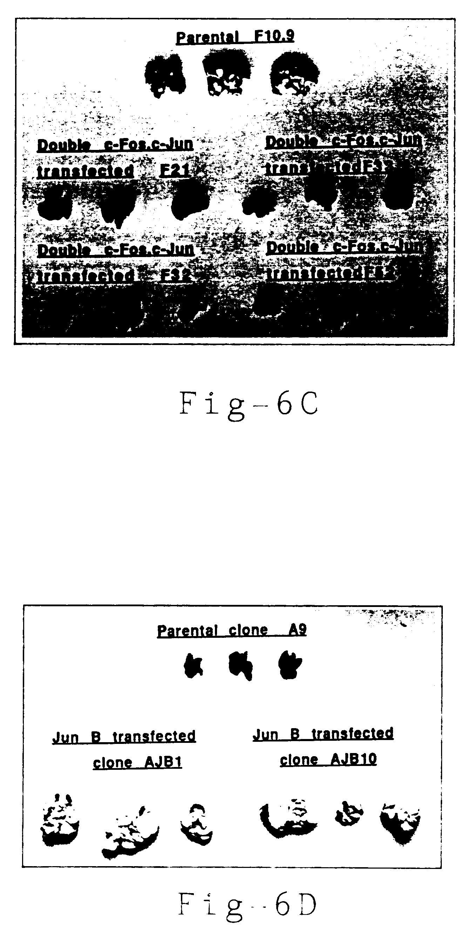

Consistent with the above results are also the experimental metastasis assays with the B16-F10.9, *c-fos*+*c-jun* transfected clones. As shown in Fig. 5C and 6C, the high expressors were of low metastatic phenotype, as opposed to the high metastatic phenotype of the parental clone.

### IMMUNOGENICITY OF THE c-fos, c-jun TRANSFECTANTS

To test *in vivo* whether the low metastatic phenotype of the double transfectants is the result of an interaction with the host's T cell dependent immune system, tumorigenicity and metastasis assays were performed in CD1 nude mice which were deficient in thymic dependent mature T cells.

Fig. 5B shows the growth curves of *c-fos*+*c-jun* transfectants, of a *c-jun* transfectant, and of the parental D122 clone. The growth of the double transfectants is similar to the growth rates of the *c-jun* transfectant in the nude mice. Interestingly, all types of transfectants grew slightly slower than the D122 cells in nude mice. These clones exhibited a high metastatic phenotype, both in spontaneous and experimental assays (data not shown). Thus, the regain of the tumorigenic and metastatic properties by the *c-fos*+*c-jun* transfectants suggests that the suppression of the parental phenotype manifested in the C57BL mice is a result of an elevated immunogenicity which is dependent on a mature T-cell reaction.

Further evidence to the acquired immunogenicity was observed when mice that had been injected by the double transfectants and did not show growth of the primary tumor after 120-150 days (Fig. 5A) were reinjected, intrafootpad, with the parental, high metastatic, D122 cells. In groups previously injected by D13 or FJ23 cells, the D122 cells grew significantly slower than in the control group (p<0.005 compared to naive mice injected with D122 cells), while groups previously injected by D6 or D56 cells showed a smaller, less significant reduction of tumor growth (data not shown). These results suggest that the rejection of the primary tumors created memory cells capable of interacting with a D122 challenge.

The correlation between CTL activity and the malignant phenotypes of the various transfectants were tested. Figure 7 shows the autologous, *in vitro* lytic activity of CTLs elicited in C57BL mice by inactivated (irradiated) D122 clone, *c-fos* transfectant F6A2, *c-jun* transfectant J67 and *c-fos*+*c-jun* cotransfectant clone D13. *In vitro* resensitized splenocytes were reacted with homologous S³⁵-methionine labeled target cells in 16 hours assays. Spontaneous release was under 20% of total release. Triplicate samples were under 5% mean error. Immunization of D122 and J67 elicits low levels of CTL against autologous D122 or J67. In contrast, immunization by the *c-fos* transfectants or by *c-fos*+*c-jun* cotransfectants, elicit higher levels of CTL, that lyse efficiently autologous targets. These data are in agreement with *in vivo* results, and indicate that CTL induction and sensitivity might be a major effector in inhibition of tumorigenicity and metastasis in these transfectants.

### JunB TRANSFECTION CONVERTS LOW TO HIGH METASTATIC CELLS

The negative effect of *junB* transfection of MHC class I expression of A9 (3LL) cells raised the question of the potential effect of *junB* on the metastatic potential. Thus, the *junB* A9 transfected clones (AJB1 and AJB10) were tested for their tumorigenic and metastatic properties in syngeneic C57BL/6 mice. Spontaneous metastasis assays indicated that the local tumor in mice inoculated by the transfectants did not grow significantly faster than the parental A9 clone and no spontaneous metastases developed up to 150 days after amputation. Conversely, when injected directly into the vein, six out of seven mice from the AJB1 group developed metastases 65 days post injection. Thereafter all other mice were sacrificed. Three out of seven mice from the AJB10 group but none of the A9 parental group developed metastases before the cessation of the experiment. Figures 5C (right hand), and Figure 6D demonstrate the i.v. results; while parental A9 cells were nonmetastatic, *junB* transfectants were moderately (AJB10) or high (AJB1) metastatic. Based on the previous result, the increase in the metastatic potency following *junB* transfection is a result of reduced immunogenicity and reduced susceptibility to T-cell lysis.

The above data provide clear evidence that *c-fos*, *c-jun* and *junB* over-expression suppress certain aspects of the cancer process in murine carcinoma and melanoma cells while transfection with *junB* induces a metastatic phenotype. Tumor progression and metastases is a highly complicated process dependent on positive and negative regulation of many genes. Expression of MHC class I genes, that is obligatory for production of cellular immunity, was shown to be a rate limiting step in metastasis of 3LL carcinoma (6), B16 melanoma, T10 sarcoma and a variety of other tumors (39). The data above show a link between expression of MHC class I and expression of *c-fos* and *jun* family genes.

The above results further show that vaccines of human tumors can be generated by transfer of MHC genes and particularly, with two parental MHC genes tailor made for the heterozygous HLA phenotype of each patient. A simpler approach is to activate the expression of the endogenous MHC genes of each heterozygous tumor. This was achieved in accordance with the above experimental data by transfecting tumor cells with the *c-fos* and *c-jun* genes or the *c-fos* gene alone. This is based on conclusions drawn from the data wherein the nonmetastatic H-2K expression clones of the 3LL tumor coexpress the *c-fos* gene. Accordingly, the question is raised whether the expression of the two gene products is causually related and whether the *c-fos* gene is involved in the up regulation of MHC class I genes. Earlier studies (7,8) by applicants indicated that the application in culture of γ-Interferon to D122 cells induced the expression of H-2 genes. The above data demonstrates that following treatment with Interferon, first *c-fos* transcripts were induced, followed by the appearance of H-2K transcripts. Transfection with *c-fos* gene, whether of mouse (SEQ ID No:5) or of human (SEQ ID No:6) origin, converted the H-2K nonexpressors to expressor cells. When such cells were transplanted to syngeneic recipients, the generation of metastasis was significantly reduced.

Other systems in which the *c-fos* was shown to control the expression of cellular genes indicated that the *c-fos* protein does not bind by itself to promote a region, but rather forms through a leucine zipper heterodimers of *c-fos* and *c-jun*, *junB*, or *junD*. These heterodimers act as the nuclear effectors by binding to DNA consensus sequences. The data shows that transfection of tumor cells with *c-fos* induced *c-jun* and *junB* expression in reciprocally transfection with *c-jun* or *c-fos* expression. However, the *c-fos* transfectants manifested a reduction, but not complete abolishment, of the metastatic phenotype. Cotransfection of D122 cells with *c-fos* and *c-jun* genes resulted in a significant coexpression of both genes and up- regulation of H-2 genes. When such double tranfectants were then transplanted into syngeneic animals, they manifested a complete abolishment of their phenotype. In fact, even the local growth of the tranfectants was significantly arrested.

It appears that the insertion of both the *c-fos* and *c-jun* genes resulted in the acquisition of a very effective immunogenic potency. The increase in immunogenicity of double transfectants was not attributed just to the up-regulation of the H-2K expression, since the levels of the H-2K transcripts following *c-fos* transcription was similar to the levels induced following *c-fos* or *c-jun* gene transfer. It is possible that in the double transfectants, only the class I gene products were elevated. The expression of tumorous associated antigens or their process to cell surface peptides could also have been up-regulated following transfection with the *c-fos* and *c-jun* genes. The complete abolishment of the metastatic phenotype due to the acquisition of potent immunogenic properties following transfection with the *c-fos* and *c-jun* gene was achieved also with the B16-F10.9 melanoma. Accordingly, the insertion of the *c-fos* and *c-jun* genes which control the expression of MHC class I genes is a useful strategy for the generation of cellular tumor vaccines made in accordance with the methods described above, avoiding the necessity to tailor-make gene insertion in accordance with the individual MHC phenotypes.

Studies were conducted on a number of lines of human tumor cells. Applying differentiation inducers to such cells in culture, it was observed that whenever terminal differentiation was involved in the up-regulation of HLA expression, *c-fos* transcripts proceeded the appearance of the HLA transcripts. No *c-fos* expression was observed when the differentiation pattern did not involve HLA expression. It is therefore believed that transfer of *c-fos* and *c-jun* genes into human neoplastic cells aiming at increased immunogenicity, is a modality for the generation of cellular vaccines of human tumors. Immunotherapy via gene therapy then comprises the surgical removal of the primary tumor, followed by vaccination with inactivated tumor cells into which *c-fos* and *c-jun* or *c-jun* alone had been inserted, resulting in highly immunogenic vaccine in accordance with the present invention.

The invention has been described in an illustrative manner, and it is to be understood that the terminology which has been used is intended to be in the nature of words of description rather than of limitation.

Obviously many modifications and variations of the present invention are possible in light of the above teachings. It is, therefore, to be understood that within the scope of the appended claims the invention may be practiced otherwise than as specifically described.

### REFERENCES

1. Bishop, M.J. (1991). Molecular Themes in Oncogenesis. Cell 64, 235-248.
2. Baldwin, A.S., Sharp, P.A. (1987). Binding of a Nuclear Factor to a Regulatory Sequence in the Promotor of the Mouse H-2K^{b} Class I Major Histocompatibility Gene. Mol. Cell. Biol. 7, 305-313.
3. Hart, I.R., Goode, N.T., Wilson, R.E. (1989). Molecular Aspects of the Metastatic Cascade. Biochem. Biophys. Acta 989, 65-84.
4. Liotta, L.A., Steet, P.S. (1991). Cancer Metastasis and Angiogenesis: Animbalance of Positive and Negative Regulation. Cell, 64.
5. Eisenbach, L., Feldman, M. (1991). Tumor Cell Surface Determinants in Host Immunity Against Metastases. Seminars in Cancer Biology 2: 179-188.
6. Eisenbach, L., Segal, S., Feldman, M. (1983). MHC Imbalance and Metastatic Spread in Lewis Lung Carcinoma Clones. Int. J. Cancer 32, 113-120.
7. Kushtai, G., Barzilay, J., Eisenbach, L. (1988). The c-fos Protooncogene in Murine 3LL Clones Controlls the Expression of MHC Genes. Oncogene 2, 119-128.
8. Kushtai, G., Feldman, M. Eisenbach, L. (1990). The c-fos Transfection of 3LL Tumor Cells Turns on MHC Gene Expression and Consequently Reduces Their Metastatic Competence. Int. J. Cancer 45, 1131-1136.
9. Barzilay, J., Kushtai, G, Eisenbach, L. (1987). Expression of Major Histocompatibility Class I Genes in Differentiating Leukemic Cells is Temporarily Related to Activation of c-fos Protooncogene. Leukemia 1, 198-204.
10. Miyazaki, J.I., Appella,, E., Ozato, K. (1986b). Negative Regulation of the Major Histocompatibility Class I Gene in Undifferentiated Embryonal Carcinoma Cells. Proc. Natl. Acad. Sci. USA 83, 9537-9541.
11. Diamond, M.I., Miner, J.N., Yamamoto, K.R. (1990). Transcriptional Factor Interactions: Selectors of Positive or Negative regulation From a Single DNA Element. Science 249, 1266-1272.
12. Franza, B.R., Rauccher, F.J., Curran, T. (1988). The fos Complex and fos-related Antigens Recognize Sequence Elements That Contain AP-1 Binding Sites. Science 239, 1150.
13. Miller, R., Curran, T., Miller, D., Guilbert, L. (1985). Induction of c-fos During Myelomonocytic Differentiation and Macrophage Proliferation. Nature 314, 546-548.
14. Chiu, R., Boyle, W.J., Karin, M. (1988). The c-fos Protein Interacts With c-jun/AP-1 to Stimulate Transcription of AP1 Responsive Genes. Cell 54, 541-552.
15. Distel, R.J., Ro, H.S., Spigelman, B.M. (1987). Nucleoprotein Complexes That Regulate Gene Expression in Adipocyte Differentiation: Direct Participation of c-fos. Cell 49, 835-844.
16. Ryseck, R.P., Bravo, R. (1991). c-jun, jun B and jun D Differ in Their Binding Affinities to AP1 and CRE Consensus Sequences: Effect of FOS Proteins. Oncogene 6, 533-542.
17. Fidler, I.J. (1973). Selection on Sacsessive Tumor Lines for Metastasis. Adv. Cancer Res. 28, 149-150.
18. Poste, G., Fidler, J.I. (1978). The Pathogenesis of Cancer Metastasis. Nature 283, 139-146.
19. Porgador, A., Feldman, M, Eisenbach, L. (1989). H-2K Transfection of B16 Melanoma Cells Results in Reduced Tumorigenicity and Metastatic Competence. J. Immuongen. 16, 291-303.
20. Fidler, I.J., Hart, I.R. (1978). The Biologu of Cancer Invasion and Metastasis. Adv. Cancer Res. 28, 149-250.
21. Chirgwin, J.M., Przyblya, A.G., MacDonals, R.J., Rutter, W.J. (1974). Isolation of Biologically Active Ribonucleic Acid From Sources Enriched in Ribonuclease. Biochem. 18, 5294-5299.
22. Verma, I.M., Visvader, J., Lamph, W.W., DeTogni, P., Barber, J., Sassone-Corsi, P. (1988). Regulation of fos Gene: A Paradigm For Nuclear Oncogenes. UCLA Symposia on Molecular and Cellular Biology. New York, New Series.
23. Angel, P., Allegretto, E.A., Okino, S., Hattori, K., Karin, M. (1988a). Oncogen jun Encodes a Sequence Specific Trans-Activator Similar to AP-1. Nature 332, 166-171.
24. Ryder, K. Lanahan, A., Nathans, D. (1988). A Gene Activated by Growth Factors is Related to the Oncogene v-jun. Proc. Natl. Acad. Sci. USA 85, 1487-1491.
25. Nudel, U., Zakut, R., Shani, M., Neuman, S., Levi, Z., Yaffe, D. (1983). The Nucleotide Sequence of the Rat Cytoplasmatic b actin Gene. Nucl. Acids Res. 11, 1759-1771.
26. Baldwin, A., Sharp, P. (1988). Two Transcription Factors, NF-κB and H2TF1, Interact With a Single Regulatory Sequence in the Class I Major Histocompatibility Complex Promoter. Proc. Natl. Acad. Sci. USA 85, 723-727.
27. Ozato, K., Sachs, D.H. (1981). Monoclonal Antibodies Reacting to Antigens of the H-2^{b} Haplotypes Reveal Genetic Control of Isotype Expression. J. Immunol. 126, 317-321.
28. Schreiber, E., Matthias, P., Muller, M., Schaffner, W. (1989). Rapid Deterction of Octamer Binding Proteins With "Mini-Extracts", Prepared From a Small Number of Cells. Nucl. Acids. Res. 17, 6419.
29. Eisenbach, L, Hollander, N., Feldman, M. (1984). The Differential Expression of H-2K versus H-2D Antigens, Distinguishing Low Metastatic From High Metastatic Clones, is Correlated With the Immunogenic Properties of the Tumor Cells. Int. J. Cancer 34, 567-573.
30. Kripke, M.L. (1979). Speculations on the Role of Ultraviolet Radiation in the Development of Malignant Melanoma. J. Natl. Cancer Inst. 63, 541-548.
31. Angel, P., Imagawa, M., Karin, M. (1987). Phorbol Ester-Inducible Contain a Common cis Element Recognizable by a TPA Modulated Transacting Factor. Cell 49, 729-739.
32. Angel, P. Hattori, K, Karin, M. (1988b). The jun Proto-oncogene is Positively Autoregulated by its Product, jun/AP1. Cell 55, 875-885.
33. Ryder, K., Lanahan, A., Nathans, D. (1989). jun-D: A Third Member of the jun Gene Family. Proc. Natl. Acad. Sci. USA 86, 1500-1503.
34. Sassone-Corsi, P., Sisson, C., Verma, M. (1988). Trasncriptional Autoregulation of the Proto-Oncogene fos. Nature 334, 314-319.
35. Lucibello, C.F., Lowag, C., Muller, R. (1989). Trans-Repression of the Mouse c-fos Promoter: A Novel Mechanism of fos-Mediated Transregulations. Cell 59, 999-1007.
36. Verma, I.M., Ransone, L.J., Visvader, J., Sassone-Corsi, P., Lamph, W.V. (1990a). fos-jun Conspiracy: Implications For the Cell. CIBA Found. Symp. 150 on Proto-Oncgenes in Cell Development, Chichester pp. 128-146.
37. Wang, Z.Q., Grigoriadis, A.G., Mohhle-Steinlein, U., Wagner, E.F. (1991). A Novel Target Cell For c-fos Induced Oncogenesis: Development of Chondrogenic Tumors in Embryonic Stem Cell Chimeras. EMBO J. 10, 2437-2450.
38. Israel, A., LeBail, O., Kourilsky, P. (1989). TNF Stimulates Expression of Mouse MHC Class I Genes by Inducing an NFκB-like Enhancer Binding Activity which Displaced Constitutive Factors. EMBO J. 8, 3793-3800.
39. Israel, A., Kimura, A., Kieran, M., Yano, O., Kourilsky, P. (1987). A Common Positive Transacting Factor Binds to Enhancer Sequences in the Promoters of Mouse H-2 and β2-Microglobulin Genes. Proc Natl. Acad. Sci. 84, 2653-2657.
40. Graham, R., Van der Eb,A. (1983). A New Technique For the Assay of Infectivity of Juman Adenovirus 5 DNA. Virol. 52, 456-467.
41. Schule, R. Muller, M. Kaltschmidt, C., Renkawitz, R. (1988). Many Transcription Factors Interact Synergistically With Steroid Receptors. Science 242, 1418-1430.
42. Nielsen, D.A., Chou, J. Mackrell, A.J., Casabadan, M.J. Steiner, D.F. (1983). Expression of a Preproinsulin b-Galactosidase Gene Fusion in Mammalian Cells. Proc. Natl. Acad. Sci. USA 80, 5198-5202.
43. Muller, R., Wagner, E.F. (1984). Differentiation of F9 Tetratocarcinoma Stem Cells After Transfer of c-fos Proto-Oncogenes. Nature 311, 438-442.
44. Schutte, J., Viallet, J., Minna, J. (1989). jun-B Inhibits and c-fos Stimulates the Transforming and Transactivating Activities of c-jun. Cell 59, 987-997.
45. Fidler, I.J., Radinsky, R. (1990). Genetic Control of Cancer Metastasis. J. Natl. Cancer Inst. 82, 166-168.
46. Marnino R.J. and S. Gould-Fogeritz, 1988, biotechniques 6:682)
47. Vaheri A. and J.S. Pagano, 1965, Virology, 27:434)
48. Kawai S. and M. Nishizawa, 1984, Mol. Cell Biol.4:1172
49. Schaffner, W. 1980, Proc. Natl. Acad. Sci. USA 77:2163
50. Porgador, A., Tzehoval, E. , et al. 1992, IL6 gene transfection into 3LL tumor cells suppresses the metastatic phenotype and confers. immunotherapeutic competence against parental metastatic cells. Cancer Res. 52:3679-3686
51. Plaksin, D. Gelber, C., Feldman M, Eisenbach L., Reversal of the metastatic phenotype of Lewis Lung Carcinoma cells following transfection with syngeneic H-2K^{b} gene. Proc Natl. Acad. Sci. USA 85:4463-4467
52. Lider, O. et al., 1986, Vaccination agains experimental autoimmune disease using T lymphocytes treated with hydrostatic pressure. Ann. N.Y. Acad. Sci. Part V, New Forms of Therapy, pp. 267-273
53. Sullenger, B.A., Lee, T.C., Smith, C.A., Gilboa, E., 1990, Expression of chimeric tRNA driven antisense transcripts renders NIH-3T3 cells highly resistant to moloney murine leukemia virus replication. Mol. Cell Biol. 10:6512-6523
54. Mastrangelo, M.G. et al., "Immunotherapy with microbial products", in Immunological approaches to cancer therapeutics", New York, John Wiley and Sons, p. 75, 1982
55. Bystryn, J.C. et al., Immunogenicity of polyvalent melanoma vaccines in human. Cancer 61:1065. 1988
56. Hollinshead A. et al., Pilot studies using melanoma tumor associated antigens (TAA) in specific active immunotherapy of malignant melanoma, Cancer 49:1387, 1982
57. Katz A., Feldman, M., Eisenbach, L. (1992) The Use of ³⁵Smithionine as a target cell label in long-term cytotoxic assays. J. Immunol. Meth. 49:255-260
58. Kokoahka, E.M., Michsche M. Active specific immunotherapy as adjuvant treatment for stage II malignant melanoma. Pigm. Cell (1979) 5,11
59. Gilboa et al. (1986)
60. Bradford M., Anal. Biochem., 72:248 (1976)

## Claims

1. Anti-tumor cellular vaccine comprising tumor cells into which *c-fos* gene (SEQ ID No:6) alone or together with *c-jun* gene (SEQ ID No:3) have been inserted.

2. An anti-tumor vaccine according to claim 1 wherein said tumor cells are human tumor cells.

3. An anti-tumor vaccine according to claim 1 or 2 wherein said tumor cells are derived from tumor cells having metastatic competence.

4. An anti-tumor vaccine according to any of claims 1 to 3 comprising human tumor cells transfected with *c-fos* gene.

5. An anti-tumor vaccine according to any of claims 1 to 3 comprising human tumor cells transfected with both *c-fos* and *c-jun* genes.

6. An anti-tumor vaccine according to claim 5 wherein the *c-fos* and *c-jun* genes have been introduced into said tumor cells on a single expression vector enabling constitutive production of the *c-fos* and *c-jun* gene products *in vivo*.

7. An anti-tumor vaccine according to claim 5 wherein the *c-fos* and *c-jun* genes have been introduced into said tumor cells on different expression vectors enabling constitutive production of the *c-fos* and *c-jun* gene products *in vivo*.

8. An anti-tumor vaccine according to claim 4 wherein *c-fos* gene has been introduced into said tumor cells on an expression vector enabling constitutive production of the *c-fos* gene product *in vivo*.

9. An anti-tumor vaccine according to any of claims 1 to 8 wherein said tumor cells are cells from a tumor from the individual to be vaccinated.

10. An anti-tumor vaccine according to any of claims 1 to 8 wherein said tumor cells are derived from individuals other than the individual to be vaccinated.

11. An anti-tumor vaccine according to any of claims 1 to 8 comprising transfected tumor cells which show increased levels of expression of MHC class I protein.

12. An anti-tumor vaccine according to any of claims 1 to 11 wherein said tumor cells have been inactivated.

13. An anti-tumor vaccine according to claim 12 wherein said tumor cells have been inactivated by treatment with gamma or X-rays and/or mitomycin C.

14. An anti-tumor vaccine according to any of claims 1 to 13 comprising from about 1 x 10⁶ to about 1 x 10⁹ transfected tumor cells.

15. An anti-tumor vaccine according to any of claims 1 to 14 characterized in that it is formulated as an injection.

16. An anti-tumor vaccine comprising antigens expressed by c-fos gene alone or together with c-jun genes.

17. An anti-tumor vaccine according to any of claims 1 to 16 for the treatment of a patient suffering from cancer to prevent and/or inhibit the development of metastases.

18. A method for the production of an anti-tumor vaccine according to any of claims 1 to 16 comprising the steps of:
a) removing cells from a primary tumor of the patient by biopsy or surgery;
b) dispersing the cells in a medium;
c) inserting into said cells a vector comprising the human *c-fo*s gene or the human *c-fos* and *c-jun* genes;
d) optionally selecting the positive transfectants that show high expression of *c-fos* and MHC class I genes; and
e) inactivating the transfectants by gamma- or X-ray irradiation and/or treatment with mitomycin C.

19. A method of inducing MHC expression in tumor cells from endogenous genes by transfecting the tumor cells with *c-fos* or *c-fos* and *c-jun* genes.
